(19) Europäisches Patentamt
European Patent Office
Office européen des brevets

(11) **EP 4 202 041 A1**

(12) **EUROPEAN PATENT APPLICATION**
published in accordance with Art. 153(4) EPC

(43) Date of publication:
**28.06.2023 Bulletin 2023/26**

(21) Application number: **21858422.5**

(22) Date of filing: **17.08.2021**

(51) International Patent Classification (IPC):
***C12N 5/075*** (2010.01)   ***C12N 5/0735*** (2010.01)

(52) Cooperative Patent Classification (CPC):
**C12N 5/06**

(86) International application number:
**PCT/JP2021/030701**

(87) International publication number:
**WO 2022/039279 (24.02.2022 Gazette 2022/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.08.2020 US 202063066914 P**
**19.08.2020 US 202063067776 P**

(71) Applicant: **Kyoto University**
**Kyoto-shi, Kyoto 606-8501 (JP)**

(72) Inventors:
• **SAITOU, Mitinori**
**Kyoto-shi Kyoto 606-8501 (JP)**
• **MURASE, Yusuke**
**Kyoto-shi Kyoto 606-8501 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **METHOD FOR MAINTAINING AND AMPLIFYING HUMAN PRIMORDIAL GERM CELLS / HUMAN PRIMORDIAL GERM CELL-LIKE CELLS**

(57)    The present invention provides a method for maintaining and expanding a human primordial germ cell-like cell (hPGCLC) derived from a human primordial germ cell (hPGC) or a human pluripotent stem cell, including culturing hPGCLC in the presence of (i) a forskolin or phosphodiesterase 4 (PDE4) inhibitor, and (ii) one or more cytokines selected from the group consisting of a basic fibroblast growth factor (bFGF), a leukemia inhibitory factor (LIF), and an epidermal growth factor (EGF).

EP 4 202 041 A1

**Description**

[Technical Field]

This application claims priority based on a provisional patent application No. 63/066,914 filed in the US on August 18, 2020 and a provisional patent application No. 63/067,776 filed in the US on August 19, 2020, the contents of both applications are incorporated herein.

The present invention relates to a method for maintaining and expanding human primordial germ cells/human primordial germ cell-like cells, and reagents and the like therefor.

[Background Art]

Germ cell lineage ensures the continuity and diversity of not only genetic information but also epigenic information across generations, thereby providing the basis for the permanence and evolution of a given species. In humans, abnormal germ cell development results in pathological conditions including infertility and genetic or epigenetic disorders in offspring. Therefore, the study of mechanism of germ cell development is a theme underlying both biology and medicine.

The mechanism of the development of mammalian germ cells has been studied using mouse as an animal model, and in vitro reconstitution of germ cell development in mouse has been reported. As one embodiment thereof, the present inventors previously reported a method for in vitro proliferation of mouse primordial germ cells (PGC)/primordial germ cell-like cells (PGCLC) using forskolin and rolipram, which stimulate intracellular production of cAMP (Patent Literature 1, Non Patent Literature 1) (hereinafter PGC of mouse is sometimes referred to as "mPGC", and PGCLC of mouse is sometimes referred to as "mPGCLC").

On the other hand, research on the mechanism of human germ cell development has not been sufficiently conducted to date, due to the difficulty in the analysis of human germ cell development and the lack of an appropriate experimental system. Under such circumstances, Gell et al. reported that human primordial germ cell-like cells (hereinafter PGCLC of human is sometimes referred to as "hPGCLC") were cultured in vitro in a culture system containing forskolin and rolipram (Non Patent Literature 2). However, the proliferation of hPGCLC in the report of Gell et al. is about 2-fold in 10 days, which is not sufficient for practical use.

[Citation List]

[Patent Literature]

[PTL 1]
WO 2019/107576

[Non Patent Literature]

    [NPL 1]
    Ohta et al., The EMBO Journal, (2017) Vol.36, p1888-1907
    [NPL 2]
    Gell et al., Stem Cell Reports, (2020) Vol.14, p433-446

[Summary of Invention]

[Technical Problem]

It is therefore the problem of the present invention to a provide a method for more efficiently proliferating human primordial germ cells (hereinafter human PGC is sometimes referred to as "hPGC")/hPGCLC.

[Solution to Problem]

The present inventors have found that hPGCLC can be maintained and expanded by culturing isolated hPGCLC in the presence of forskolin and cytokine. According to the method of the present invention, hPGCLC can be cultured for a long period of time (e.g., 120 days or more). It was confirmed from the analysis of the gene expression state that the hPGCLC cultured by the method of the present invention proliferated while maintaining the properties of initial human PGC. In addition, it was clarified from the analysis of the genomic DNA methylation state that hPGCLC generally maintained the DNA methylation state during the culture period.

This is in contrast to the mPGCLC culture systems (Patent Literature 1, Non Patent Literature 1) in which genome-wide DNA demethylation was observed along with proliferation, and it was suggested that the reorganization of epigenomic information may progress in human by a mechanism different from that in mouse.

To verify the function of proliferated hPGCLC, the present inventors have further performed reconstituted ovarian culture mimicking the intraovarian environment. As a result, it was confirmed that hPGCLC-derived cells express genes such as DDX4 (also known as VASA homolog) and DAZL, and differentiate into oogonia-like cells having morphological characteristics of oogonium. That is, it was confirmed that the properties as a germ cell are maintained in this culture system.

In the maintenance and expansion culture of hPGCLC, the present inventors have established, by FACS analysis using an hPGC marker, a dead cell marker, and a human specific cell surface antigen, a method for evaluating the hPGCLC maintenance and expansion efficiency in the culture system by measuring the cell numbers of expanded hPGCLC and dedifferentiated cells, and calculating the logarithmic conversion value (enrichment score) of the ratio of the number of expanded PGCLC cells to the number of dedifferentiated cells. In addition, they have found that, once an appropriate feeder cell is selected, the cells dedifferentiated from hPGCLC and the feeder cells can be distinguished without sorting hPGCLC-derived cells and feeder cells but by sorting hPGC marker-positive cells and other cells, analyzing non-hPGC marker-positive cells by FACS, and subjecting the cells to an FSC/SSC two-dimensional plot.

Using such evaluation method, the present inventors have further evaluated hPGCLC maintenance and expansion efficiency (dedifferentiation rate from hPGCLC) by adding various signal transduction pathway inhibitors to the above-mentioned maintenance and expansion culture system for hPGCLC. As a result, they have found that Wnt signal transduction inhibitors, particularly a substance that promotes the degradation of β-catenin and/or inhibits the nuclear translocation thereof remarkably suppresses the dedifferentiation of hPGCLC.

The present inventors have conducted further studies based on these findings and completed the present invention.

That is, the present invention provides the following.

[1] A method for maintaining and expanding a human primordial germ cell (hPGC) or a human primordial germ cell-like cell (hPGCLC) derived from a human pluripotent stem cell, comprising culturing hPGC or hPGCLC in the presence of (i) a forskolin or phosphodiesterase 4 (PDE4) inhibitor, and (ii) one or more cytokines selected from the group consisting of a basic fibroblast growth factor (bFGF), a leukemia inhibitory factor (LIF), and an epidermal growth factor (EGF).

[2] The method of [1], wherein the aforementioned inhibitor of (i) is forskolin.

[3] The method of [1] or [2], wherein the aforementioned cytokine is a combination of LIF and EGF, bFGF alone, or a combination of LIF, EGF, and bFGF.

[4] The method of any one of [1] to [3], wherein the hPGCLC is induced via a human initial mesoderm-like cell (hiMeLC).

[5] The method of [4], wherein the hPGCLC is induced by culturing hiMeLC for 5 to 8 days in the presence of bone morphogenic protein 4 (BMP4), and optionally one or more cytokines selected from the group consisting of a stem cell factor (SCF), LIF, and EGF.

[6] The method of any one of [1] to [5], wherein the aforementioned culture comprises sorting hPGC or hPGCLC with hPGC marker positivity as an index and passaging the hPGC or hPGCLC.

[7] The method of [6], wherein the hPGC marker is BLIMP1 and/or TFAP2C, or INTEGRINα6 and EpCAM.

[8] The method of [6] or [7], wherein the hPGC or hPGCLC is cultured for at least 30 days.

[9] The method of any one of [1] to [8], wherein, in the aforementioned culture, the hPGC or hPGCLC is cultured under the conditions further including (iii) a Wnt signal transduction inhibitor.

[10] The method of [9], wherein the Wnt signal transduction inhibitor is a substance that promotes degradation and/or inhibits nuclear translocation of β-catenin.

[10a] The method of [9], wherein the Wnt signal transduction inhibitor is IWR1 or XAV939.

[11] A method for producing a cell population comprising hPGC or hPGCLC, comprising a step of maintaining and expanding the hPGC and/or hPGCLC by the method of any one of [1] to [10] and [10a] .

[12] An expanded hPGC population or hPGCLC population obtained by the method of any one of [1] to [10] and [10a].

[13] A reagent kit for maintaining and expanding hPGC or hPGCLC, comprising (a) a forskolin or PDE4 inhibitor, and (b) one or more cytokines selected from the group consisting of bFGF, LIF, and EGF.

[14] The reagent kit of [13], wherein the inhibitor of the aforementioned (a) is forskolin, and the cytokine of the aforementioned (b) is a combination of LIF and EGF, bFGF alone, or a combination of LIF, EGF, and bFGF.

[15] The reagent kit of [13] or [14], further comprising a Dulbecco's modified Eagle medium (DMEM) with a glucose concentration of 1 g/L.

[16] The reagent kit of any one of [13] to [15], further comprising a Wnt signal transduction inhibitor.

[16a] The reagent kit of [16], wherein the Wnt signal transduction inhibitor is a substance that promotes the degradation and/or inhibits nuclear translocation of β-catenin.

[16b] The reagent kit of [16], wherein the Wnt signal transduction inhibitor is IWR1 or XAV939.

[17] The reagent kit of any one of [13] to [16], [16a], and [16b], further comprising an antibody against INTEGRINα6 and/or an antibody against EpCAM.

[18] A method for producing a human oogonia-/gonocyte-like cell comprising culturing in aggregates the hPGC or hPGCLC of [12] with ovarian somatic cells.

[18a] The method of [18], wherein the ovarian somatic cell is derived from a non-human mammal.

[19] A method for evaluating maintenance culture of hPGC or hPGCLC, comprising

(1) a step of culturing hPGC or hPGCLC on feeder cells under given conditions,
(2) a step of sorting viable cells from a cell population obtained in (1),
(3) a step of sorting the viable cells obtained in (2) into hPGC marker-positive cells and other cells,
(4) a step of subjecting the cells obtained in (3) other than the hPGC marker-positive cells to flow cytometry, and distinguishing cells dedifferentiated from the hPGC or hPGCLC and the feeder cells by two dimensional plots of FSC/SSC, and
(5) a step of evaluating a maintenance efficiency of the hPGC or hPGCLC under the aforementioned given conditions, based on the number of the hPGC marker-positive cells obtained in (3) and the number of the dedifferentiated cells obtained in (4).

[19a] The method of [19], wherein the feeder cell is an m220-5 cell.

[19b] The method of [19] or [19a], wherein the evaluation in step (5) is performed based on the enrichment score represented by the following formula:

$$\text{enrichment score} = \log_2 (\text{number of hPGC marker-positive cells/number of dedifferentiated cells}).$$

[20] The method of [19], [19a], or [19b], wherein the hPGC marker-positive cell is a BLIMP1 and TFAP2C double-positive cell.

[21] A method for screening for an hPGC or hPGCLC dedifferentiation inhibitor, comprising

(a) culturing hPGC or hPGCLC on feeder cells under given conditions in the presence or absence of a candidate substance,
(b) evaluating a maintenance efficiency of hPGC or hPGCLC by the method of [19], [19a], [19b], or [20],
(c) selecting a candidate substance as a dedifferentiation inhibitor of hPGC or hPGCLC when the maintenance efficiency in the presence of the candidate substance is high as compared with the maintenance efficiency in the absence of the candidate substance.

The present invention also provides the following.

[22] A method for maintaining and expanding PGC or PGCLC, comprising culturing PGC or PGCLC in the presence of a Wnt signal transduction inhibitor.

[22a] The method of [22], wherein the culture is performed under the conditions further comprising a forskolin and/or PDE4 inhibitor.

[22b] The method of [22], wherein the PGC or PGCLC is a human cell, and the culture is performed under the conditions

(i) further comprising forskolin or a PDE4 inhibitor, and/or
(ii) further comprising one or more cytokines selected from the group consisting of bFGF, LIF, and EGF.

[22c] The method of [22], wherein the PGC or PGCLC is a non-human animal cell, and the culture is performed under the conditions

(i) further comprising forskolin and a PDE4 inhibitor, and/or
(ii) further comprising cyclosporin A (CsA).

[22d] The method of [22], [22a], [22b], or [22c], wherein the Wnt signal transduction inhibitor is a substance that promotes the degradation and/or inhibits nuclear translocation of β-catenin.

[22e] The method of [22d], wherein the Wnt signal transduction inhibitor is IWR1 or XAV939.

[23] A method for suppressing dedifferentiation of PGC or PGCLC, comprising, in maintenance and expansion culture of PGC or PGCLC, culturing the cells in the presence of a Wnt signal transduction inhibitor.

[23a] The method of [23], wherein the culture is performed under the conditions further comprising a forskolin and/or PDE4 inhibitor.

[23b] The method of [23], wherein the PGC or PGCLC is a human cell, and the culture is performed under the conditions

> (i) further comprising forskolin or a PDE4 inhibitor, and/or
> (ii) further comprising one or more cytokines selected from the group consisting of bFGF, LIF, and EGF.

[23c] The method of [23], wherein the PGC or PGCLC is a non-human animal cell, and the culture is performed under the conditions

> (i) further comprising forskolin and a PDE4 inhibitor, and/or
> (ii) further comprising CsA.

[23d] The method of [23], [23a], [23b], or [23c], wherein the Wnt signal transduction inhibitor is a substance that promotes the degradation and/or inhibits nuclear translocation of β-catenin.

[23e] The method of [23d], wherein the Wnt signal transduction inhibitor is IWR1 or XAV939.

[24] A PGC or PGCLC dedifferentiation inhibitor comprising a Wnt signal transduction inhibitor.

[25] A method for producing PGCLC, comprising inducing differentiation of a pluripotent stem cell (PSC) into PGCLC in the presence of a Wnt signal transduction inhibitor.

[Advantageous Effects of Invention]

Using the method of the present invention, it is possible to culture hPGC or hPGCLC for a long term (e.g., 120 days or longer) and expand the hPGC or hPGCLC one million times or more.

[Brief Description of Drawings]

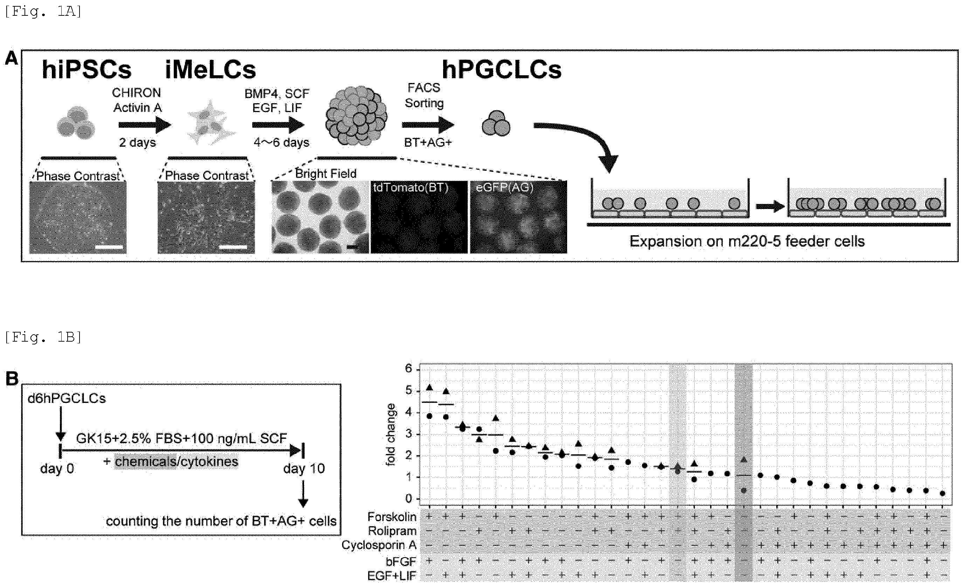

[Fig. 1A] Exploration of conditions for expanding hPGCLC in vitro. (A) Scheme of induction and culture on m220-5 feeder. hPGCLCs were sorted by FACS as BLIMP1-tdTomato (BT) and TFAP2C-EGFP (AG) positive (BT$^+$AG$^+$) cells. Scale bar, 200 um.

[Fig. 1B] Exploration of conditions for expanding hPGCLC in vitro. (B) (Left) Scheme for exploration of conditions for BT$^+$AG$^+$ cell proliferation. (Right) Plot showing the number of BT$^+$AG$^+$ cells after 10 days of culture under the indicated conditions determined by FACS analysis. (Blue) Combination of chemical substances [forskolin (10 μM), rolipram (10 μM), and cyclosporin A (5 μM)] used for BT$^+$AG$^+$ cell proliferation. (Yellow) Combination of cytokines [LIF (10 ng/ml), EGF (50 ng/ml), and basic fibroblast growth factor (bFGF: 20 ng/ml)] used for BT$^+$AG$^+$ cell proliferation. About 5000 hPGCLCs were used as the starting cell population. Since cyclosporin A adversely affected the proliferation of BT$^+$AG$^+$ cells, a reproducibility experiment was performed under the conditions without cyclosporin A. Mean fold changes are indicated by horizontal bars. Conditions for mPGCLC expansion: green. Conditions without additional chemical substance/cytokine: gray.

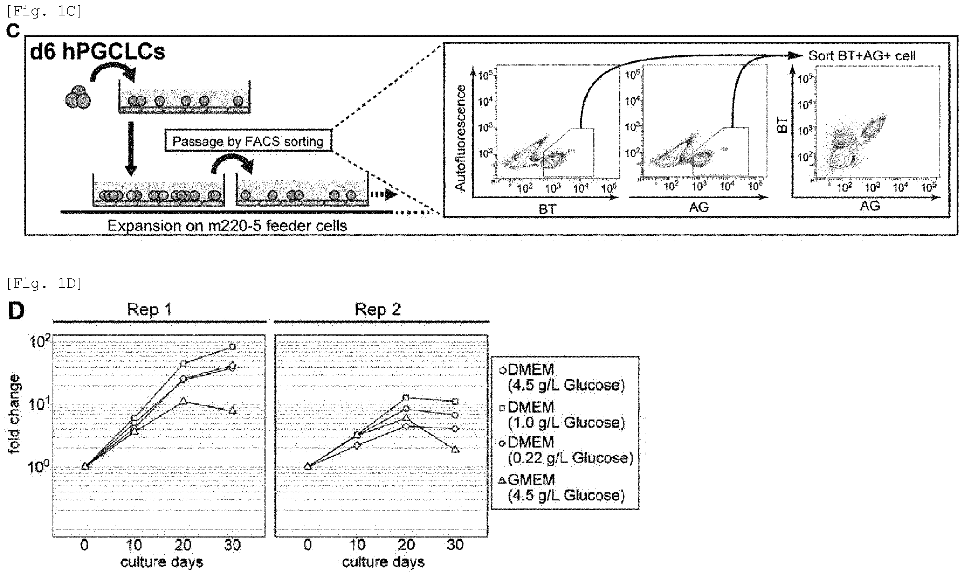

[Fig. 1C] Exploration of conditions for expanding hPGCLC in vitro. (C) Scheme of passage of BT$^+$AG$^+$ cells by FACS. BT$^+$ and AG$^+$ cells were defined as cells within the indicated FACS gate.

[Fig. 1D] Exploration of conditions for expanding hPGCLC in vitro. (D) Results of duplicate experiments of expansion of BT$^+$AG$^+$ cells in the indicated basal media containing 15% KSR, 2.5% FBS, 100 ng/ml SCF, 10 μM forskolin, and 20 ng/ml bFGF. About 5000 hPGCLCs were used as a starting cell population (0 day of culture: c0) and increases in the numbers thereof measured by FACS at passages c10, c20, and c30 were shown as fold changes from c0.

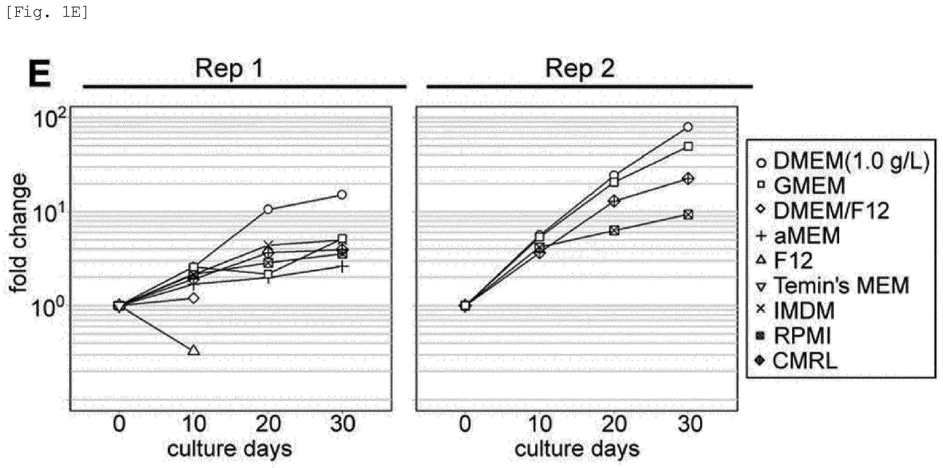

[Fig. 1E] Exploration of conditions for expanding hPGCLC in vitro. (E) Results of duplicate experiments of expansion of BT$^+$AG$^+$ cells in the indicated basal media containing 15% KSR, 2.5% FBS, 100 ng/ml SCF, 10 μM forskolin, and 20 ng/ml bFGF. About 5000 hPGCLCs were used as a starting cell population (0 day of culture: c0) and increases in the numbers thereof measured by FACS at passages c10, c20, and c30 were shown as fold changes from c0.

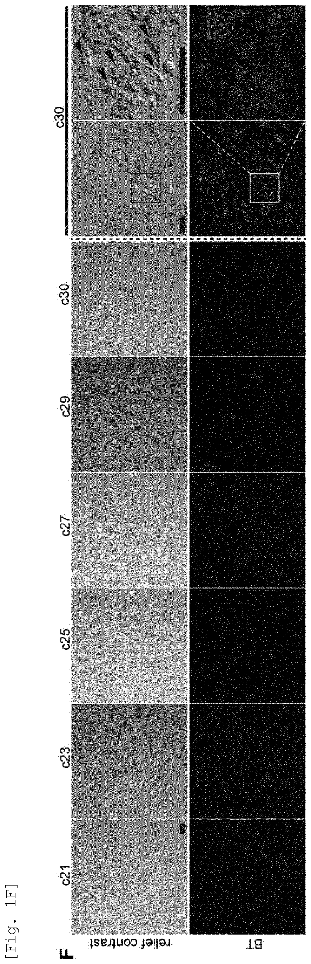

[Fig. 1F] Exploration of conditions for expanding hPGCLC in vitro. (F) Typical examples of relief contrast and fluorescence (BT) images of BT$^+$AG$^+$ cell expansion culture of c21 to c30. Images of C21, 23, 25, 27, 29, and 30 are shown. The rightmost panel is an enlargement of the boxed area at c30 of the central panel. Arrows indicate BT[+] cells with typical morphology. Scale bar: left, 100 um; second from right, 200 $\mu$m; right, 50 um.

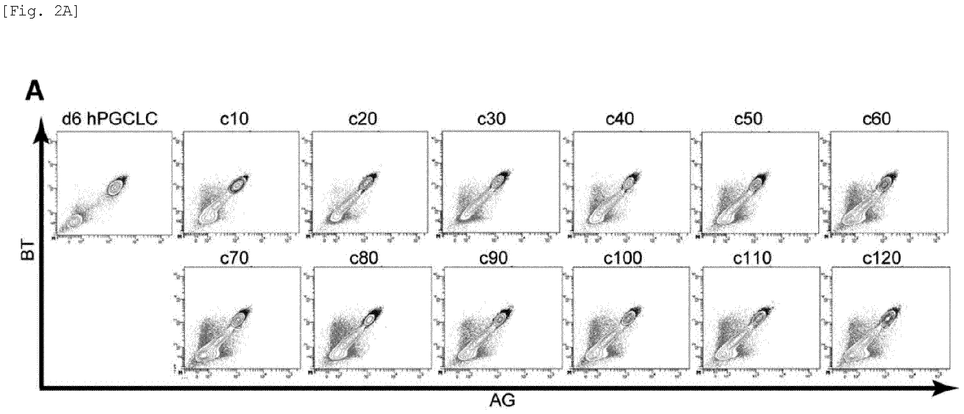

[Fig. 2A] Long-term proliferation of hPGCLC under indicated conditions in vitro. (A) FACS plots of BTAG in hPGCLC-induced d6 and c10 to c120 of BT[+]AG[+] cell expansion culture starting from d6 hPGCLC on m220 feeder cells in DMEM (glucose 1 g/L) containing 15% KSR, 2.5% FBS, 100 ng/ml SCF, 10 $\mu$M forskolin, and 20 mg/nl bFGF. Positive BTAG expression is shown in Fig. 1C.

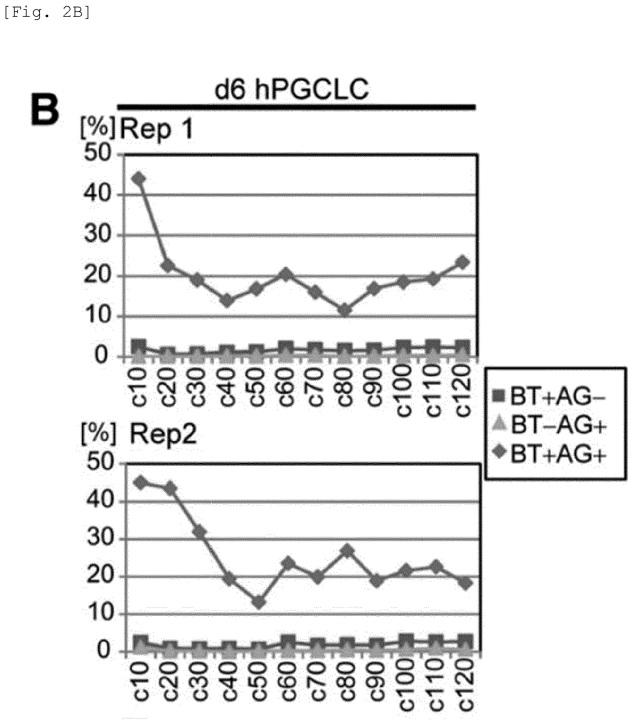

[Fig. 2B] Long-term proliferation of hPGCLC under indicated conditions in vitro. (B) Changes in the percentage of BT[+]AG[+], BT[+], and AG[+] cells during BT[+]AG[+] cell proliferation up to c120. The meaning of the colors is as shown in a different frame.

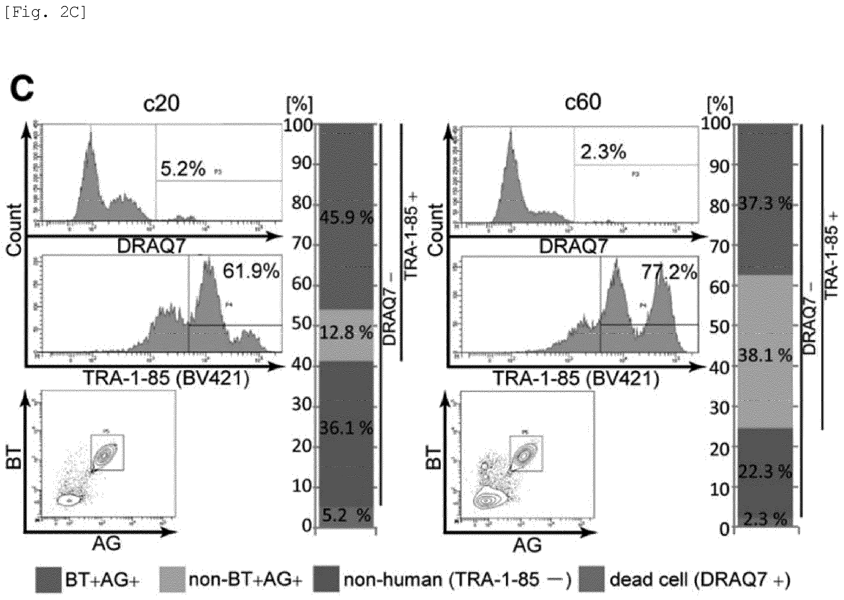

[Fig. 2C] Long-term proliferation of hPGCLC under indicated conditions in vitro. (C) FACS analysis charts for DRAQ7 uptake (top), TRA-1-85 expression (middle) at c20 (left) and c60 (right) cells, and BT AG (bottom) positivity in TRA-1-85[+] cells. The graphs on the right side of the chart show percentages of TRA-1-85+/BT[+]AG[+] cells (purple), TRA-1-85[+]/non-BT[+]AG[+] cells (green), TRA-1-85[-] cells (red), and DRAQ7[+] cells (blue).

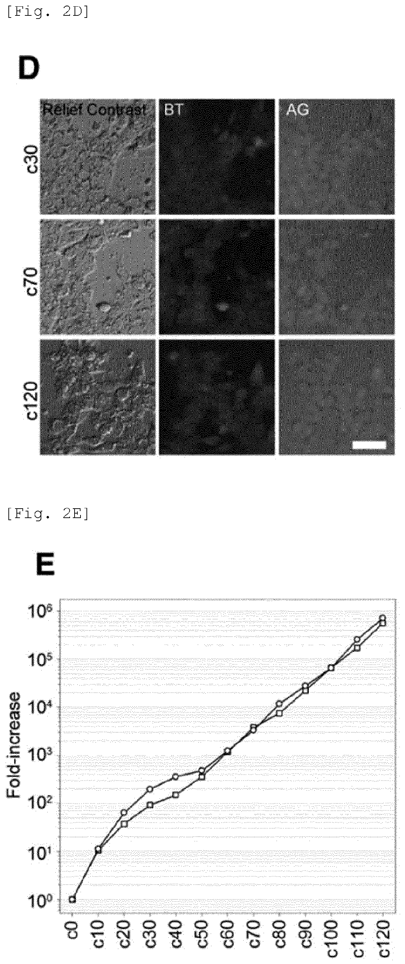

[Fig. 2D] Long-term proliferation of hPGCLC under indicated conditions in vitro. (D) Relief contrast (left) and fluorescence images [BT (middle) and AG (right)] of BT[+]AG[+] cell expansion culture of c30, 70 and 120. When AG[+] cells were cultured two-dimensionally on plates, the fluorescence of AG became dim. Scale bar, 50 $\mu$m.

[Fig. 2E] Long-term proliferation of hPGCLC under indicated conditions in vitro. (E) Growth curve of BT[+]AG[+] cells as indicated by $\log_{10}$ (fold increase) during expansion culture of BT[+]AG[+] cells up to c120 in duplicate experiments. 10,000 hPGCLCs were used as a starting cell population and 10,000 BT[+]AG[+] cells were replated in each passage.

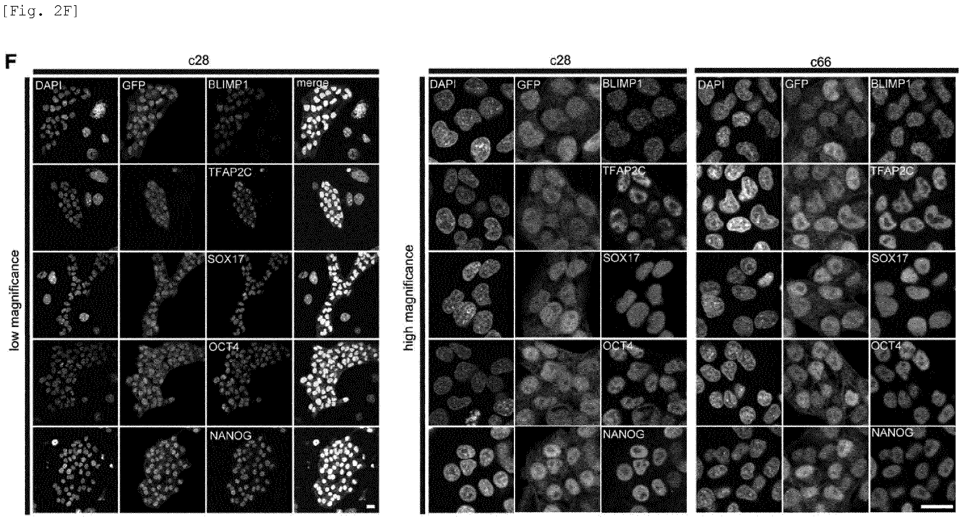

[Fig. 2F] Long-term proliferation of hPGCLC under indicated conditions in vitro. (F) Immunofluorescence (IF) analysis of the expression of BLIMP1, TFAP2C, SOX17, OCT4 (POU5F1), and NANOG (cyan) in AG[+] (GFP) (yellow) cells during BT[+]AG[+] cell expansion cultures at c28 [low magnification (left) and high magnification (middle)] and c66 [high magnification (right)]. Cells were counterstained with DAPI (white). For c28 [low magnification], the merged image is shown on the right end. Scale bar, 20 um.

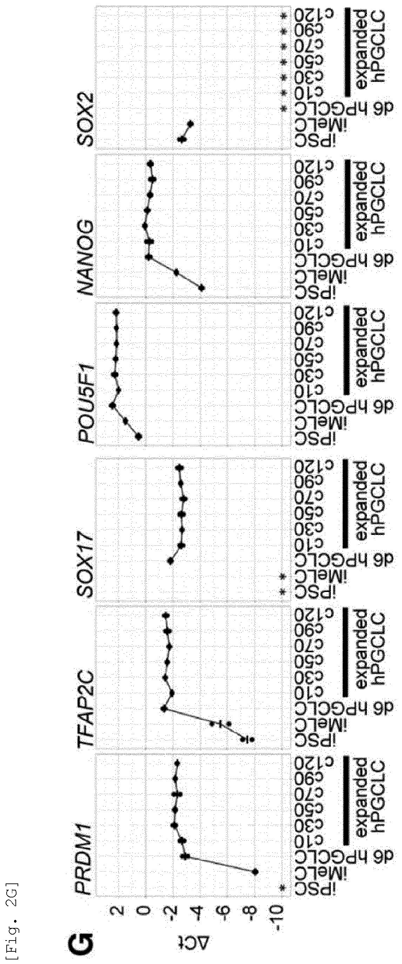

[Fig. 2G] Long-term proliferation of hPGCLC under indicated conditions in vitro. (G) Quantitative PCR (qPCR) analysis of the expression of the indicated gene during hPGCLC induction and BT[+]AG[+] cell expansion culture. For respective genes tested, $\Delta$Ct from the average Ct values of two independent housekeeping genes, RPLPO and PPIA (set as 0), was calculated and plotted for two independent experiments. The average values were connected with a line. *: Not detected or $\Delta$Ct <-10

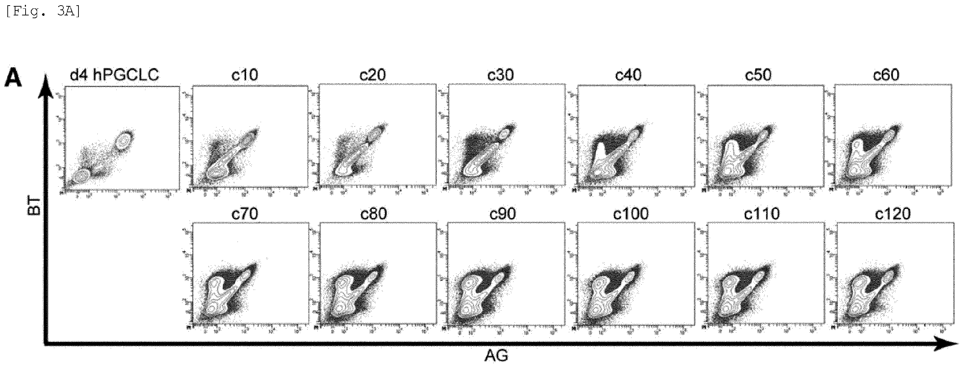

[Fig. 3A] Long-term expansion of BT[+]AG[+] cells from d4 hPGCLC. FACS plots of BTAG expression at d4 of hPGCLC induction and BTAG expression at c10 - c120 of the expansion culture of BT[+]AG[+] cells started from d4 hPGCLC on the m220 feeder in DMEM (glucose 1g/L) containing 15% KSR, 2.5% FBS, 100 ng/ml SCF, 10 $\mu$M forskolin, and 20 ng/ml bFGF. The positivity of BTAG expression was defined as shown in Fig. 1C.

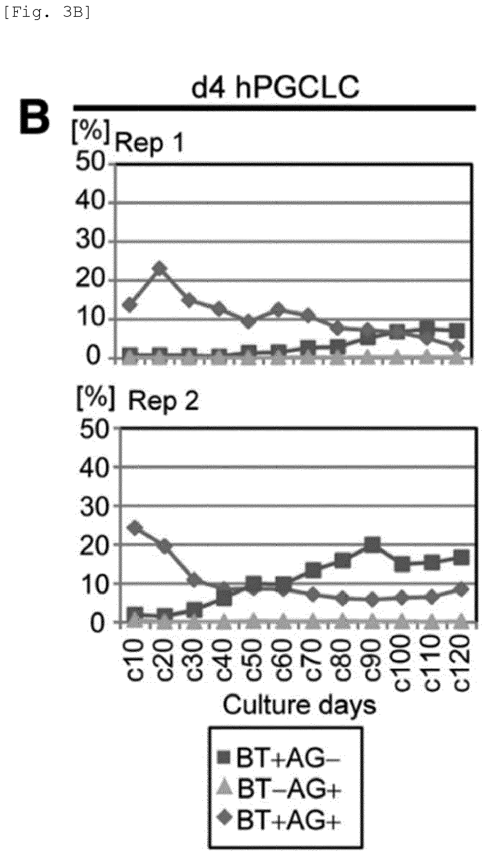

[Fig. 3B] Long-term expansion of BT[+]AG[+] cells from d4 hPGCLC. (B) Changes in the percentages of BT[+]AG[+], BT[+], and [+]AG[+] cells during expansion culture of BT[+]AG[+] cells up to c120. The colors mean as shown therein.

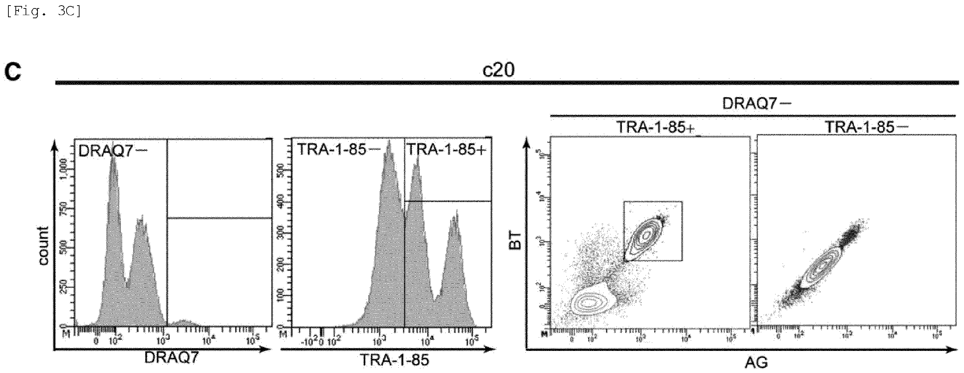

[Fig. 3C] Long-term expansion of BT[+]AG[+] cells from d4 hPGCLC. (C) FACS analysis of BTAG fluorescence levels of TRA-1-85[-] cells (m220 feeder). (Left) DRAQ7[-] cells were sorted as viable cells and analyzed for TRA-1-85 expression. (Right) BTAG fluorescence of TRA-1-85[+] human cells and TRA-1-85[-]m220 feeder. TRA-1-85[-]m220 feeder shows weak autofluorescence and are plotted diagonally under BT[+]AG[+] cells when sorted by BTAG fluorescence.

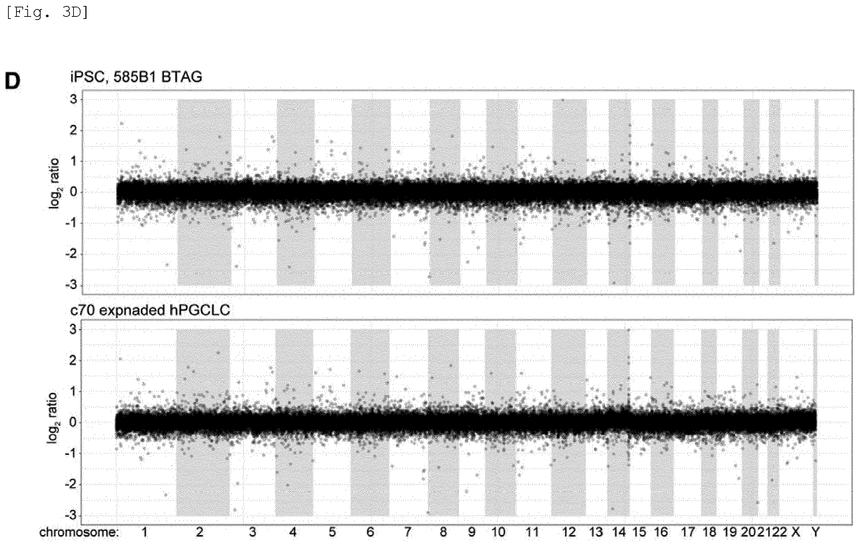

[Fig. 3D] Long-term expansion of BT[+]AG[+] cells from d4 hPGCLC. (D) Karyotype analysis based on array comparative genomic hybridization (aCHG) of parent 585B1 BTAG hiPSC (top) and BT[+]AG[+] cells (bottom) at c70 of expansion culture, showing that the karyotype is substantially maintained in BT[+]AG[+] cells at c70.

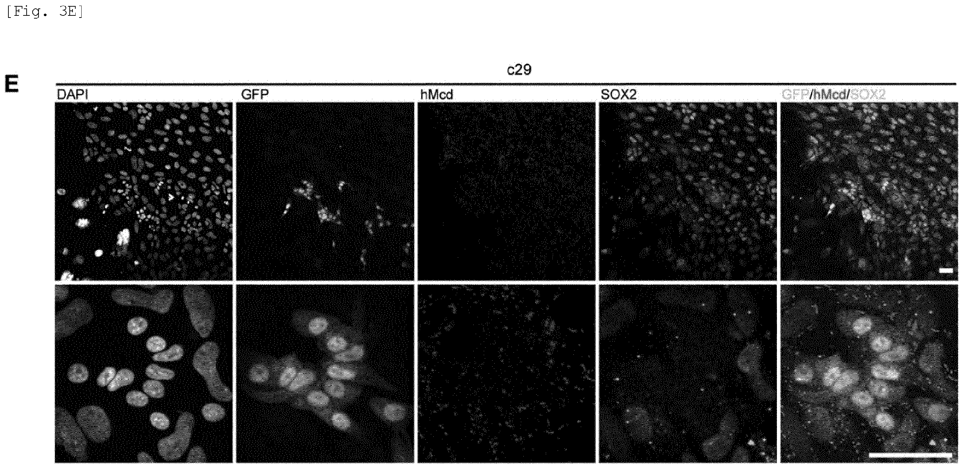

[Fig. 3E] Long-term expansion of BT[+]AG[+] cells from d4 hPGCLC. (E) IF analysis of GFP(AG), human mitochondria antigen (hMcd), and SOX2 expression in expansion culture of BT[+]AG[+] cells at c29. AG[-] cells in culture expressed SOX2 and were developed together with AG[+]/SOX2[-] cells. Scale bar, 50 $\mu$m.

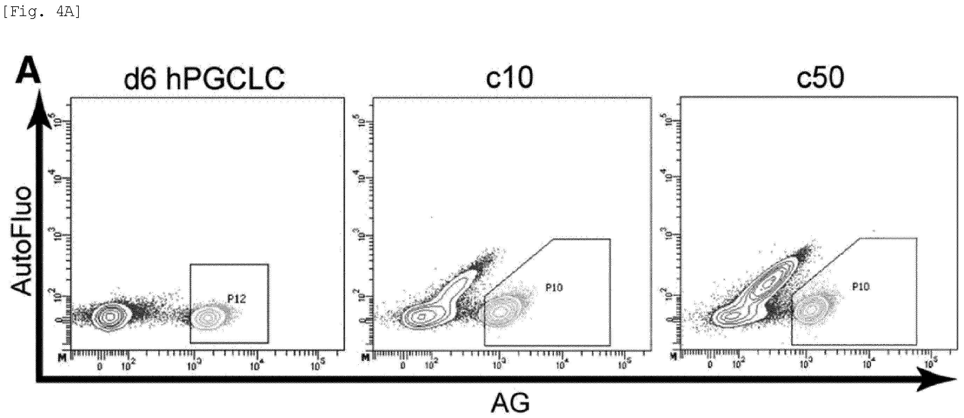

[Fig. 4A] Expansion of hPGCLC from independent hiPSC by surface marker selection. (A) FACS plot of AG expression in culture generated from AG[+] cells at d6 of hPGCLC induction, and having AG positivity at c10 and c50.

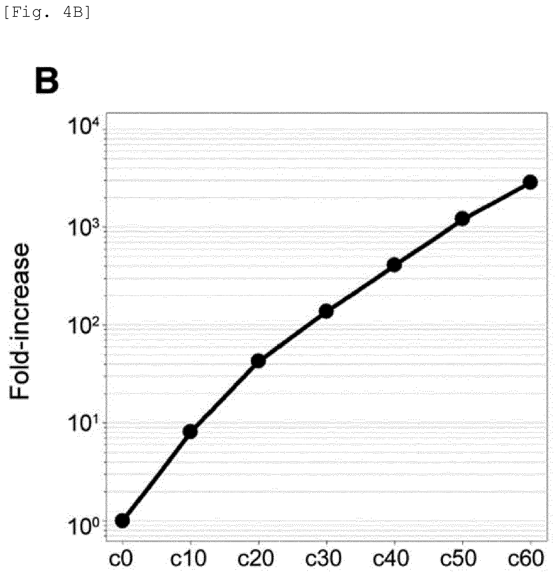

[Fig. 4B] Expansion of hPGCLC from independent hiPSC by surface marker selection. (B) Growth curve of AG[+] cells indicated by $\log_{10}$ (fold increase) during expansion of AG[+] cells up to c60. 10,000 AG[+] cells at d6 of hPGCLC induction were used as a starting cell population, and 10,000 AG[+] cells were replated in each passage.

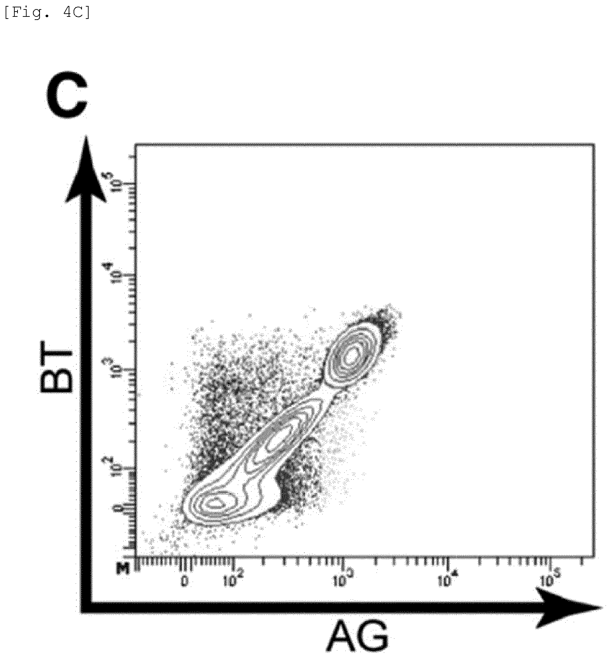

[Fig. 4C] Expansion of hPGCLC from independent hiPSC by surface marker selection. (C) FACS plot of BTAG expression of cultures similar to (B) at c60.

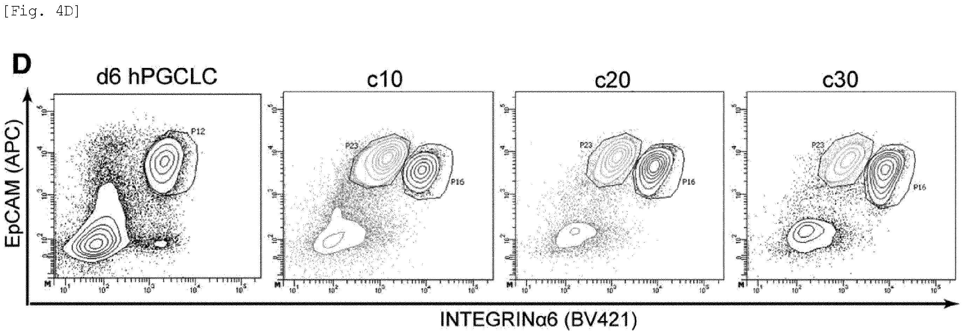

[Fig. 4D] Expansion of hPGCLC from independent hiPSC by surface marker selection. (D) FACS plot of INTEGRIN$\alpha$ and EpCAM expression in cultures generated from INTEGRIN$\alpha$[high]/EpCAM[high] cells (shown in blue) in d6 of hPGCLC induction, and having INTEGRIN$\alpha$[high]/EpCAM[high] expression, and passaged at c10, 20 and 30. INTEGRIN$\alpha$[low]/EpCAM[high] cells that emerged during culture are shown in yellow.

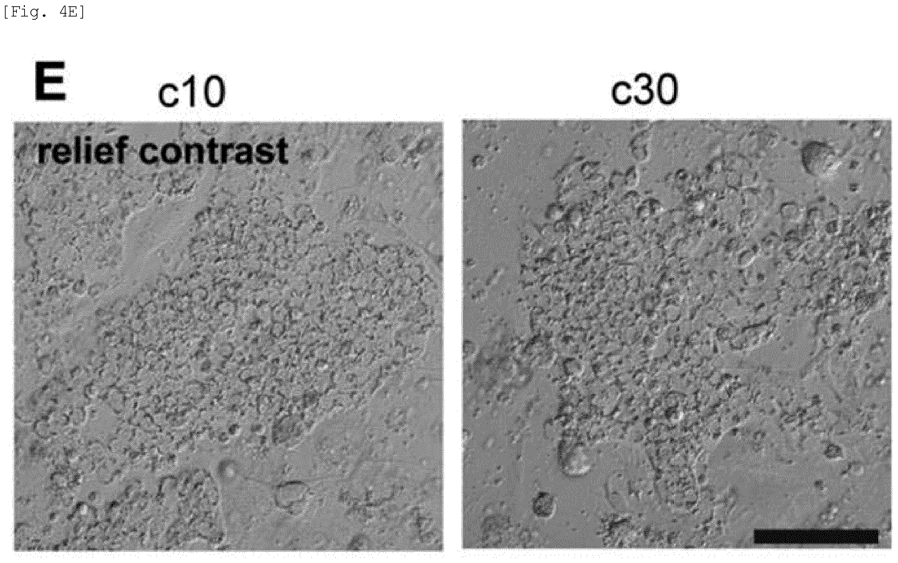

[Fig. 4E] Expansion of hPGCLC from independent hiPSC by surface marker selection. (E) Relief contrast image of cultures similar to (D) at c10 and c30. Scale bar, 200 $\mu$m.

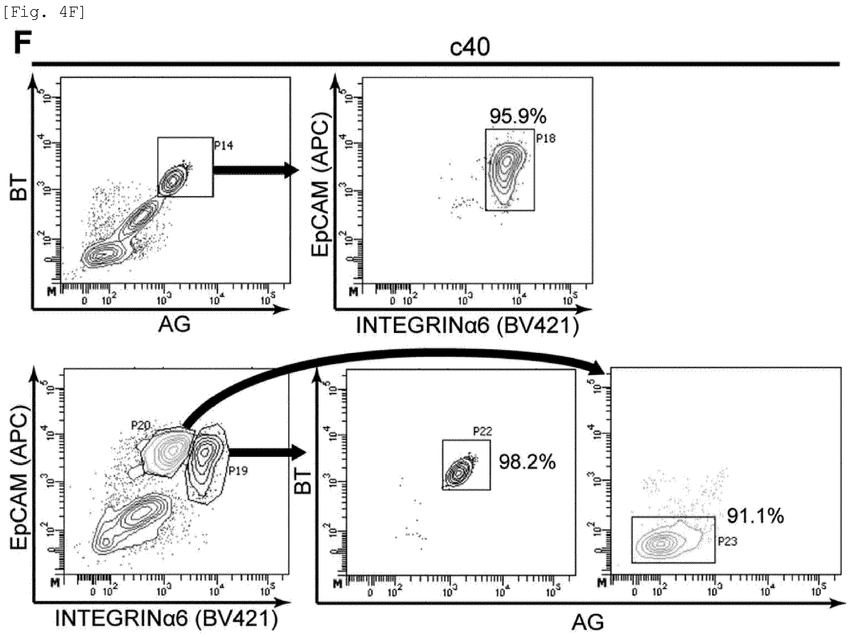

[Fig. 4F] Expansion of hPGCLC from independent hiPSC by surface marker selection. (F) (Top) expression pattern of INTEGRIN$\alpha$ and EpCAM in BT$^+$AG$^+$ cells (derived from 585B1 BTAG hiPSC) at c40 during BT$^+$AG$^+$ cell expansion culture. (Bottom) expression pattern of BTAG in INTEGRINa6$^{high}$/EpCAM$^{high}$ (blue) or INTEGRINa6$^{low}$/EpCAM$^{high}$ (yellow) cells at c40 (see Fig. 3C).

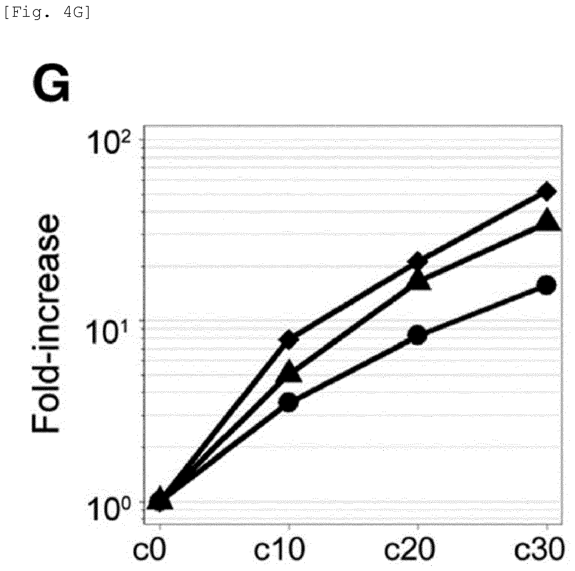

[Fig. 4G] Expansion of hPGCLC from independent hiPSC by surface marker selection. (G) Growth curve of INTE-GRIN$\alpha^{high}$/EpCAM$^{high}$ cells indicated by $\log_{10}$ (fold increase) during expansion of the cells up to c30 (triplicate experiments). 10,000 cells in d6 of hPGCLC induction were used as a starting cell population and 10,000 cells were replated in each passage.

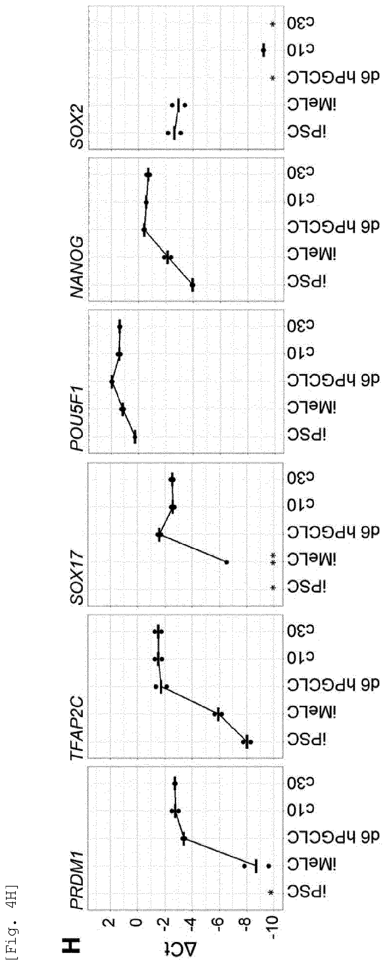

[Fig. 4H] Expansion of hPGCLC from independent hiPSC by surface marker selection. (H) qRNA analysis of the expression of indicated gene during hPGCLC induction and INTEGRIN$\alpha^{high}$/EpCAM$^{high}$ cell expansion culture. For respective genes tested, $\Delta$Ct from the average Ct values of two independent housekeeping genes, RPLPO and PPIA (set as 0), was calculated and plotted for two independent experiments. The average values were connected with a line. *: Not detected or $\Delta$Ct <-10. **. Detected in only one experiment.

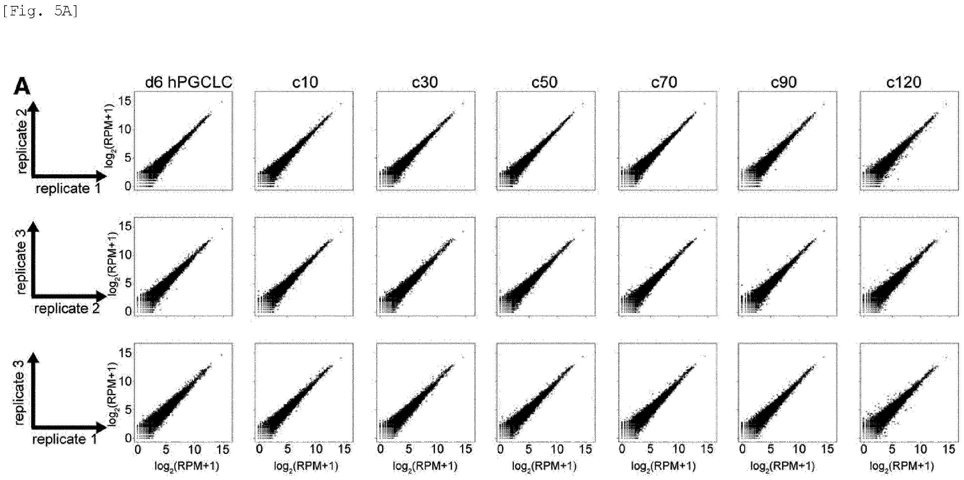

[Fig. 5A] Transcriptome analysis and expression of key genes during hPGCLC induction, BT$^+$AG$^+$ cell expansion, and differentiation in xr ovary (xrOvaries). (A) Comparison of dispersion plots of transcriptomes between the indicated replicates of BT$^+$AG$^+$ cells in expansion culture.

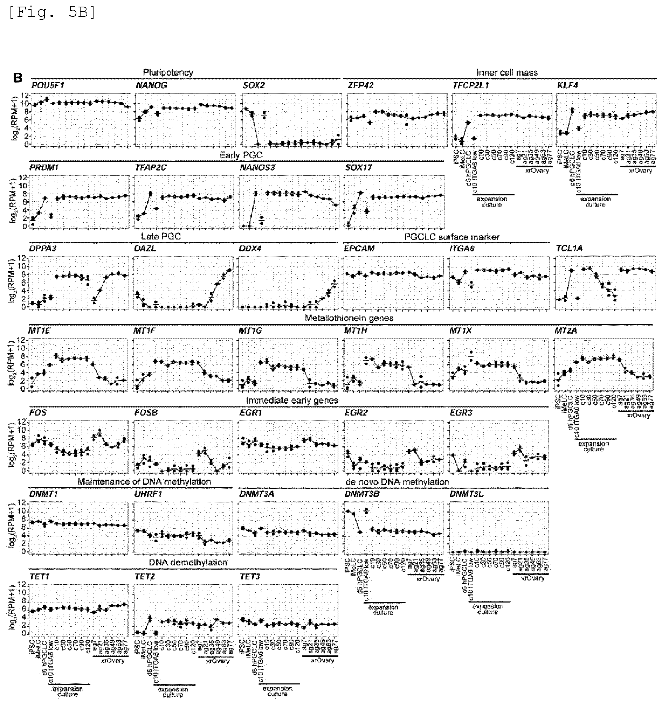

[Fig. 5B] Transcriptome analysis and expression of key genes during hPGCLC induction, BT$^+$AG$^+$ cell expansion, and differentiation in xr ovary (xrOvaries). (B) Expression dynamics measured by RNA-seq analysis of the indicated gene to be the key in the indicated cell type. For the respective indicated genes, $\log_2$(RPM+1) values derived from three replicates for hiPSC, iMeLC, d6 hPGCLC, and c10 - c120 BT$^+$AG cells, and two replicates ag7 - ag77 BT$^+$AG$^+$ cells (excluding ag49 BT$^+$AG$^+$ cells having one replicate) are shown as line-connected average values. c10ITGA6$^{low}$ cells are derived from 1383D6 hiPSC, while all other cell types are derived from 585B1 BTAG hiPSC.

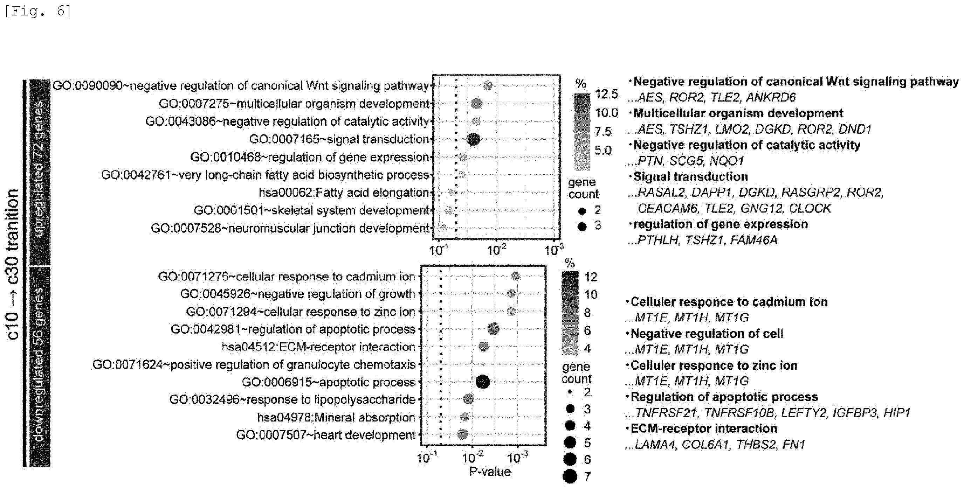

[Fig. 6] Transcriptome analysis of BT$^+$AG$^+$ cells during expansion culture. GO enrichments and representative genes in genes differentially expressed between c10 and c30 BT$^+$AG$^+$ cells. The colors mean as shown therein.

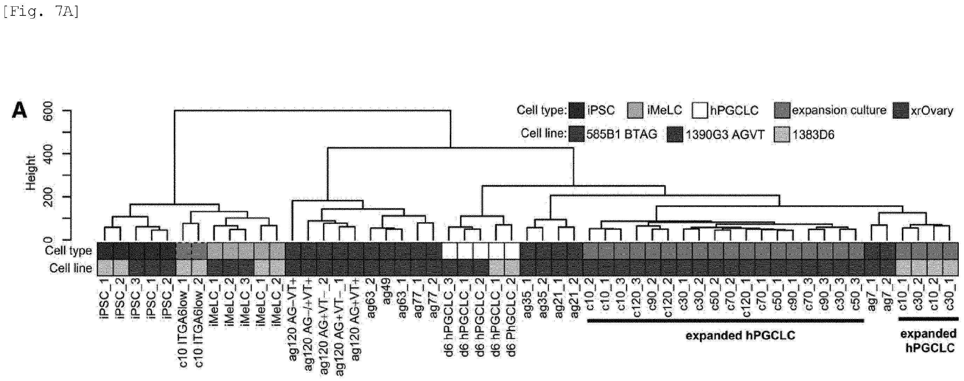

[Fig. 7A] Transcriptome analysis of BT$^+$AG$^+$ cells during expansion culture. (A) Unsupervised hierarchical clustering (UHC) of transcriptome of BT$^+$AG$^+$ cells during expansion culture of BT$^+$AG$^+$ cells with relevant cell types [hiPSC, iMeLC, d6 hPGCLC, and d6 hPGCLC-derived cells cultured in xrOvaries (Yamashiro et al., 2018)]. The original hiPSC line and cellular state used are shown by color. The number below the cellular state indication shows the number of replicates. c: culture days during expansion culture; ag: culture days of aggregation culture in xrOvaries

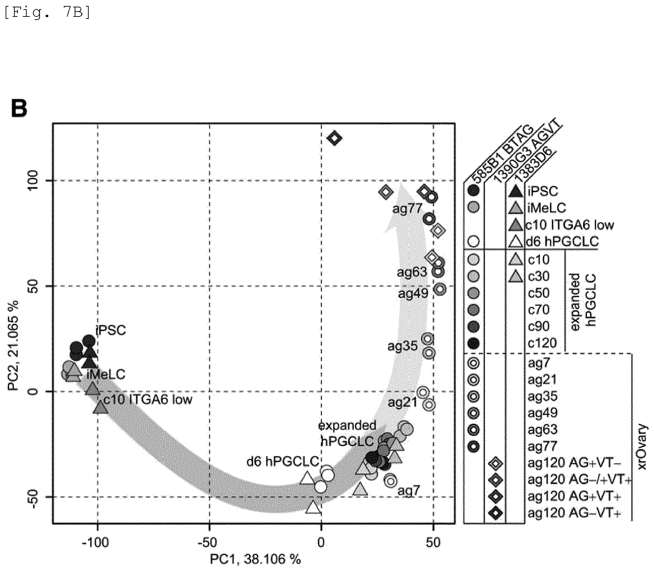

[Fig. 7B] Transcriptome analysis of BT$^+$AG$^+$ cells during expansion culture. (B) Principal component analysis of transcriptome of cells similar to (A) (PCA). Cells were plotted on a two-dimensional flat plane defined by PC1 value and PC2 value. The colors mean as shown therein.

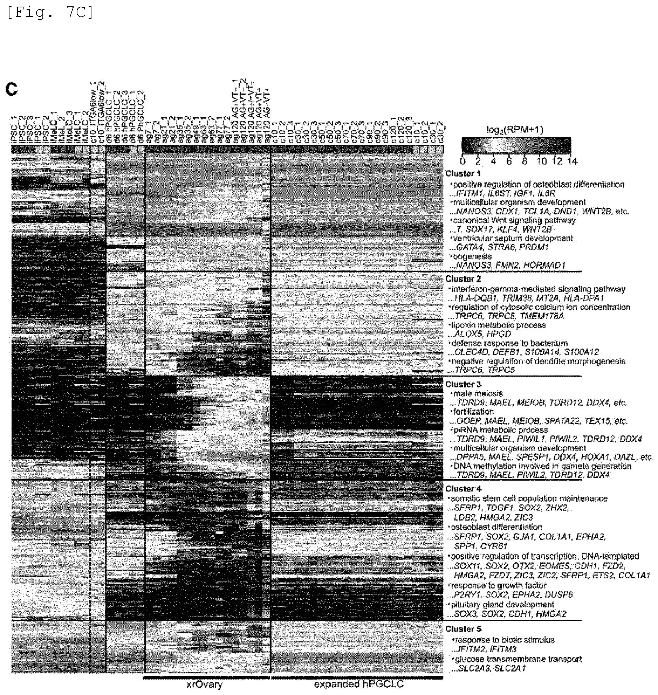

[Fig. 7C] Transcriptome analysis of BT$^+$AG$^+$ cells during expansion culture. (C) Heat map display of gene expression characterizing differentiation process of oogonia-/gonocyte-like cells derived from hiPSC in cells similar to (A) (genes in clusters 1 - 5; Yamashiro et al., 2018). Representative genes and gene ontology (GO) functional term enrichments in respective clusters are shown on the right. The colors mean the same as those shown in (A).

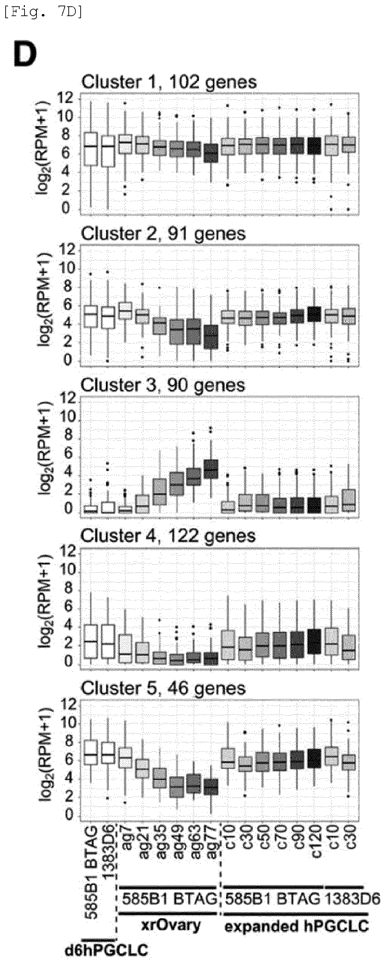

[Fig. 7D] Transcriptome analysis of BT$^+$AG$^+$ cells during expansion culture. (D) Box plot analysis of average expression level of genes in clusters 1 - 5 of (C) in the indicated cell type. Boxes show the 25th and 75th percentiles, and bars indicate median values.

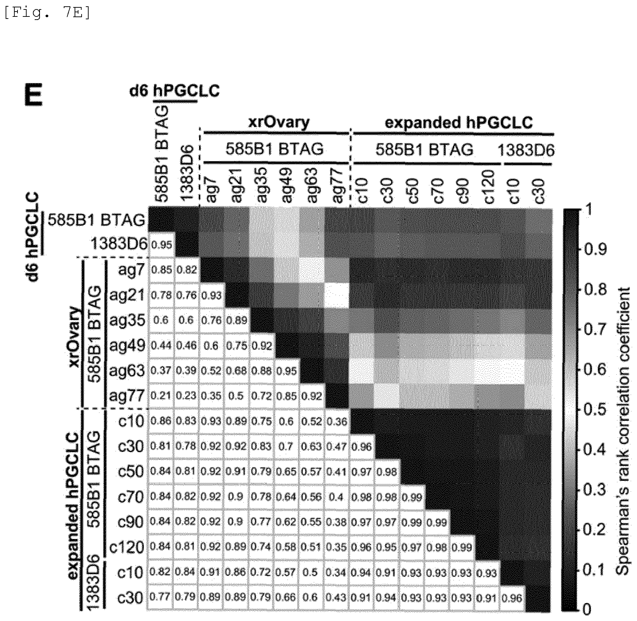

[Fig. 7E] Transcriptome analysis of BT$^+$AG$^+$ cells during expansion culture. (E) Spearman's rank correlation analysis of gene expression profiles of clusters 1-5 of (C) among the indicated cell types. The colors mean as shown therein.

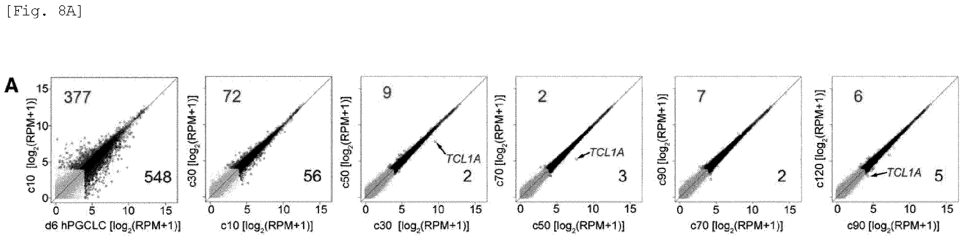

[Fig. 8A] Genes (DEG) differentially expressed between BT$^+$AG$^+$ cells during expansion culture of the BT$^+$AG$^+$ cells. (A) Comparison of transcriptome scatter-plots between the indicated cell types during expansion of BT$^+$AG$^+$ cells from d6 hPGCLCs. Genes upregulated and downregulated [$\log_2$(RPM+1)$\geq$4, $\log_2$ (fold change)$\geq$1] in cell type on the Y-axis are shown in red and blue, respectively.

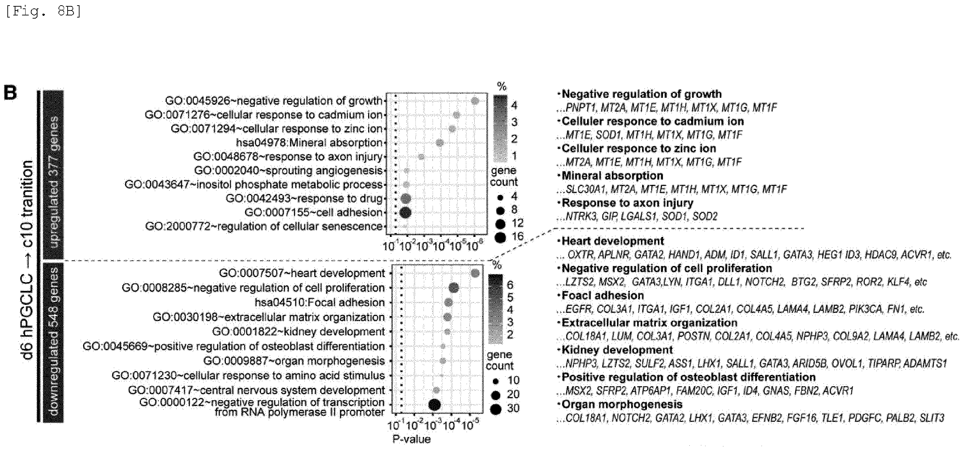

[Fig. 8B] Genes (DEG) differentially expressed between BT$^+$AG$^+$ cells during expansion culture of the BT$^+$AG$^+$ cells. (B) GO enrichments in genes differentially expressed between d6 hPGCLC and c10 BT$^+$AG$^+$ cells and representative genes of DEG. The colors mean as shown therein.

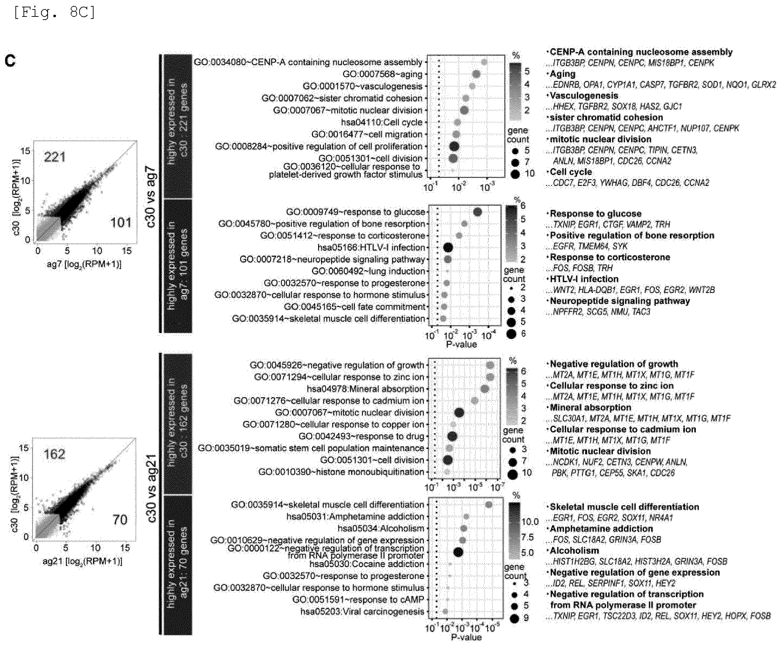

[Fig. 8C] Genes (DEG) differentially expressed between BT$^+$AG$^+$ cells during expansion culture of the BT$^+$AG$^+$ cells. (C) (Left) Comparison of scatter-plots between c30 of BT$^+$AG$^+$ cells and ag7 (top) or ag21 (bottom). The colors mean as shown therein in (A). (Right) GO enrichments and representative genes in genes differently expressed between BT$^+$AG$^+$ cells between c30 and ag7 (top) or ag21 (bottom). The colors mean as shown therein.

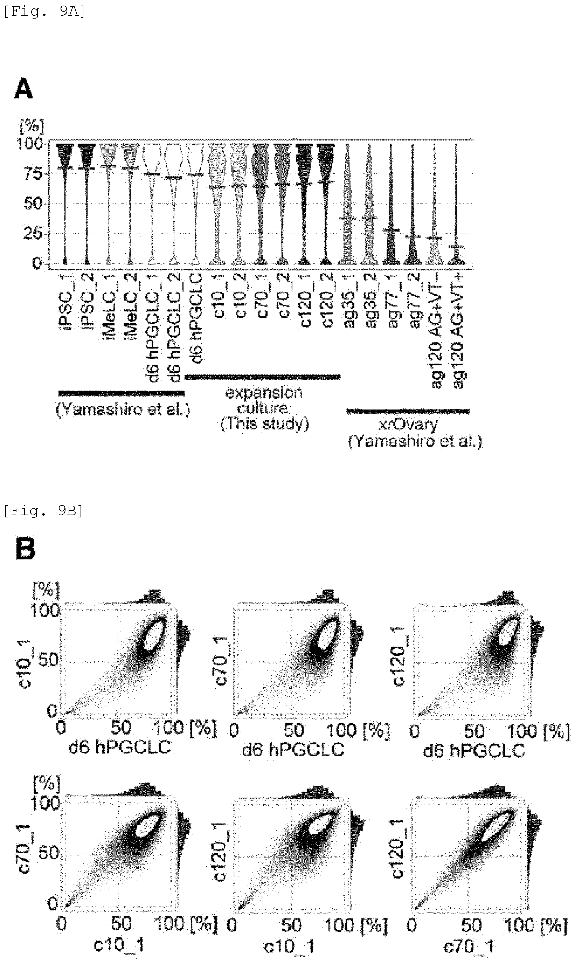

[Fig. 9A] Genome-wide methylation profile of BT$^+$AG$^+$ cells during expansion culture of the BT$^+$AG$^+$ cells. (A) Violin-plot of genome-wide DNA methylation profile level [5-methylcytosine (5mC) level], measured by whole-genome bisulfite sequence (WGBS) analysis in BT$^+$AG$^+$ cells during expansion culture of BT$^+$AG$^+$ cells and the indicated relevant cell types. Average levels are indicated by red bars.

[Fig. 9B] Genome-wide methylation profile of BT$^+$AG$^+$ cells during expansion culture of the BT$^+$AG$^+$ cells. (B) Comparison of scatter-plots combining genome-wide 5mC level (genome-wide 2kb window) with histogram representation (top and right sides of the scatter-plot) between the indicated cell types.

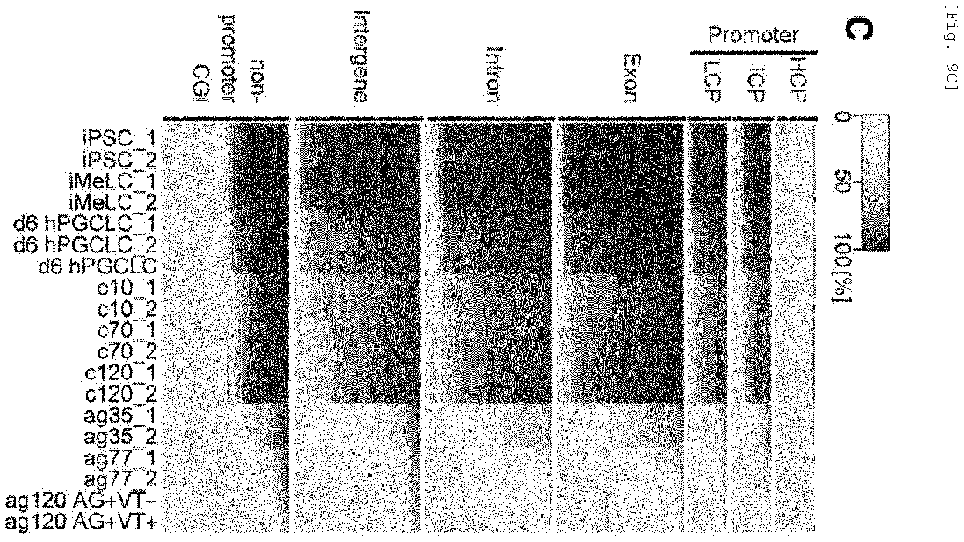

[Fig. 9C] Genome-wide methylation profile of BT+AG+ cells during expansion culture of the BT+AG+ cells. (C) Heat map display showing 5 mC level in the indicated genomic element on autochromosome in the indicated cell type. HCP/ICP/LCP: high/intermediate/low CpG promoter. The colors mean as shown therein.

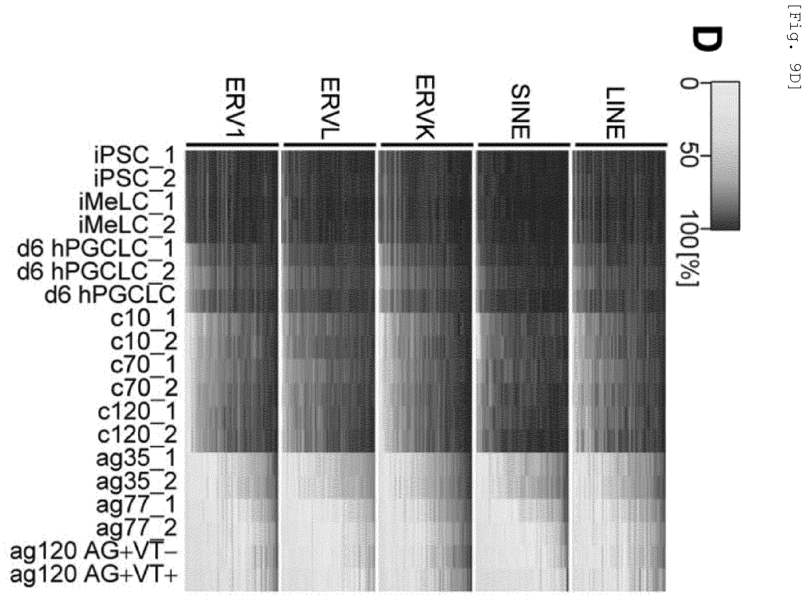

[Fig. 9D] Genome-wide methylation profile of BT+AG+ cells during expansion culture of the BT+AG+ cells. (D) Heat map display showing 5 mC level in the indicated repeat element on autochromosome in the indicated cell. Repeated elements with repStart≤5 and repEnd>5 kb for LINE, ERVK, ERVL, and ERV1, and repStart≤5 and repEnd≥310 for SINE were used to exclude shortened elements.

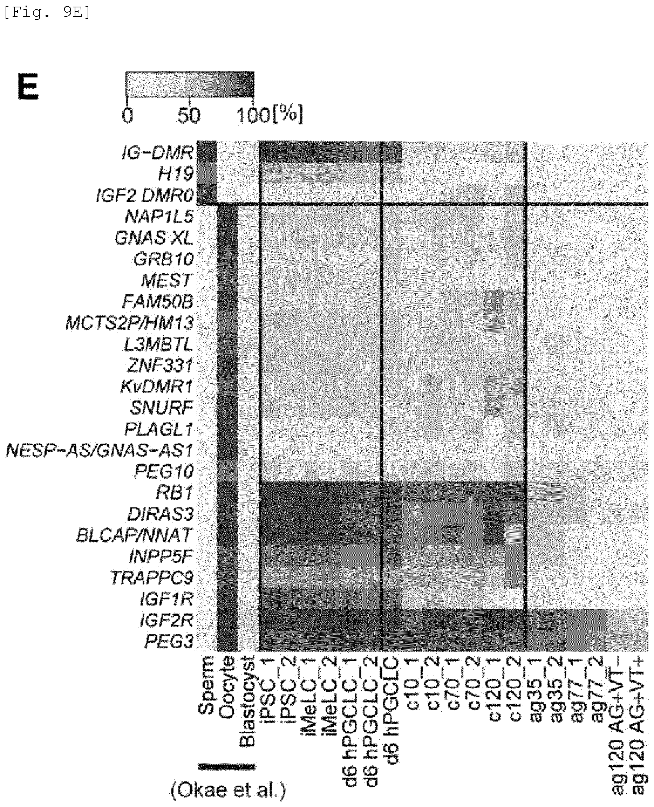

[Fig. 9E] Genome-wide methylation profile of BT+AG+ cells during expansion culture of the BT+AG+ cells. (E) Heat map display showing 5mC level in differentially methylated regions of the indicated imprinted genes in the indicated cells.

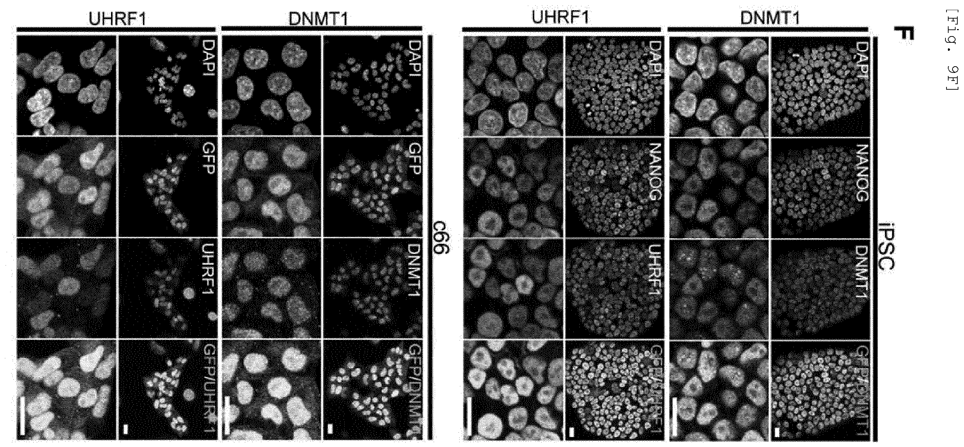

[Fig. 9F] Genome-wide methylation profile of BT+AG+ cells during expansion culture of the BT+AG+ cells. (F) IF analysis of DNMT1 or UHRF1 expression in hiPSC and c66 BT+AG+ cells. hiPSC was co-stained with NANOG; c66 BT+AG+ cells were co-stained with GFP (AG); and both cell types were counterstained with DAPI. The bottom panel is an enlarged view of the top panel. Scale bar, 20 μm.

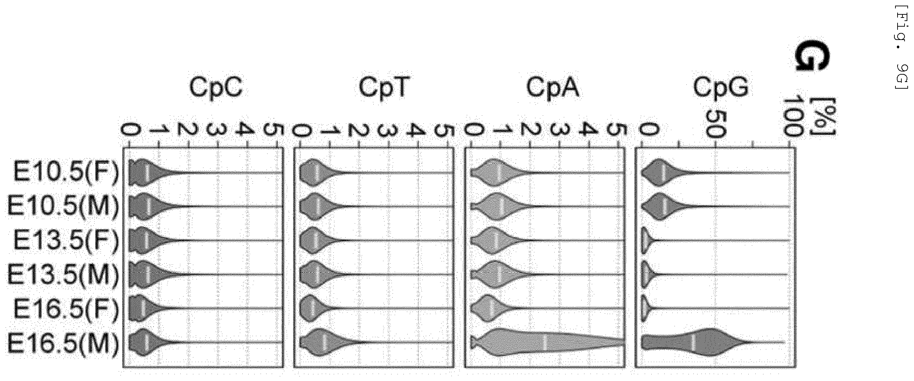

[Fig. 9G] Genome-wide methylation profile of BT+AG+ cells during expansion culture of the BT+AG+ cells. (G) Violin-plot showing genome-wide 5 mC level in CpG, CpA, CpT, and CpC sequences in mouse germ cells in vivo. Median levels are indicated by yellow bars. E: embryogenic day; F: female; M: male. c: culture days in expansion culture; ag: culture days in aggregation culture in xrOvaries.

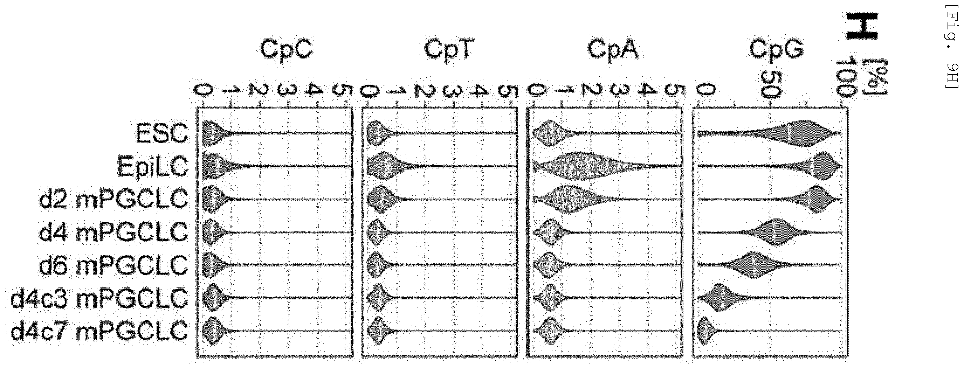

[Fig. 9H] Genome-wide methylation profile of BT+AG+ cells during expansion culture of the BT+AG+ cells. (H) Violin-plot showing genome-wide 5 mC level in CpG, CpA, CpT, and CpC sequences in cells during in vitro mPGCLC induction and expansion. Median levels are indicated by yellow bars. c: culture days in expansion culture; ag: culture days in aggregation culture in xrOvaries.

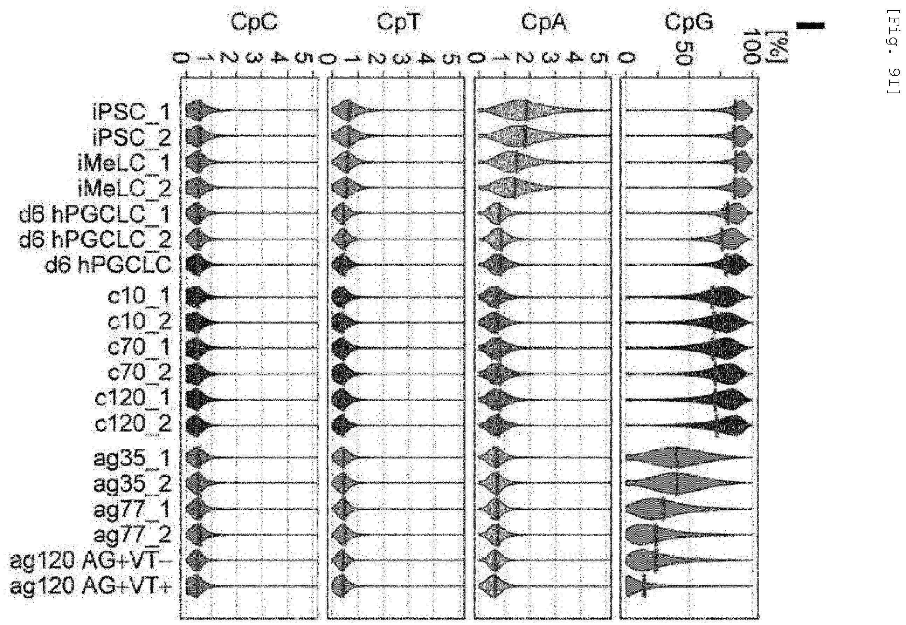

[Fig. 9I] Genome-wide methylation profile of BT+AG+ cells during expansion culture of the BT+AG+ cells. (I) Violin-plot showing genome-wide 5 mC level in CpG, CpA, CpT, and CpC sequences in cells during hPGCLC induction, expansion, and differentiation in xrOvaries in vitro. Median levels are indicated by red bars. The number below the cellular state indication shows the number of replicates. c: culture days in expansion culture; ag: culture days in aggregation culture in xrOvaries.

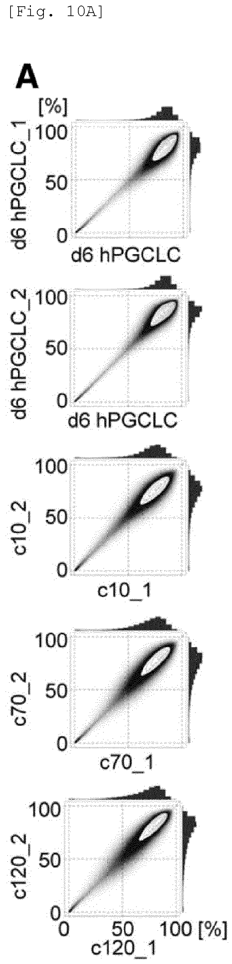

[Fig. 10A] Genome-wide DNA methylation profile of BT+AG+ cells during expansion culture. (A) Comparison of scatter-plots of DNA methylation profile between the indicated replicates of d6 hPGCLC/BT+AG+ cells during expansion culture.

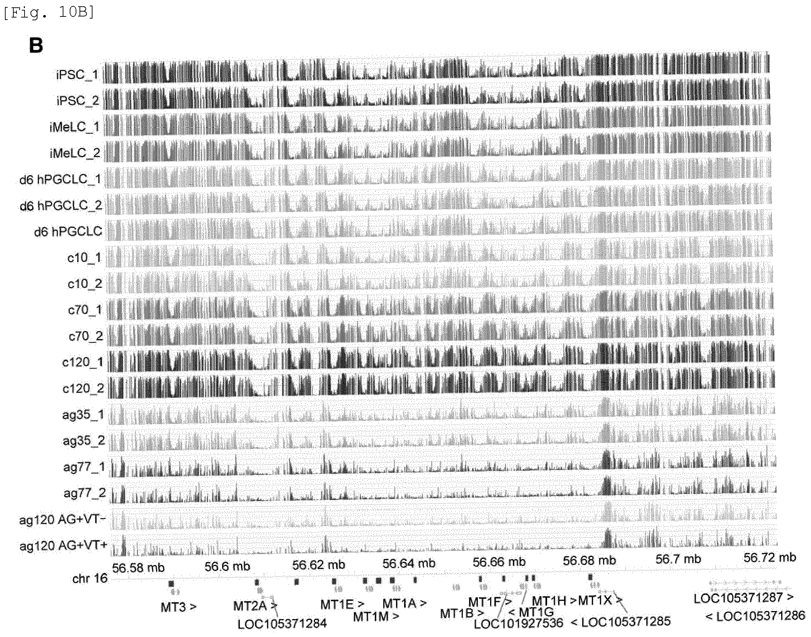

[Fig. 10B] Genome-wide DNA methylation profile of BT+AG+ cells during expansion culture. (B) DNA methylation profile near MT1 gene cluster in cells during hPGCLC induction, expansion, and differentiation in xrOvaries.

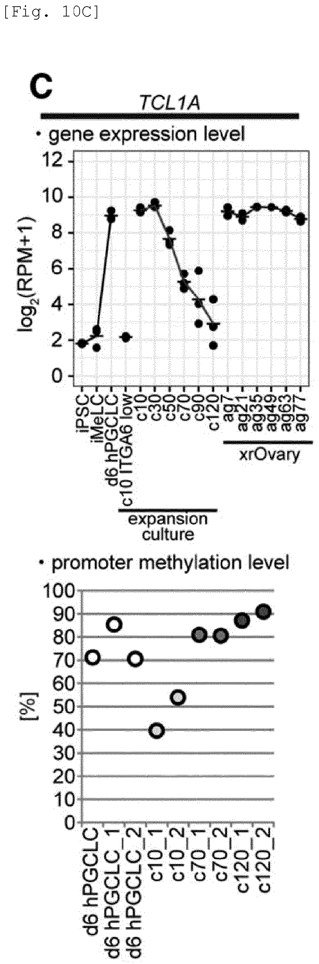

[Fig. 10C] Genome-wide DNA methylation profile of BT+AG+ cells during expansion culture. (C) 5 mC level in TCL1A promoter in (top) Expression dynamics measured by RNA-seq analysis of TCL1A in the indicated cell type also seen in Fig. 5, and (bottom) BT+AG+ cells of d6 PGCLC (three replicates) and c10, c70, and c120 (two replicates, each).

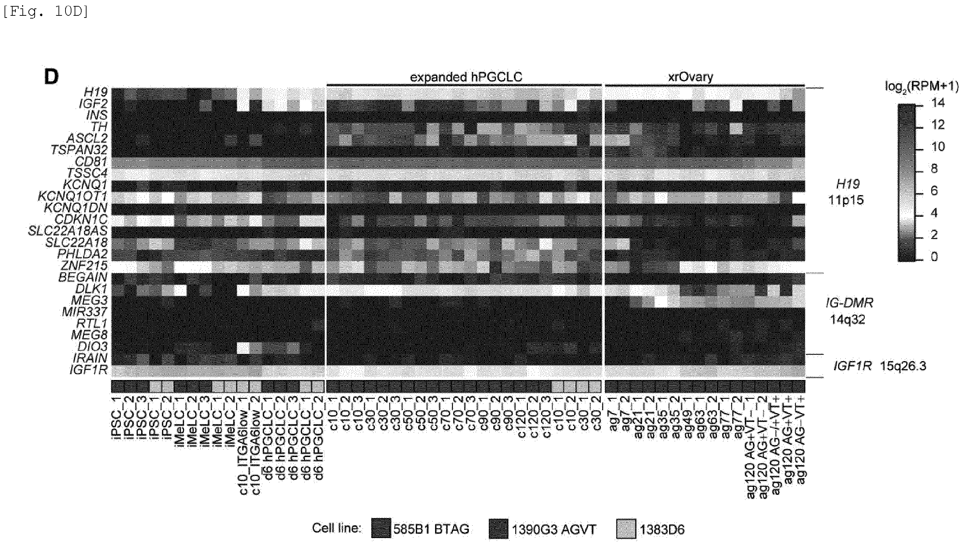

[Fig. 10D] Genome-wide DNA methylation profile of BT+AG+ cells during expansion culture. (D) Heat map display of indicated, imprinted gene expression under control of imprint control region (ICR) of H19 (chromosomal location: 11p15), IG-DMR (14q32), and IGF 1R (15q26.3) in the indicated cell type. The number below the cellular state indication shows the number of replicates. The colors mean as shown therein. H19 expression level in d6c120 cells is significantly upregulated as compared with the expression levels in d6 hPGCLC (Welch's t-test, P = 0.009). Imprinted gene was obtained from the Catalogue of Parent of Origin Effects website (http://igc.otago.ac.nz/home.html; Morison et al, 2005).

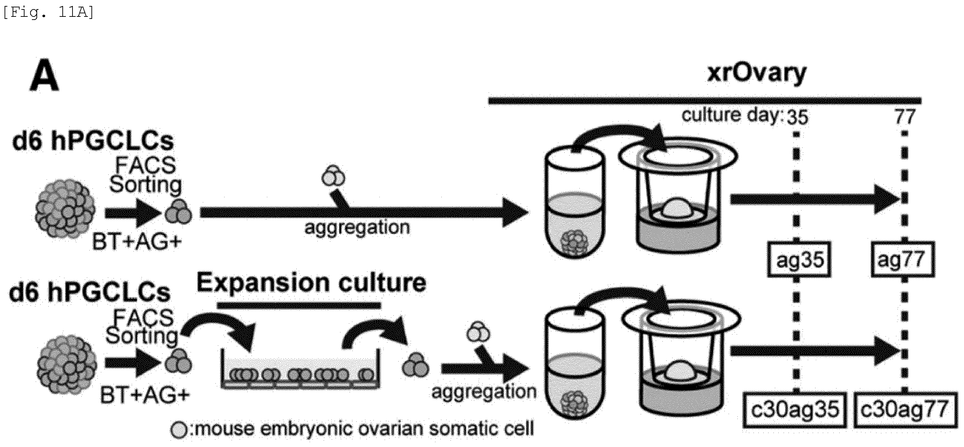

[Fig. 11A] Expanded BT+AG+ cell differentiates into oogonia-/gonocyte-like cell in xrOvaries. (A) Scheme for producing xrOvaries by d6 hPGCLC (top) or d6c30 BT+AG+ cells (bottom).

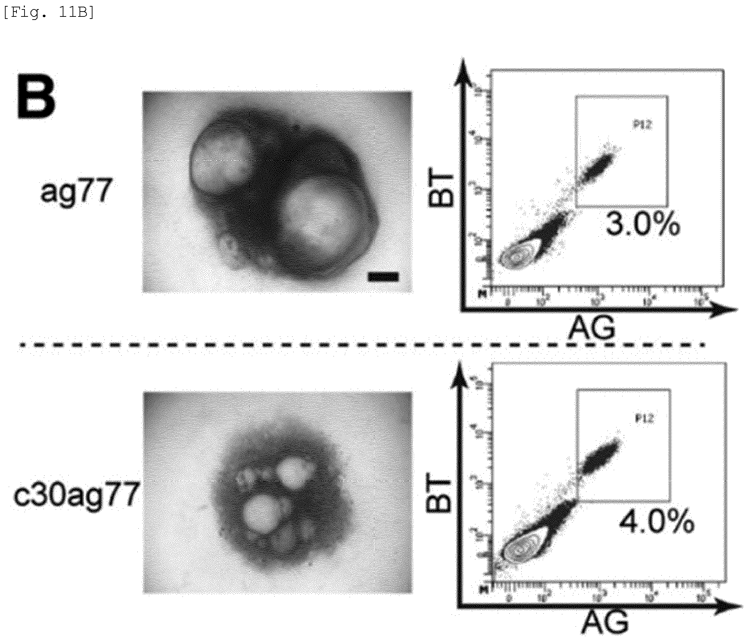

[Fig. 11B] Expanded BT+AG+ cell differentiates into oogonia-/gonocyte-like cell in xrOvaries. (B) Bright field image and FACS plot of BTAG expression in cells of xr ovaries in ag77 by d6 hPGCLC (top) or d6c30 BT+AG+ cells (bottom). Scale bar, 200 um.

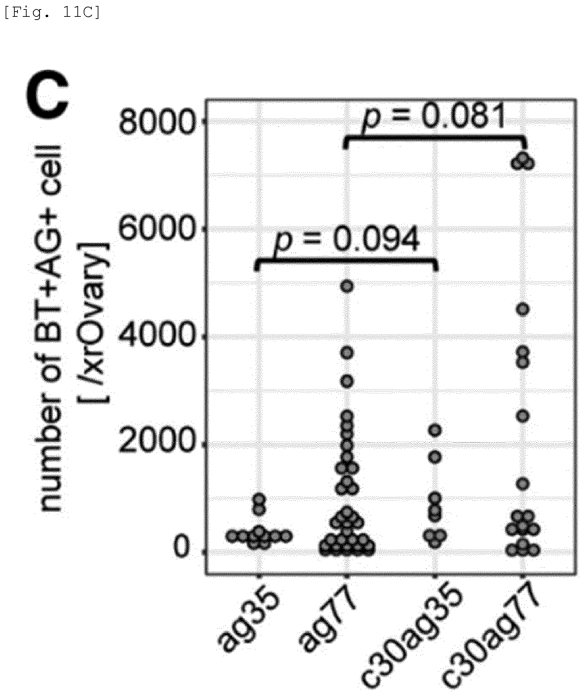

[Fig. 11C] Expanded BT+AG+ cell differentiates into oogonia-/gonocyte-like cell in xrOvaries. (C) Number of BT+AG+ cells in xrOvaries of ag35 and ag77 derived from d6 hPGCLC or d6c30 BT+AG+ cells. P value was calculated by Welch's t-test.

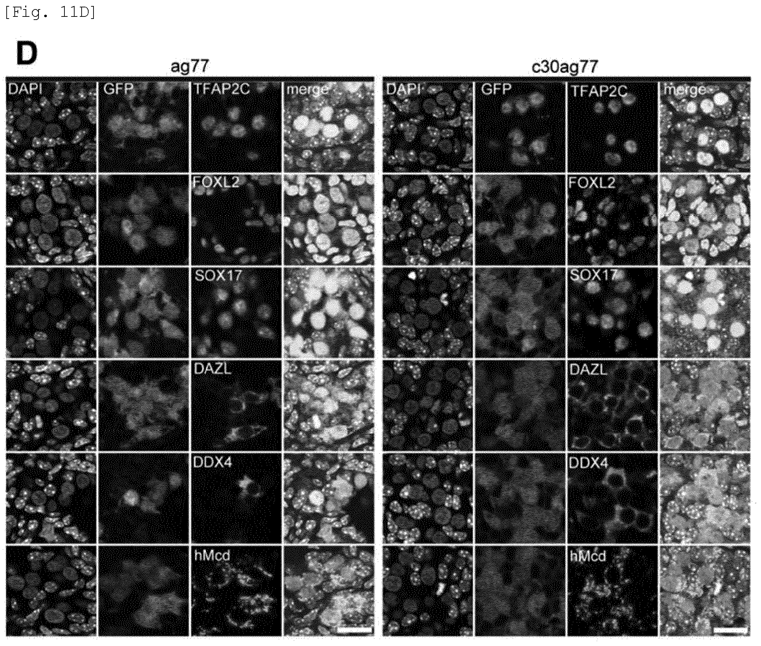

[Fig. 11D] Expanded BT+AG+ cell differentiates into oogonia-/gonocyte-like cell in xrOvaries. (D) IF analysis of TFAP2C, FOXL2, SOX17, DAZL, DDX4, and human mitochondria antigen (hMC; cyan) expression in AG+ (yellow) cells/mouse ovarian somatic cells in xrOvaries in ag77 by d6 hPGCLC (left) or d6c30 BT+AG+ cells (right). Cells were counter-stained with DAPI (white), and a merged image is shown on the right. Scale bar, 20 μm.

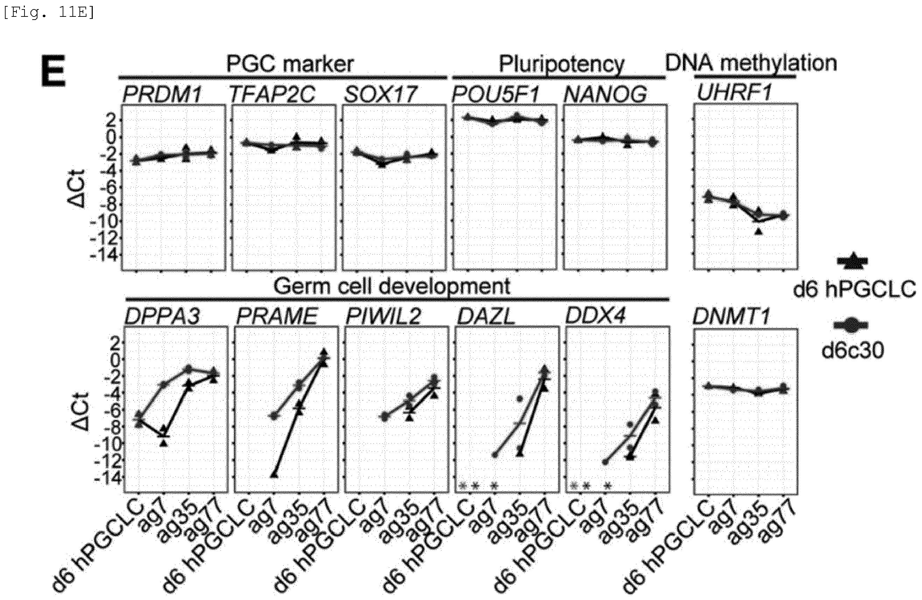

[Fig. 11E] Expanded BT+AG+ cell differentiates into oogonia-/gonocyte-like cell in xrOvaries. (E) qPCR analysis of expression of the indicated gene in BT$^+$AG$^+$ cells isolated from xrOvaries in ag7, ag35, and ag77 produced by d6 hPGCLC (black) or d6c30 BT$^+$AG$^+$ cells (red). For respective genes tested, $\Delta$Ct from the average Ct values of two independent housekeeping genes, RPLPO and PPIA (set as 0), was calculated and plotted for two independent experiments. The average values were connected with a line. Point without an average bar indicates that gene expression was detected in only one of the two replicates. *: Not detected.

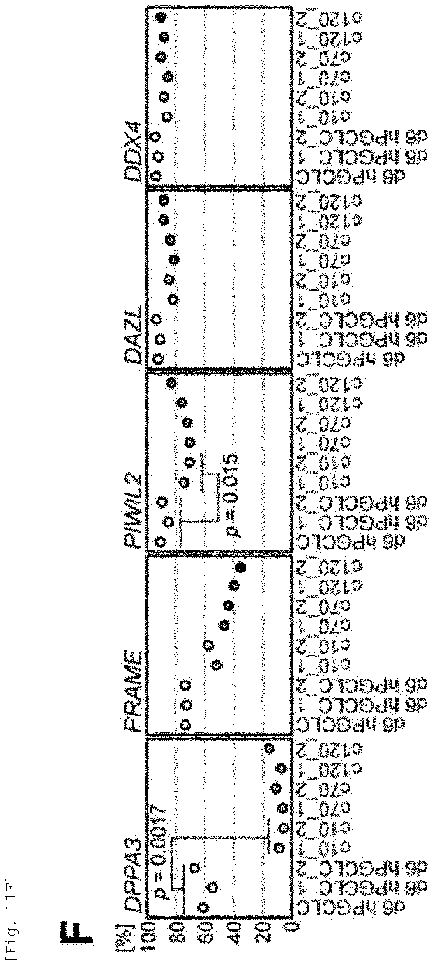

[Fig. 11F] Expanded BT$^+$AG$^+$ cell differentiates into oogonia-/gonocyte-like cell in xrOvaries. (F) 5 mC level of promoter of the indicated gene in the indicated cell type. The number below the cellular state indication shows the number of replicates. The average promoter methylation levels of DPPA3 and PIWIL2 in d6c10 cells significantly decreased as compared with that of d6 hPGCLC (Welch's t-test, P<0.05).

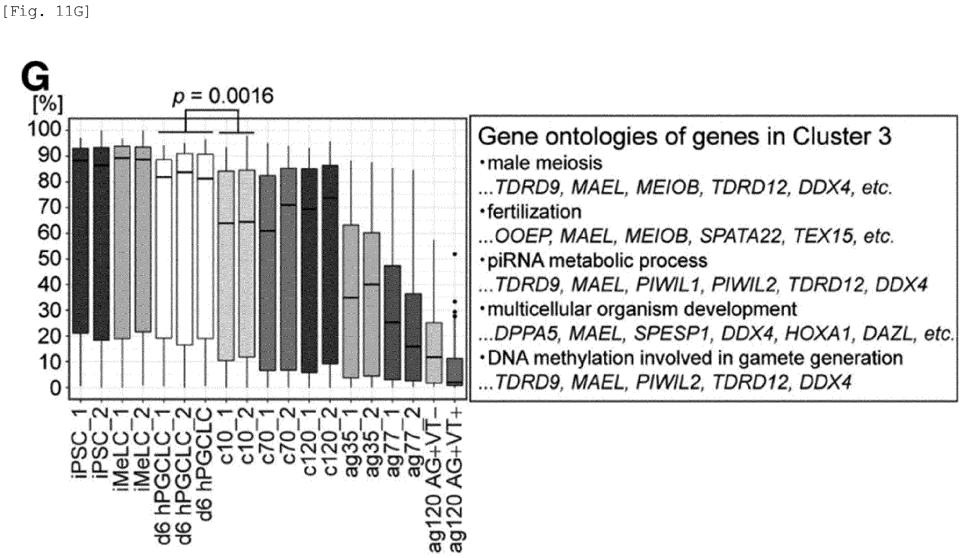

[Fig. 11G] Expanded BT$^+$AG$^+$ cell differentiates into oogonia-/gonocyte-like cell in xrOvaries. (G) Box plot analysis at 5 mC level of promoter of cluster 3 gene as in (Fig. 7C) in the indicated cell type. Boxes show the 25th and 75th percentiles, and bars indicate median values. The number below the cellular state indication shows the number of replicates. The average promoter methylation level of d6c10 cells significantly decreased as compared with that of d6 hPGCLC (Wilcoxon's rank-sum test, P<0.05).

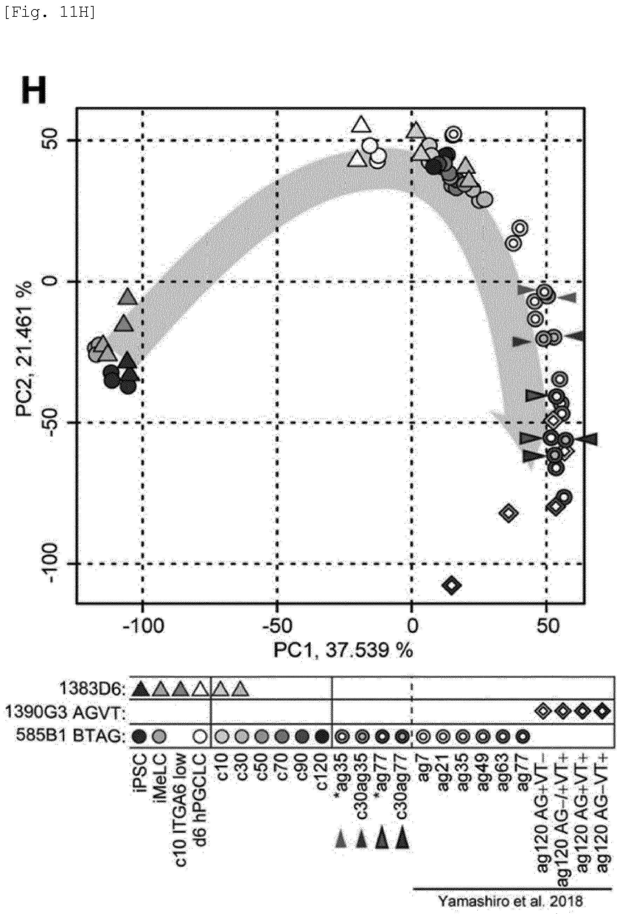

[Fig. 11H] Expanded BT$^+$AG$^+$ cell differentiates into oogonia-/gonocyte-like cell in xrOvaries. (H) PCA of transcriptome of BT$^+$AG$^+$ cells during expansion culture and differentiation of BT$^+$AG$^+$ cells in xrOvaries having the indicated relevant cell type. The asterisk (*) means the RNA-seq data of ag35 and ag77 cells produced in this study. In PCA, blue and red arrows indicate data for ag35/77 BT$^+$AG$^+$ cells derived from d6 hPGCLC and c30 BT$^+$AG$^+$ cells, respectively. The colors mean as shown therein.

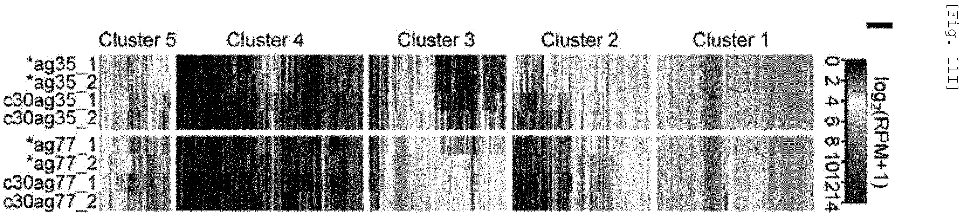

[Fig. 11I] Expanded BT$^+$AG$^+$ cell differentiates into oogonia-/gonocyte-like cell in xrOvaries. (I) Heat map display showing expression of genes in clusters 1-5 in Fig. 7C in BT$^+$AG$^+$ cells isolated from xrOvaries in ag35 and ag77 produced by d6 hPGCLC or d6c30 BT$^+$AG$^+$ cells. The colors mean as shown therein.

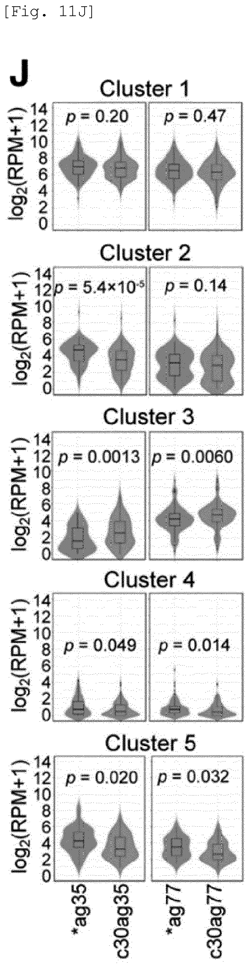

[Fig. 11J] Expanded BT$^+$AG$^+$ cell differentiates into oogonia-/gonocyte-like cell in xrOvaries. (J) Violin-plot showing average expression levels of genes in clusters 1 - 5 in (I) in the indicated cell type. Squares indicate the 25th and 75th percentiles, and bars indicate median values. As compared with cells derived from d6 hPGCLC, genes in cluster 3 gene shows remarkable upregulation, and clusters 4 and 5 show remarkable downregulation in cells derived from d6c30 BT$^+$AG$^+$ cells (Wilcoxon's rank-sum test, p<0.05).

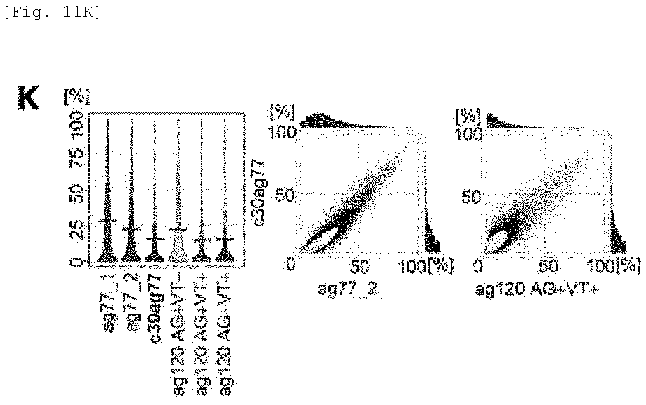

[Fig. 11K] Expanded BT$^+$AG$^+$ cell differentiates into oogonia-/gonocyte-like cell in xrOvaries. (K) (Left) Violin-plot at genome-wide 5mC level measured by whole-genome bisulfite sequence analysis in the indicated cell type. Average levels are indicated by red bars. (Right) Comparison of scatter-plots combining genome-wide 5mC level (genome-wide 2kb window) with histogram representation (top and right sides of the scatter-plot) between the indicated cell types.

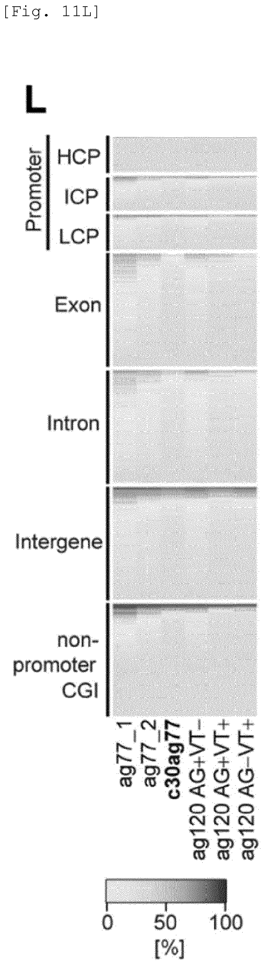

[Fig. 11L] Expanded BT$^+$AG$^+$ cell differentiates into oogonia-/gonocyte-like cell in xrOvaries. (L) Heat map display showing 5mC level in the indicated genomic elements of autochromosome in the indicated cells. The colors mean as shown therein.

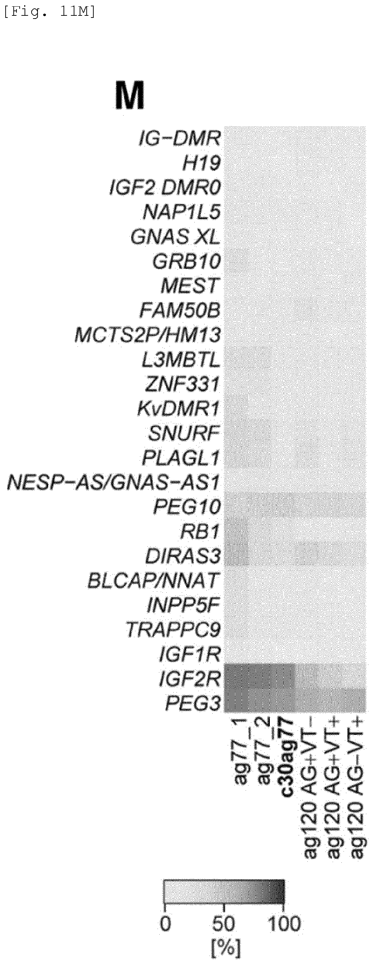

[Fig. 11M] Expanded BT$^+$AG$^+$ cell differentiates into oogonia-/gonocyte-like cell in xrOvaries. (M) Heat map display showing 5mC level in differentially methylated region of the indicated imprinted gene in the indicated cells. The colors mean as shown therein.

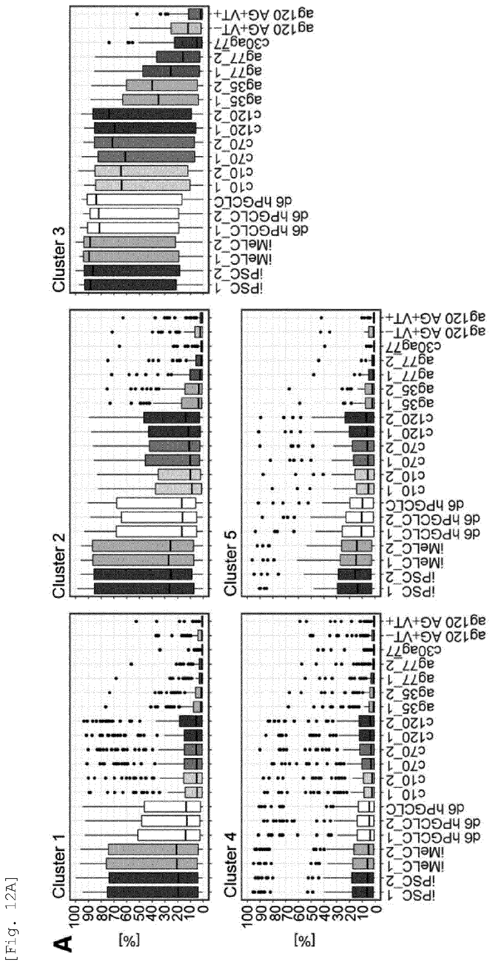

[Fig. 12A] BT$^+$AG$^+$ cells in maintenance and expansion culture differentiate into oogonium-/gonocyte-like cells in xrOvaries. (A) 5 mC levels of promoters of the gene group of clusters 1 - 5 in Fig. 7C in the indicated cell type are shown in a box plot. Boxes indicate the 25th to 75th percentiles, and lines within the box indicate median values. The number below the cellular state indication shows the number of replicates.

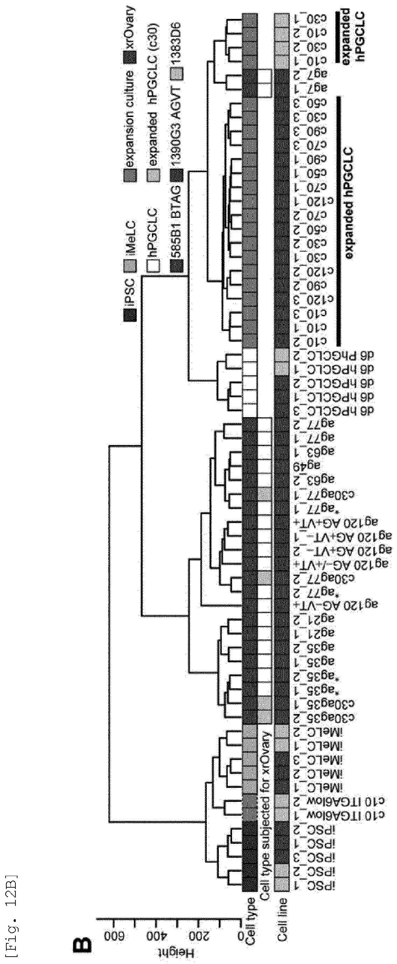

[Fig. 12B] BT$^+$AG$^+$ cells in maintenance and expansion culture differentiate into oogonium-/gonocyte-like cells in xrOvaries. (B) Unsupervised hierarchical clustering of transcriptomes of BT$^+$AG$^+$ cells during maintenance and expansion culture and during differentiation in xrOvaries is shown, along with the indicated relevant cell type. Euclidean distance and Ward's method were used. * indicates the RNA-seq data of ag35 and ag77 cells obtained in the present invention. Each color means as shown on the upper right.

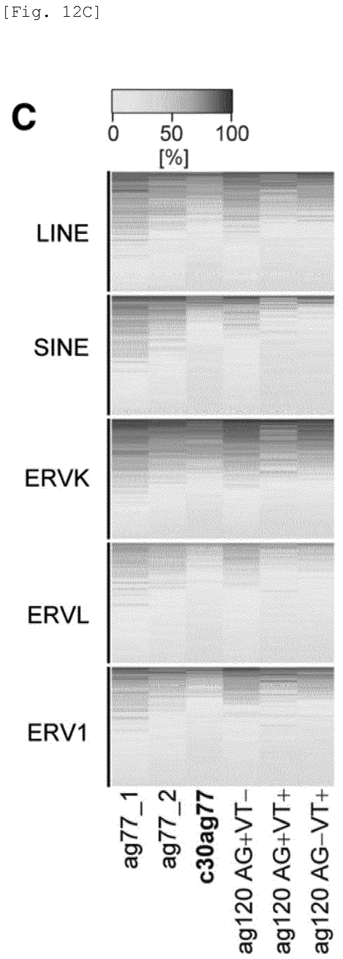

[Fig. 12C] BT$^+$AG$^+$ cells in maintenance and expansion culture differentiate into oogonium-/gonocyte-like cells in xrOvaries. (C) Heat map showing 5 mC level in the indicated repeat element on autochromosome in the indicated cell. Repeated elements with repStart $\leq$ 5 and repEnd> 5 kb for LINE, ERVK, ERVL and ERV1, and repStart $\leq$ 5 and repEnd $\geq$ 310 for SINE were used to exclude shortened elements.

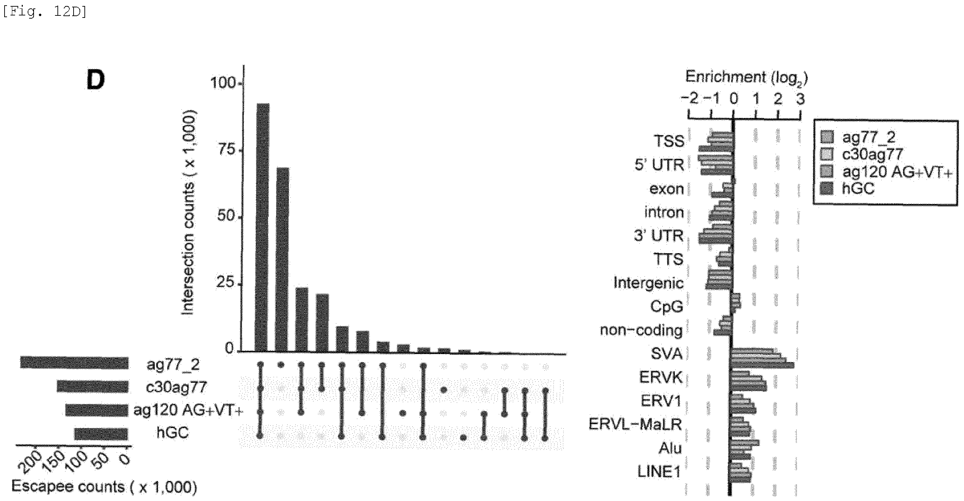

[Fig. 12D] BT$^+$AG$^+$ cells in maintenance and expansion culture differentiate into oogonium-/gonocyte-like cells in xrOvaries. (D) (Left) Upset plot showing the number of DNA demethylation "escape" (Example 1, <experimental method> see the "WGBS data processing and analysis" section) of the indicated cell types and the numbers common between them. hGC: 7-9 week human germ cells described in Tang et al, 2015. (Right) Enrichment of the indicated genomic element in DNA demethylation "escape" in the indicated cell type. TSS: transcription start site, UTR: untranslated region, TTS: transcription termination site.

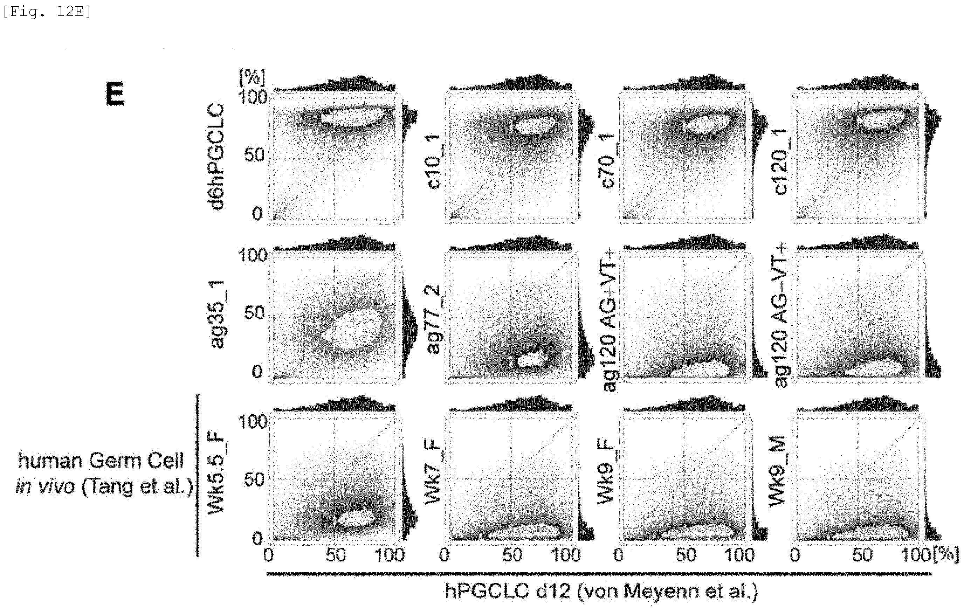

[Fig. 12E] BT$^+$AG$^+$ cells in maintenance and expansion culture differentiate into oogonium-/gonocyte-like cells in xrOvaries. (E) Comparison of genome-wide 5 mC level (genome-wide 2-kb window) scatter-plot between d12 hPGCLC reported in von Meyenn et al (2016) and the indicated cell type (Tang et al, 2015, Yamashiro et al, 2018, and the present invention) combined with histogram (top and right of the scatter plot). Wk: week-old. Embryo gender is indicated after the week-old indication. F: female, M: male.

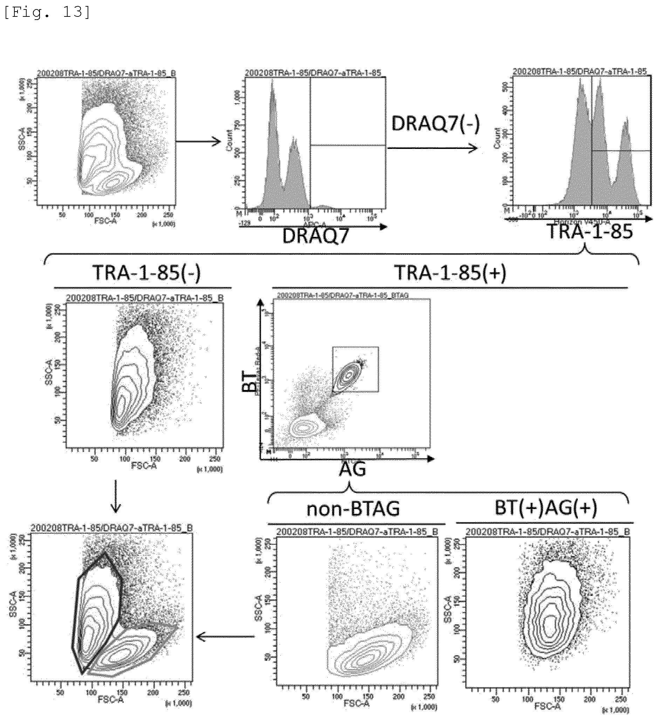

[Fig. 13] A drawing showing the effectiveness of the method for evaluating the hPGC or hPGCLC maintenance culture system of the present invention. PGCLCs were induced from human iPSCs having the PGCLC reporter gene BLIMP1-2A-tdTomato/AP2γ-2A-eGFP (BTAG) and cultured for proliferation. The dead cell staining reagent DRAQ7-negative and human-specific cell surface antigen TRA-1-85-negative cell population is considered to be mouse-derived feeder cells (m220-5), and showed a characteristic pattern (red) in which SSC takes a wide range of values in two-dimensional plot of Forward Scatter (FSC) and Side Scatter (SSC). On the other hand, TRA-1-85-positive cells are considered to be derived from hPGCLC, and showed a pattern (green) in which non-BTAG double-positive cells other than BTAG double-positive proliferated hPGCLC have low SSC values and FSC shows wide values in the FSC/SSC two-dimensional plot. When feeder cells and non-BTAG double-positive cells were displayed in the same FSC/SSC two-dimensional plot, each cell population was seen almost independently and was distinguishable (purple). This indicates that feeder cells and hPGCLC-derived dedifferentiated cells (non-BTAG double-positive cells) are distinguishable by displaying those other than BTAG double-positive proliferated PGCLCs in the FSC/SSC two-dimensional plot, even without analyzing the expression of TRA-1-85.

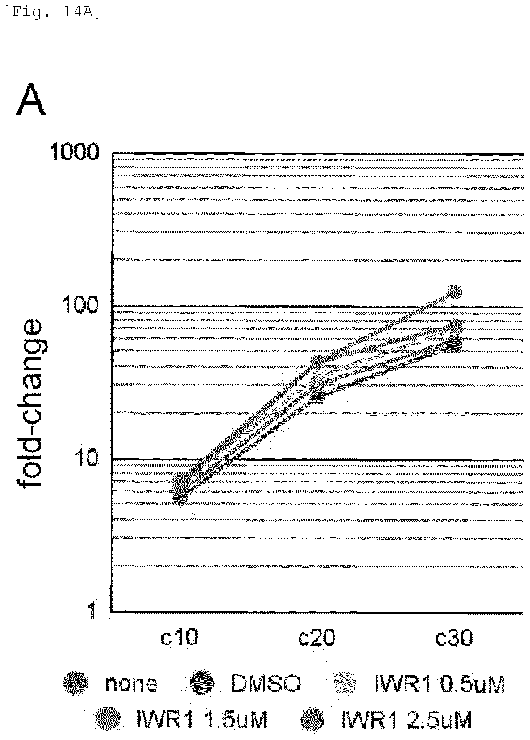

[Fig. 14A] A drawing showing the suppressive effect of Wnt signal transduction inhibitor on PGCLC dedifferentiation. (A) shows the results of an experiment in which IWR1 at various concentrations were added to the maintenance and expansion culture medium of hPGCLC, and cultures were performed for 30 days. Growth curve of BTAG double-positive cells under each condition in 30 day culture (n=3). The average values of the three experiments were plotted and connected with a straight line. The number after the lowercase letter "c" indicates the number of days of culture.

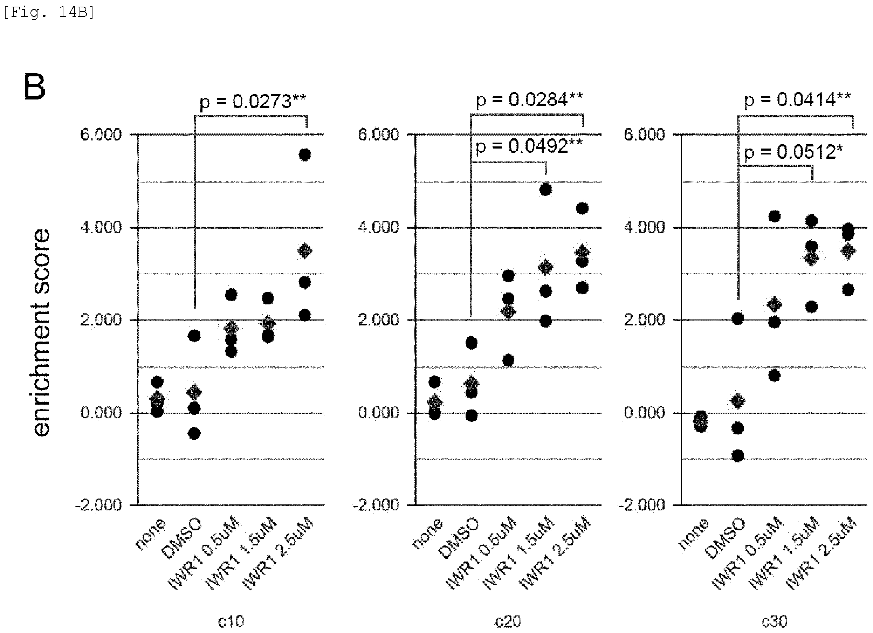

[Fig. 14B] A drawing showing the suppressive effect of Wnt signal transduction inhibitor on PGCLC dedifferentiation. (B) shows the results of an experiment in which IWR1 at various concentrations were added to the maintenance and expansion culture medium of hPGCLC, and cultures were performed for 30 days. Enrichment score (n = 3) under each condition at C10, C20 and C30. The average values were plotted with red diamonds. Using the DMSO-added group as a control group, a multiple comparison test was performed by Dunnet method. *:p<0.1, **:p<0.05

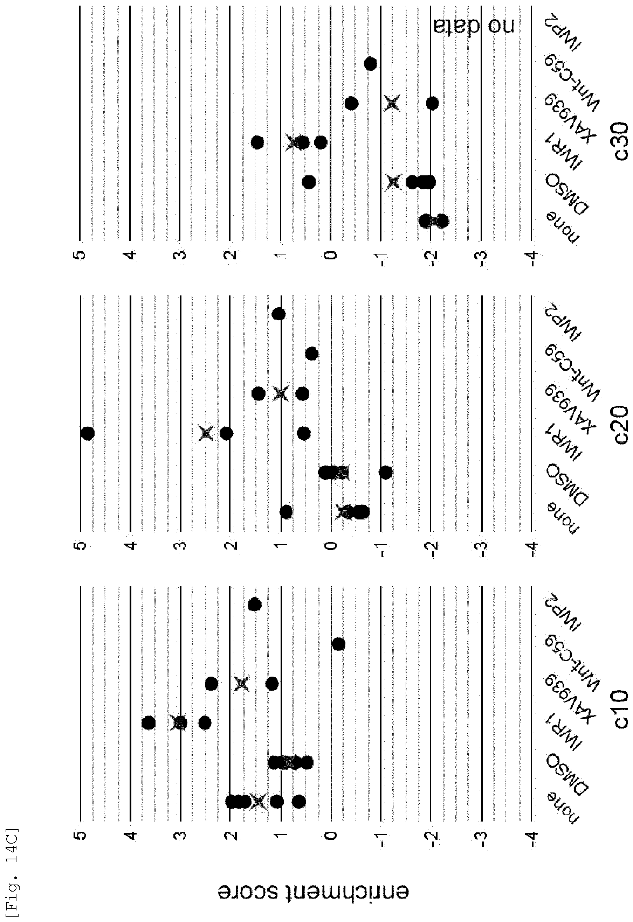

[Fig. 14C] A drawing showing the suppressive effect of Wnt signal transduction inhibitor on PGCLC dedifferentiation. (C) shows the results of experiments in which various Wnt signal transduction inhibitors (IWR1, XAV939, Wnt-C59, IWP2) were added to a medium at a concentration of 2.5 μM, and cultures were performed for 30 days. Enrichment score under each Wnt signal inhibitor addition condition.at c10, c20 and c30. none: n=5 (c10, c20); n=2 (c30), DMSO: n=5 (c10, c20); n=4 (c30), IWR1: n=3, XAV939: n=2, IWP2: n=1, Wnt-C59: n=1

[Description of Embodiments]

[I] Method for maintaining and expanding hPGC or hPGCLC

The present invention provides a method for maintaining and expanding a human primordial germ cell (hPGC) or a human primordial germ cell-like cell (hPGCLC) derived from a human pluripotent stem cell (hPSC). The method includes culturing hPGC or hPGCLC in the presence of

    (i) a forskolin or phosphodiesterase 4 (PDE4) inhibitor, and
    (ii) one or more cytokines selected from the group consisting of a basic fibroblast growth factor (bFGF), a leukemia inhibitory factor (LIF), and an epidermal growth factor (EGF).

hPGC to be used in the present invention can be isolated by any method known per se in the art (e.g., Patent Literature 1). For example, it can be isolated from the tissue of a dead fetus at 5 to 9 weeks of gestation by a method such as FACS or the like using the expression of an hPGC-specific marker (e.g., INTEGRINα6, EpCAM, etc.) as an index, though the method is not limited to this. The hPGCLC for use in the present invention may be any as long as it is induced in vitro from isolated hPSC and has properties equivalent to those of hPGC. For example, the hPGCLC can be produced from isolated hPSC by the method shown below.

1. Production of hPGCLC from hPSC

hPSC for use as the starting material of hPGCLC production may be any as long as it is an isolated undifferentiated cell possessing a "self-renewal" that enables it to proliferate while retaining the undifferentiated state, and "pluripotency" that enables it to differentiate into all the three primary germ layers of the embryo. As used herein, "isolated" means being placed outside a living body (in vitro) from a living body (in vivo), and does not necessarily mean being purified. Examples of the isolated PSC include iPS cells, ES cells, embryonic germ (EG) cells, embryonic cancer (EC) cells and the like, with preference given to iPS cells or ES cells.

(1) Production of hPSC

(i) ES cell (ESC)

The method includes, but is not limited to, a method for culturing inner cell mass (Thomson JA, et al., Science. 282, 1145-1147, 1998). Also, ES cells can be obtained from certain depositories and are commercially available. For example, human ES cell lines H1 and H9 can be obtained from WiCell Institute of University of Wisconsin and KhES-1, KhES-2 and KhES-3 can be obtained from Institute for Frontier Medical Sciences, Kyoto University.

(ii) iPS cell (iPSC)

iPS cell is an artificial stem cell derived from somatic cells and having properties almost equivalent to those of ES cells, for example, differentiation pluripotency and proliferation potency by self-renewal, and it can be produced by introducing specific reprogramming factors in the form of nucleic acids (DNA or RNA) or proteins into somatic cells (Takahashi, K. and S. Yamanaka (2006) Cell, 126: 663-676; Takahashi, K. et al. (2007) Cell, 131: 861-872; Yu, J. et al. (2007) Science, 318: 1917-1920; Nakagawa, M. et al. (2008) Nat. Biotechnol. 26: 101-106; WO2007/069666).

The term "somatic cell" used in the present specification means any human cell excluding germ line cells and differentiated totipotent cells such as ovum, oocyte, ES cell, and the like. Somatic cells non-limitatively include any of fetal somatic cells, neonatal somatic cells, and mature healthy or diseased somatic cells, as well as any primarily cultured cells, subcultured cells, and established cells. Specific examples of the somatic cell include (1) tissue stem cells (somatic stem cells) such as nerve stem cells, hematopoietic stem cells, mesenchymal stem cells, and dental pulp stem cells, (2) tissue progenitor cell, (3) differentiated cells such as lymphocyte, epithelial cell, endothelial cell, muscle cells, fibroblast (skin cell etc.), hair cell, hepatocyte, gastric mucosa cell, enterocyte, splenocyte, pancreatic cell (pancreatic exocrine cell etc.), brain cell, lung cell, nephrocyte, adipocyte, and the like, and the like.

The reprogramming factors may be composed of genes specifically expressed in ES cells, gene products thereof, or non-coding RNA, or genes that play an important role in maintaining an undifferentiated state of ES cells, gene products thereof, or non-coding RNA, or low-molecular-weight compounds. Examples of the gene contained in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2, Tbx3, Glis1 and so on. These reprogramming factors may be used alone or in combination. Examples of the combination of the reprogramming factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu, D. et al. (2008) Nat. Biotechnol., 26: 795-797, Shi, Y. et al. (2008) Cell Stem cell, 2: 525-528, Eminli, S. et al. (2008) Stem Cells, 26: 2467-2474, Huangfu, D. et al. (2008) Nat. Biotechnol., 26: 1269-1275, Shi, Y. et al. (2008) Cell Stem Cell, 3: 568-574, Zhao, Y. et al. (2008) Cellstem Cell, 3: 475-479, Marson, A. (2008) Cell Stem Cell, 3: 132-135, Feng, B. et al. (2009) Nat. Cell Biol., 11: 197-203, Judson, R.L. et al. (2009) Nat. Biotechnol., 27: 459-461, Lyssiotis, C.A. et al. (2009) Proc. Natl. Acad. Sci. USA, 106: 8912-8917, Kim, J.B. et al. (2009) Nature, 461: 649-643, Ichida, J.K. et al. (2009) Cell Stem Cell, 5: 491-503, Heng, J.C. et al. (2010) Cell Stem Cell, 6: 167-74, Han, J. et al. (2010) Nature, 463: 1096-100, Mali, P. et al. (2010) Stem Cells, 28: 713-720, and Maekawa, M. et al. (2011) Nature, 474: 225-9.

In a preferred embodiment, Oct3/4, Sox2, and Klf4 (OSK) can be used as reprogramming factors. More preferably, Myc family members (M) selected from L-Myc, N-Myc, and c-Myc (including T58A variant) can be used in addition to the three factors. Furthermore, Lin28 promotes the formation of TRA-1-60-positive cells and inhibits reverse conversion to TRA-1-60-negative cells. Therefore, Lin 28 is also preferably used as a reprogramming factor in addition to the 3 factors (OSK) or 4 factors (OSKM).

The above-mentioned reprogramming factors also include, but are not limited to, histone deacetylase (HDAC) inhibitors [e.g., low-molecular inhibitors such as valproic acid (VPA), trichostatin A, sodium butyrate, MC 1293, and M344, nucleic acid-based expression inhibitors such as siRNA and shRNA against HDAC (e.g., HDAC1 siRNA Smartpool (registered trade mark) (Millipore), HuSH 29mer shRNA Constructs against HDAC1 (OriGene), etc.), and the like, and the like], MEK inhibitors (e.g., PD184352, PD98059, U0126, SL327 and PD0325901), glycogen synthase kinase-3 inhibitors (e.g., Bio and CHIR99021), DNA methyltransferase inhibitors (e.g., 5-azacytidine), histone methyltransferase inhibitors (e.g., small molecule inhibitors such as BIX-01294 and the like, nucleic acid-based expression inhibitors such as siRNA and shRNA against Suv39hl, Suv39h2, SetDBl and G9a, and the like, and the like), L-channel calcium agonists (e.g., Bayk8644), butyric acid, TGFβ inhibitors or ALK5 inhibitors (e.g., LY364947, SB431542, 616453 and A-83-01), p53 inhibitors (e.g., siRNA and shRNA for p53), ARID3A inhibitor (e.g., siRNA and shRNA against ARID3A), miRNAs such as miR-291-3p, miR-294, miR-295, mir-302, and the like, Wnt signaling agonists (e.g., soluble Wnt3a), neuropeptide Y, prostaglandins (e.g., prostaglandin E2 and prostaglandin J2), factors used for the purpose of increasing the establishment efficiency of hTERT, SV40LT, UTF1, IRX6, GLIS1, PITX2, DMRTB1, and the like. In the present specification, the factors used for the purpose of increasing the establishment efficiency are not particularly distinguished from the reprogramming factors.

When the reprogramming factor is in the form of a protein, it may be introduced into somatic cells by, for example, a means such as lipofection, fusion with cell membrane penetrating peptides (e.g., TAT derived from HIV and polyarginine) (Cell Stem Cell. 2009 May 8; 4(5) :381-4), microinjection, and the like.

When the reprogramming factor is in the form of a DNA, it can be introduced into somatic cells by, for example, a means such as vector such as virus, plasmid, artificial chromosome, and the like, lipofection, liposome, microinjection, and the like. Examples of the virus vector include retrovirus vector, lentivirus vector (Cell, 126: 663-676, 2006; Cell, 131: 861-872, 2007; Science, 318: 1917-1920, 2007), adenovirus vector (Science, 322: 945-949, 2008), adeno-associated virus vector, Sendaivirus vector (vector of Hemagglutinating Virus of Japan) (WO 2010/008054), and the like. Furthermore, examples of the artificial chromosome vector include human artificial chromosome (HAC), yeast artificial chromosome (YAC), bacterium artificial chromosome (BAC, PAC), and the like. As the plasmid, plasmid for mammalian cells can be used (Science, 322: 949-953, 2008). Vector can contain control sequences such as promoter, enhancer, ribosome-binding sequence, terminator, polyadenylated site, and the like, so that a nuclear reprogramming substance can be expressed. Furthermore, where necessary, vectors may contain a drug resistance gene (e.g., kanamycin resistance gene, ampicillin resistance gene, puromycin resistance gene, and the like), a selection marker sequence such as thymidine kinase gene, diphtheria toxin gene and the like, a green fluorescent protein (GFP), β glucuronidase (GUS), a reporter gene sequence such as FLAG and the like, and the like. In addition, in order to allow for excising of a gene encoding the reprogramming factor, or both a promoter and a gene encoding the reprogramming factor and binding to the promoter, after introduction into somatic cells, a LoxP sequence may be provided before and after them in the above-mentioned vector.

When the reprogramming factor is in the form of RNA, for example, it may be introduced into somatic cells by a means such as lipofection, microinjection, and the like, or RNA containing 5-methylcytidine and pseudouridine (TriLink Biotechnologies) may be used to suppress degradation (Warren, L. (2010) Cell Stem Cell, 7: 618-630). A reprogramming factor in the form of RNA may be introduced into somatic cells by using an RNA virus vector such as Sendaivirus vector and the like (JP-B-5963309). In addition, a method of introducing a given synthetic mRNA into somatic cells by using a cationic vehicle to allow for reprogramming (Cell Stem Cell. 2010 Nov 5; 7(5):618-30), and a method of reprogramming somatic cells by transfection of a give miRNA into the somatic cells (Cell Stem Cell. 2011 Jun 3; 8(6):633-8) are also known.

The reprogramming factor may also be a low-molecular-weight compound. For example, methods using VPA, CHIR99021, 616452, tranylcypromine, forskolin, and DZNep are known (Science. 2013 Aug 9; 341(6146):651-4).

After the reprogramming factors are brought into contact with the cell, the cell are contained in, for example, 10 to 15% FBS-containing DMEM, DMEM/F12 or DME culture medium (these culture media can further contain LIF, penicillin/streptomycin, puromycin, L-glutamine, nonessential amino acids, β-mercaptoethanol and the like as appropriate) or a commercially available culture medium [for example, culture medium for primate ES cell (culture medium for primate ES/iPS cell, Reprocell), serum-free medium (mTeSR, Stemcell Technologies)] and the like.

Examples of the culture method include contacting a somatic cell with a reprogramming factor on 10% FBS-containing DMEM or DMEM/F12 culture medium at 37°C in the presence of 5% $CO_2$ and culturing for about 4 to 7 days, thereafter reseeding the cells on feeder cells (e.g., mitomycin C-treated STO cells, SNL cells etc.), and culturing the cells in a bFGF-containing culture medium for primate ES cell from about 10 days after the contact of the somatic cell and the reprogramming factor, whereby iPS-like colonies can be obtained after about 30 to about 45 days or longer from the contact.

Alternatively, the cells are cultured on feeder cells (e.g., mitomycin C-treated STO cells, SNL cells etc.) at 37°C in the presence of 5% $CO_2$ in a 10% FBS-containing DMEM culture medium (which can further contain LIF, penicillin/streptomycin, puromycin, L-glutamine, nonessential amino acids, β-mercaptoethanol and the like as appropriate), whereby ES-like colonies can be obtained after about 25 to about 30 days or longer. Desirably, a method using a somatic cell itself to be reprogrammed (Takahashi K, et al. (2009), PLoS One. 4:e8067 or WO2010/137746), or an extracellular substrate (e.g., Laminin-5 (WO2009/123349) and Matrigel (BD)), instead of the feeder cells.

A candidate colony of iPS cells can be selected by a method with drug resistance and reporter activity as indicators, and also by a method based on visual examination of morphology. As an example of the former, a colony positive for drug resistance and/or reporter activity is selected using a recombinant somatic cell wherein a drug resistance gene and/or a reporter gene is targeted to the locus of a gene highly expressed specifically in pluripotent cells (e.g., Fbx15, Nanog, Oct3/4 and the like, preferably Nanog or Oct3/4). Examples of the method of selecting candidate colonies based on visual examination of morphology include the method described in Takahashi et al. in Cell, 131, 861-872 (2007). Although the method using reporter cells is convenient and efficient, it is desirable from the viewpoint of safety that colonies be selected by visual examination when iPS cells are prepared for the purpose of human treatment. When the three factors Oct3/4, Klf4 and Sox2 are used as reprogramming factors, the number of clones established decreases but the resulting colonies are mostly of iPS cells of high quality comparable to ES cells, so that iPS cells can efficiently be established even without using reporter cells.

The identity of the cells of a selected colony as iPS cells can be confirmed by positive responses to a Nanog (or Oct3/4) reporter (puromycin resistance, GFP positivity and the like) as well as by the formation of a visible ES cell-like colony, as described above. However, to ensure higher accuracy, it is possible to perform tests such as analyzing the expression of various ES-cell-specific genes and transplanting the cells selected to a mouse and confirming the formation of teratomas.

The hPSCs obtained by the aforementioned method and cultured using feeder cells and serum show diversity, and therefore, are desirably cultured under culture conditions of known composition. Such serum-free and feeder-free conditions include, for example, the method described in Nakagawa M, et al., Sci Rep. 4, 3594, 2014 and the like, which is a method including culturing on an extracellular substrate (e.g., laminin5 (WO 2009/123349), laminin5 fragment (e.g., Laminin-5E8 (Nippi. Inc.)) and Matrigel (BD)) using a serum-free medium (e.g., mTeSR (Stemcell Technology), Essential 8 (Life Technologies) and StemFit (Ajinomoto Co., Inc.)).

(2) Induction of direct differentiation from hPSC into hPGCLC

Human ES cells and iPS cells have very different biological (morphological, molecular and functional) properties from mouse ES cells and iPS cells. Mouse pluripotent stem cells can exist in two functionally distinct states, LIF-dependent ES cells and bFGF-dependent epiblast stem cells (EpiSCs). Molecular analyses suggest that the pluripotent state of human ES cells and iPS cells is similar to that of mouse EPiSCs rather than that of mouse ES cells and iPS cells. Therefore, hPSC can be induced to differentiate into hPGCLC by culturing hPSC in the presence of BMP4 and LIF (Cell. 2009 May 1; 137(3):571-84).

In addition, human ES and iPS cells in a mouse ES cell-like pluripotent state (also referred to as naive human ES and iPS cells) have been established by ectopic induction of Oct3/4, Sox2, Klf4, c-Myc and Nanog in the presence of LIF (Cell Stem Cells, 2010 Jun 4; 6(6):535-46), or ectopic induction of Oct3/4, Klf4 and Klf2 combined with LIF and inhibitors of GSK3$\beta$ and ERK1/2 pathway (Proc. Natl. Acad. Sci. USA, 2010 May 18; 107(20):9222-7). These naive human ES and iPS cells can be used as starting materials for the present invention due to their pluripotent more immature compared with that of conventional human ES and iPS cells.

Basal media for differentiation induction include, but are not limited to, Neurobasal medium, Neural Progenitor Basal medium, NS-A medium, BME medium, BGJb medium, CMRL 1066 medium, minimum essential medium (MEM), Eagle MEM, $\alpha$MEM, Dulbecco's modified Eagle medium (DMEM), Glasgow minimum essential medium (GMEM), Improved MEM Zinc Option medium, IMDM medium, Medium 199 medium, DMEM/F12 medium, Ham's medium, RPMI1640 medium, Fischer's medium, and mixtures thereof.

The basal medium can be a serum-containing or serum-free medium. Preferably, a serum-free medium can be used. The serum-free medium (SFM) refers to media with no unprocessed or unpurified serum and accordingly, can include media with purified blood-derived components or animal tissue-derived components (such as growth factors). The concentration of serum (for example, fetal bovine serum (FBS), human serum, etc.) can be 0-20%, preferably 0-5%, more preferably 0-2%, most preferably 0% (i.e., serum-free). The SFM may contain or may not contain any serum replacements. The serum replacement can include materials which appropriately contain albumin (albumin replacements such as lipid-rich albumin, recombinant albumin, plant starch, dextrans and protein hydrolysates), transferrin (or other iron transporters), fatty acids, insulin, collagen precursors, trace elements, 2-mercaptoethanol, 3'-thiolglycerol, or equivalents thereto. The serum replacements can be prepared by the method disclosed in WO 98/30679, for example. Alternatively, any commercially available materials can be used for more convenience. The commercially available materials include Knockout™ Serum Replacement (KSR), Chemically-defined Lipid concentrated, and Glutamax (Invitrogen).

The basal medium can also contain other additives known per se. The additive is not subject to limitation, for example, growth factors (for example, insulin and the like), polyamines (for example, putrescine and the like), minerals (for example, sodium selenate and the like), saccharides (for example, glucose and the like), organic acids (for example, pyruvic acid, lactic acid and the like), amino acids (for example, non-essential amino acids (NEAA), L-glutamine and the like), reducing agents (for example, 2-mercaptoethanol and the like), vitamins (for example, ascorbic acid, d-biotin and the like), steroids (for example, [beta]-estradiol, progesterone and the like), antibiotics (for example, streptomycin, penicillin, gentamycin and the like), buffering agents (for example, HEPES and the like), nutritive additives (for example, B27 supplement, N2 supplement, StemPro-Nutrient Supplement and the like) and the like can be mentioned. It is preferable that each of the additives be contained in a concentration range known per se.

hPSC may be cultured in the presence or absence of feeder cells. The feeder cells are not particularly limited, and feeder cells known per se for use in culturing pluripotent stem cells such as ESCs and iPSCs can be used. For example, fibroblasts (mouse embryonic fibroblasts, mouse fibroblast cell line STO and the like) can be mentioned. The feeder cells are preferably inactivated by a method known per se, for example, treatment with radiation (gamma rays and the like), an anticancer agent (mitomycin C and the like) and the like. However, in one preferred embodiment, hPSC is cultured under feeder-free conditions.

The concentration of BMP4 to be added to the basal medium is, for example, about 100 ng/ml or more, preferably about 200 ng/ml or more, more preferably about 300 ng/ml or more. Also, the concentration of BMP4 is, for example, about 1,000 ng/ml or less, preferably about 800 ng/ml or less, more preferably 600 ng/ml or less. BMP4 may include variants, fragments, or modified forms thereof as long as they have activity usable in this step. The concentration of LIF to be added to the basal medium is, for example, about 300 U/ml or more, preferably about 500 U/ml or more, more preferably about 800 U/ml or more. Also, the concentration of LIF is, for example, about 2,000 U/ml or less, preferably about 1,500 U/ml or less, more preferably about 1,200 U/ml or less. Media containing BMP4 and LIF can be used as differentiation induction media. LIF may include variants, fragments, or modified forms thereof as long as they have activity usable in this step.

The differentiation induction medium may further contain at least one additive selected from SCF, BMP8b, and EGF. SCF, BMP8b and EGF remarkably prolong the period of time which hPGCLC is maintained in a Blimp1- and Stella-positive state, when present in ranges of effective concentrations. The concentration of SCF is, for example, about 30 ng/ml or more, preferably about 50 ng/ml or more, more preferably about 80 ng/ml or more. Also, the concentration of SCF is, for example, about 200 ng/ml or less, preferably about 150 ng/ml or less, more preferably about 120 ng/ml or less. SCF may include variants, fragments, or modified forms thereof as long as they have activity usable in this step. The concentration of BMP8b is, for example, about 100 ng/ml or more, preferably about 200 ng/ml or more, more preferably about 300 ng/ml or more. Also, the concentration of BMP8b is, for example, about 1,000 ng/ml or less, preferably about 800 ng/ml or less, more preferably about 600 ng/ml or less. The concentration of EGF is, for example, about 10 ng/ml or more, preferably about 20 ng/ml or more, more preferably about 30 ng/ml or more. BMP8b may include variants, fragments, or modified forms thereof as long as they have activity usable in this step. Also, the concentration of EGF is, for example, about 100 ng/ml or less, preferably about 80 ng/ml or less, more preferably about 60 ng/ml. EGF may include variants, fragments, or modified forms thereof as long as they have activity usable in this step.

In one preferred embodiment, the differentiation induction medium contains BMP, LIF, SCF, BMP8b and EGF in addition to the basal medium. The concentrations of these ingredients can be chosen as appropriate over the ranges of about 200 to about 800 ng/ml, preferably about 300 to about 600 ng/ml, for BMP4, about 500 to about 1500 U/ml, preferably about 800 to about 1200 U/ml, for LIF, about 50 to about 150 ng/ml, preferably about 80 to about 120 ng/ml, for SCF, about 200 to about 800 ng/ml, preferably about 300 to about 600 ng/ml, for BMP8b, and about 20 to about 80 ng/ml, preferably about 30 to about 60 ng/ml, for EGF.

The BMP4, LIF, SCF, BMP8b and EGF contained in the differentiation induction medium are not subject to limitation as to the source thereof, and may be isolated and purified from cells of any mammals (for example, human, mouse, monkey, swine, rat, dog, and the like). It is preferable to use BMP4, LIF, SCF, BMP8b and EGF that are derived from a human. BMP4, LIF, SCF, BMP8b, and EGF may also be chemically synthesized or biochemically synthesized using a cell-free translation system, or produced from a transformant bearing a nucleic acid encoding each of the proteins. The recombinant products of BMP4, LIF, SCF, BMP8b, and EGF are commercially available.

A culture vessel used for this step can include, but is particularly not limited to, flask, flask for tissue culture, dish, petri dish, dish for tissue culture, multi dish, micro plate, micro-well plate, multi plate, multi-well plate, micro slide, chamber slide, schale, tube, tray, culture bag, and roller bottle. The culture vessel can be cell-adhesive. The cell-adhesive culture vessel can be coated with any of substrates for cell adhesion such as extracellular matrix (ECM) to improve the adhesiveness of the vessel surface to the cells. The substrate for cell adhesion can be any material intended to attach pluripotent stem cells or feeder cells (if used). The substrate for cell adhesion includes collagen, gelatin, poly-L-lysine, poly-D-lysine, poly-L-ornithine, laminin, and fibronectin and mixtures thereof for example Matrigel, and lysed cell membrane preparations (Klimanskaya I et al 2005. Lancet 365: p1636-1641).

In this culture, hPSCs are seeded to a non-cell-adhesive or low-adhesive culture vessel known per se to obtain a cell density of, for example, about 3 to $10 \times 10^4$ cells/mL, preferably about 4 to $8 \times 10^4$ cells/mL, and cultured in an incubator in an atmosphere of 1 to 10% $CO_2$/99 to 90% air at about 30 to 40°C, preferably about 37°C, for about 4 to about 10 days, preferably about 4 to about 8 days, more preferably about 4 to about 6 days, further preferably about 4 days.

The fact of differentiation into hPGCLC can be confirmed by, for example, analyzing the expression of BLIMP1 by RT-PCR and the like. As required, furthermore, the expression of other genes and cell surface antigens can also be examined. Examples of other genes include TFAP2C. When pluripotent stem cells bearing fluorescent protein genes under the control of BLIMP1- and/or TFAP2C-promoters are used as a starting material, the fact of differentiation into hPGCLC can be confirmed by FACS analysis. When human PSC has no appropriate transgenic reporter, it is preferable to confirm the fact of differentiation into hPGCLC by FACS analysis and the like using one or more cell surface antigens specifically expressed on hPGCLC. As the cell surface antigens, at least one marker gene selected from the group consisting of PECAM (CD31), INTEGRINα6 (CD49f), INTEGRINβ3 (CD61), KIT (CD117), EpCAM, PODOPLANIN, and TRA1-81, preferably INTEGRINα6 and EpCAM, are exemplified.

(3) Induction of differentiation from hPSC via hiMeLC into hPGCLC

Induction from hPSC into hPGCLC can be performed by the method described in Hayashi K, et al., Cell. 2011 Aug 19; 146(4):519-32, similar to the case of mouse. However, when this method is used, many dead cells occur and the induction efficiency is low. On the other hand, when hPSCs are cultured in a culture medium containing activin A and a GSK3β inhibitor to induce human initial mesoderm-like cells (hiMeLCs), and the hiMeLCs are subjected to conditions for induction into mouse PGCLCs, the induction efficiency of hPGCLC increases and the occurrence of dead cells can be suppressed (WO2017/002888). Therefore, in a preferred embodiment, hPGCLC as the starting material in the method of the present invention for maintaining and expanding is induced from hPSC via hiMeLC.

(3-1) Induction of differentiation from hPSC into hiMeLC

Examples of the basal medium for inducing differentiation from hPSC into hiMeLC include, but are not limited to, basal media exemplified for use in the aforementioned (2). The basal media may contain other additives known per se that are exemplified for use in the aforementioned (2).

The medium may be a serum-containing or serum-free medium (SFM). Preferably, a serum-free medium may be used. The concentration of serum (for example, fetal bovine serum (FBS), human serum, etc.) can be 0 to about 20%, preferably 0 to about 5%, more preferably 0 to about 2%, most preferably 0% (i.e., serum-free). The SFM may contain or may not contain any serum replacement, such as KSR and the like. The detail is as described in the aforementioned (2).

The medium for inducing differentiation from hPSC to hiMeLC contains activin A and GSK-3β inhibitor as essential additives in the basal medium.

The concentration of activin A in the medium for inducing differentiation from hPSC to hiMeLC is, for example, about 5 ng/ml or more, preferably about 10 ng/ml or more, more preferably about 15 ng/ml or more, and is, for example, about 100 ng/ml or less, preferably about 90 ng/ml or less, more preferably about 80 ng/ml or less. For example, it is about 5 to about 100 ng/ml, about 10 to about 90 ng/ml, about 15 to about 80 ng/ml, about 20 to about 70 ng/ml, or about 30 to about 60 ng/ml. Particularly preferably, the concentration of activin A is about 50 ng/ml.

In the present specification, the GSK-3β inhibitor is defined as a substance that inhibits kinase activity of GSK-3β protein (e.g., phosphorylation capacity against β catenin), and many are already known. Examples thereof include lithium chloride (LiCl) first found as a GSK-3β inhibitor, BIO, which is an indirubin derivative (alias, GSK-3β inhibitor IX; 6-bromo indirubin 3'-oxime), SB216763 which is a maleimide derivative (3-(2,4-dichlorophenyl)-4-(1-methyl-1H-indol-3-yl)-1H-pyrrole-2,5-dione), GSK-3β inhibitor VII which is a phenyl α-bromomethyl ketone compound (4-dibromoacetophenone), L803-mts which is a cell membrane-permeable type phosphorylated peptide (alias, GSK-3β peptide inhibitor; Myr-N-GKEAPPAPPQSpP-NH$_2$) and CHIR99021 having high selectivity (6-[2-[4-(2,4-Dichlorophenyl)-5-(4-methyl-1H-imidazol-2-yl)pyrimidin-2-ylamino]ethylamino]pyridine-3-carbonitrile:CAS No. 252917-06-9). These compounds are commercially available from, for example, Calbiochem, Biomol and the like, and can be easily utilized. They may be obtained from other sources, or may be directly produced.

The GSK-3β inhibitor used in this step can preferably be CHIR99021.

The concentration of CHIR99021 in the medium is, though not particularly limited to, preferably about 0.1 μM to about 50 μM, for example, 1 μM, 2 μM, 3 μM, 4 μM, 5 μM, 6 μM, 7 μM, 8 μM, 9 μM, 10 μM or a concentration not less than these. Preferably, a concentration higher than the concentration of about 1 μM used for inhibition of GSK-3β is used, more preferably about 3 μM or more. Particularly preferably, the concentration of CHIR99021 is about 3 μM.

The medium is preferably free of bFGF and BMP4.

The medium may further contain a fibroblast growth factor receptor (FGFR) inhibitor. The FGFR inhibitor is not particularly limited as long as it is a drug inhibiting binding of FGF receptor and FGF or signal transduction occurring after the binding. For example, PD173074 or BGJ398 can be mentioned.

When PD173074 is used, the concentration in the medium is notparticularly limited. It is preferably about 1 nM to about 50 nM, for example, 1 nM, 2 nM, 3 nM, 4 nM, 5 nM, 6 nM, 7 nM, 8 nM, 9 nM, 10 nM, 11 nM, 12 nM, 13 nM, 14 nM, 15 nM, 16 nM, 17 nM, 18 nM, 19 nM, 20 nM, 25 nM, 30 nM, 35 nM, 40 nM, 45 nM, 50 nM, or above, more preferably about 25 nM.

The medium preferably further contains a knockout serum replacement (KSR). KSR remarkably increases the induction efficiency for mesoderm-like cells when present in a range of effective concentrations. The concentration of KSR is, for example, 5%, 10%, 15%, 20% or above. It is more preferably about 15%.

In culturing here, the medium preferably further contains a ROCK inhibitor to suppress apoptosis during separation of hPSC into single cells. The ROCK inhibitor is not particularly limited as long as it can suppress function of the Rho kinase (ROCK). For example, Y-27632 may be preferably used in the present invention.

The concentration of Y-27632 in the medium is, though not particularly limited to, preferably 1 $\mu$M to 50 $\mu$M, for example, 1 $\mu$M, 2 $\mu$M, 3 $\mu$M, 4 $\mu$M, 5 $\mu$M, 6 $\mu$M, 7 $\mu$M, 8 $\mu$M, 9 $\mu$M, 10 $\mu$M, 11 $\mu$M, 12 $\mu$M, 13 $\mu$M, 14 $\mu$M, 15 $\mu$M, 16 $\mu$M, 17 $\mu$M, 18 $\mu$M, 19 $\mu$M, 20 $\mu$M, 25 $\mu$M, 30 $\mu$M, 35 $\mu$M, 40 $\mu$M, 45 $\mu$M, 50 $\mu$M, more preferably about 10 $\mu$M.

As the culture vessel used in this step, those exemplified in the aforementioned (2) can be similarly used, and fibronectin-coated culture vessels are preferred.

For culturing, hPSCs are seeded on the above-mentioned culture vessel to a cell density of, for example, about $10^4$ to about $10^5$ cells/cm$^2$, preferably about 2 to about $6\times10^4$ cells/cm$^2$, and cultured under an atmosphere of 1 to 10% $CO_2$/99 to 90% air in an incubator at about 30 to about 40°C, preferably about 37°C, for less than 60 hr, preferably 42 hr (e.g., error of $\pm$ 2 hr is tolerable).

The fact of differentiation into hiMeLC can be confirmed, for example, by analyzing the expression level of the marker gene of iMeLC and/or PSC by RT-PCR. iMeLCs are defined as cells having either or both of the following properties:

(1) an increase in at least one gene expression selected from T, EOMES, EVX1, SP5, MIXL1, and NODAL, compared with PSC before differentiation induction,
(2) a decrease in at least one gene expression selected from POU5F1, NANOG, and SOX2, compared with PSC before differentiation induction.

(3-2) Induction of differentiation from hiMeLC into hPGCLC

The thus-obtained hiMeLC can be induced to differentiate into hPGCLC by culturing in the presence of BMP.

Examples of the basal medium to be used for inducing the differentiation of hiMeLC into hPGCLC include, but are not limited to, the basal media exemplified for use in the aforementioned (2). The basal medium may contain other additives known per se such as those exemplified for use in the aforementioned (2). The medium may be a serum-containing medium or serum-free medium (SFM). Preferably, a serum-free medium may be used. The concentration of the serum (e.g., fetal bovine serum (FBS), human serum and the like) may be 0 to 20%, preferably 0 to 5%, more preferably 0 to 2%, most preferably 0% (that is, serum-free). SFM may or may not contain an optional serum replacement such as KSR and the like. The detail is as described in the aforementioned (2).

The medium for inducing the differentiation of hiMeLC into hPGCLC is a basal medium containing BMP as an essential additive. The BMP to be used is preferably BMP2, BMP4, or BMP7. More preferred BMP is BMP 2 or BMP 4. The concentration of BMP is, for example, about 50 ng/ml or more, preferably about 100 ng/ml or more, more preferably about 150 ng/ml or more. The concentration of BMP is, for example, about 1,000 ng/ml or less, preferably about 800 ng/ml or less, more preferably about 600 ng/ml or less. For example, it is about 50 to about 100 ng/ml, about 100 to about 800 ng/ml, about 150 to about 600 ng/ml. Particularly preferably, the concentration of BMP is about 200 ng/ml.

The medium for differentiation induction from hiMeLC into hPGCLC preferably further contains at least one cytokine selected from the group consisting of SCF, EGF, and LIF as an additive.

The concentration of SCF is, for example, about 30 ng/ml or more, preferably about 50 ng/ml or more, more preferably about 80 ng/ml or more. The concentration of SCF is, for example, about 200 ng/ml or less, preferably about 150 ng/ml or less, more preferably about 120 ng/ml or less. For example, it is about 30 to about 200 ng/ml, about 50 to about 150 ng/ml, about 80 to about 120 ng/ml. Particularly preferably, the concentration of SCF is about 100 ng/ml.

The concentration of LIF is, for example, about 300 U/ml or more, preferably about 500 U/ml or more, more preferably about 800 U/ml or more. For example, it is about 2,000 U/ml or less, preferably about 1,500 U/ml or less, more preferably 1,200 U/ml or less. The concentration of LIF is, for example, about 0.1 ng/ml or more, preferably about 1 ng/ml or more, more preferably about 5 ng/ml or more. The concentration of LIF is, for example, about 100 ng/ml or less, preferably about 50 ng/ml or less, more preferably about 25 ng/ml or less. For example, it is about 0.1 to about 100 ng/ml, about 1 to about 50 ng/ml, about 5 to about 25 ng/ml. Particularly preferably, the concentration of LIF is about 10 ng/ml.

The concentration of EGF is, for example, about 10 ng/ml or more, preferably about 20 ng/ml or more, more preferably about 30 ng/ml or more. The concentration of EGF is, for example, about 100 ng/ml or less, preferably about 80 ng/ml or less, more preferably about 60 ng/ml or less. For example, it is about 10 to about 100 ng/ml, about 20 to about 80 ng/ml, about 30 to about 60 ng/ml. Particularly preferably, the concentration of EGF is about 50 ng/ml.

In this step, the medium preferably further contains a ROCK inhibitor to suppress apoptosis during dissociation of hiMeLCs into single cells. As the ROCK inhibitor, one which is the same as the above is used.

In this culture, hiMeLCs are seeded to a non-cell-adhesive or low-adhesive culture vessel known per se to obtain a cell density of, for example, about 1 to $50\times10^3$ cells/cm$^2$, preferably about 5 to $20\times10^3$ cells/mL, and cultured in an incubator in an atmosphere of 1 to 10% $CO_2$/99 to 90% air at about 30 to 40°C, preferably about 37°C, for about 4 to 10 days, preferably 5 to 8 days, more preferably about 6 days (e.g., 144±12 hr, preferably 144±6 hr).

The fact of differentiation into hPGCLC can be confirmed in the same manner as in the aforementioned (2). In order to isolate hPGCLC to be subjected to the method of the present invention for maintaining and expanding, cells positive for the aforementioned reporter fluorescent protein or cell surface antigen are preferably sorted from the cells obtained by the above-mentioned culture by using, for example, fluorescence-activated cell sorting (FACS).

2. Maintenance and expansion of hPGC or hPGCLC

In this step, for example, hPGC or hPGCLC obtained by the above-mentioned method is cultured in the presence of

(i) a forskolin or PDE4 inhibitor, and
(ii) one or more cytokines selected from the group consisting of bFGF, LIF, and EGF.

As hPGCLC, the cells on d4 to d10, preferably d5 to d8, more preferably about d6, wherein the day of start of differentiation induction from hiMeLC is d0, may be used.

As the medium to be used in the present step, the media exemplified for the aforementioned 1.(2) can be similarly used as the basal medium. It is preferably other than F-12 medium and mixed media containing the same. For example, DMEM, GMEM, RPMI, CMRL, and the like can be mentioned, and DMEM is preferred. The glucose concentration of the medium is, for example, 0.1 to 5 g/L, preferably 0.2 to 4.5 g/L, more preferably 0.5 to 2 g/L, particularly preferably about 1 g/L.

It is preferable to add a serum and/or serum replacement to the medium. The kind and concentration of the serum or serum replacement to be used here may be the same as those exemplified for the aforementioned 1.(2). In addition, the medium may contain other additives known per se. Such additive is not particularly limited as long as it can support the maintenance and expansion of hPGCLC, and those exemplified for the aforementioned 1.(2) can be used in the same manner. Examples of the medium used in this step include, but are not limited to, DMEM (1 g/L glucose) media supplemented with 15% knockout serum replacement (KSR), 2.5% fetal bovine serum (FBS), 1% non-essential amino acid solution (NEAA), 0.1 mM 2-mercaptoethanol, 1% penicillin ·streptomycin, 2 mM L-glutamine (e.g., GlutaMAX™), and 100 ng/ml SCF, and the like.

In the maintenance and expansion of mPGCLC, the combined use of forskolin and a PDE4 inhibitor can synergistically increase the proliferation of mPGCLC up to about 50-fold. However, in the case of hPGCLC, the combined use of forskolin and PDE4 inhibitor offsets the expansion effect. Therefore, either forskolin or a PDE4 inhibitor alone is used in the maintenance and expansion method of the present invention. Forskolin is preferably used.

The concentration of forskolin is, for example, about 0.1 μM or more, preferably about 0.5 μM or more, more preferably about 1 μM or more. The concentration of forskolin is, for example, about 100 μM or less, preferably about 50 μM or less, more preferably about 30 μM or less. In a preferred embodiment, the concentration of forskolin may be appropriately selected from the range of about 0.5 to about 50 μM, preferably about 1 to about 30 μM. Particularly preferably, the concentration of forskolin is about 10 μM. Forskolin may include derivatives, salts, or solvates thereof as long as they have activity usable in this step.

The PDE4 inhibitor to be added to the above-mentioned medium is not particularly limited as long as it is a substance that can inhibit the enzyme activity of PDE4, namely, hydrolytic activity of cAMP. Preferably, it is a selective inhibitor of PDE4 (which does not inhibit not only enzyme other than phosphodiesterase (PDE) but also PDEs other than PDE4). Examples of the inhibitor include, but are not limited to, ibudilast, S-(+)-rolipram, rolipram, GSK256066, cilomilast and the like. It is preferably rolipram.

The concentration of the PDE4 inhibitor is, for example, about 0.1 μM or more, preferably about 0.5 μM or more, more preferably about 1 μM or more. The concentration of the PDE4 inhibitor is, for example, about 100 μM or less, preferably about 50 μM or less, more preferably about 30 μM or less. In a preferred embodiment, the concentration of the PDE4 inhibitor may be appropriately selected from the range of about 0.5 to about 50 μM, preferably about 1 to about 30 μM. When rolipram is used, the concentration of rolipram is particularly preferably about 10 μM. The PDE4 inhibitor may include derivatives, salts, or solvates thereof as long as they have activity usable in this step.

The medium for maintenance and expansion of hPGC or hPGCLC of the present invention further contains one or more cytokines selected from bFGF, LIF, and EGF. Preferred is a combination of LIF and EGF, bFGF alone, or a combination of LIF, EGF, and bFGF. When the drug of the aforementioned (i) is rolipram, the cytokine is preferably bFGF alone.

The concentration of LIF is, for example, about 300 U/ml or more, preferably about 500 U/ml or more, more preferably about 800 U/ml or more. The concentration of LIF is, for example, about 2,000 U/ml or less, preferably about 1,500 U/ml or less, more preferably 1,200 U/ml or less. The concentration of LIF is, for example, about 0.1 ng/ml or more, preferably about 1 ng/ml or more, more preferably about 5 ng/ml or more. The concentration of LIF is, for example, about 100 ng/ml or less, preferably about 50 ng/ml or less, more preferably about 25 ng/ml or less. For example, it is about 0.1 to about 100 ng/ml, about 1 to about 50 ng/ml, or about 5 to about 25 ng/ml. Particularly preferably, the concentration of LIF is about 10 ng/ml. LIF may include variants, fragments, or modified forms thereof as long as they have activity usable in this step.

The concentration of EGF is, for example, about 10 ng/ml or more, preferably about 20 ng/ml or more, more preferably about 30 ng/ml or more. The concentration of EGF is, for example, about 100 ng/ml or less, preferably about 80 ng/ml or less, more preferably about 60 ng/ml or less. For example, it is about 10 to about 100 ng/ml, about 20 to about 80 ng/ml, or about 30 to about 60 ng/ml. Particularly preferably, the concentration of EGF is about 50 ng/ml. EGF may include variants, fragments, or modified forms thereof as long as they have activity usable in this step.

The concentration of bFGF is, for example, about 5 ng/ml or more, preferably about 10 ng/ml or more, more preferably about 15 ng/ml or more. The concentration of bFGF is, for example, about 50 ng/ml or less, preferably about 30 ng/ml or less, more preferably about 25 ng/ml or less. For example, it is about 5 to about 50 ng/ml, about 10 to about 30 ng/ml, or about 15 to about 25 ng/ml. Particularly preferably, the concentration of bFGF is about 20 ng/ml. bFGF may include variants, fragments, or modified products thereof as long as they have activity usable in this step.

The medium for maintenance and expansion of hPGC or hPGCLC preferably further contains SCF. The concentration of SCF is, for example, about 30 ng/ml or more, preferably about 50 ng/ml or more, more preferably about 80 ng/ml or more. The concentration of SCF is, for example, about 200 ng/ml or less, preferably about 150 ng/ml or less, more preferably about 120 ng/ml or less. In a preferred embodiment, the concentration of SCF may be appropriately selected from the range of about 50 to about 150 ng/ml, preferably about 80 to about 120 ng/ml. Particularly preferably, the concentration of SCF is about 100 ng/ml. SCF may include variants, fragments, or modified forms thereof as long as they have activity usable in this step.

In the maintenance and expansion of mPGCLC, the addition of cyclosporin A in addition to forskolin and a PDE4 inhibitor further improves expansion efficiency. However, in the maintenance and expansion method of the present invention, it is desirable to not use cyclosporin A because it rather inhibits the maintenance and expansion of mPGCLC.

In the maintenance and expansion method of hPGC or hPGCLC, hPGC or hPGCLC may be cultured in the presence or absence of feeder cells. The kind of the feeder cell is not particularly limited, and a feeder cell known per se can be used. For example, fibroblasts (mouse embryonic fibroblasts, mouse fibroblast cell line STO and the like) can be mentioned. The feeder cells are preferably inactivated by a method known per se, for example, treatment with radiation (gamma rays and the like), an anticancer agent (mitomycin C and the like), or the like. When the feeder cells are vulnerable to PDE4 inhibitors and/or forskolin, it is desirable to subculture feeder cells for several generations in the presence of these additives and acclimate them to the additives in advance.

The culture vessel to be used for the maintenance and expansion of hPGC or hPGCLC is not particularly limited, and those exemplified in the aforementioned 1.(2) can be similarly used.

In this cultivation, hPGC or hPGCLC is plated onto an culture vessel (feeder cells seeded thereon in advance) to obtain a cell density of, for example, about $10^4$ to $10^5$ cells/cm$^2$, preferably about 2 to 8 $\times$ $10^4$, and cultured in an incubator under atmospheric conditions of 1 to 10% $CO_2$/99 to 90% air at about 30 to 40°C, preferably about 37°C, for 3 to 14 days, preferably 4 to 12 days, more preferably 5 to 10 days. As a result of culture, hPGCLC colonies are formed, BLIMP1 and TFAP2C are strongly expressed, and the properties of hPGC mobile phase are maintained.

After culturing for the above-mentioned period, hPGC or hPGCLC can be sorted and purified using hPGC or hPGCLC marker positivity as an index, passaged in a medium having the same composition, and continuously cultured. For example, when hPGCLC having a fluorescent protein gene under control of BLIMP1- and/or TFAP2C-promoter is used, hPGCLC can be sorted by FACS using, for example, BLIMP1 and/or TFAP2C positivity as an index. When hPGCLC induced to differentiate from hiMeLC for 5 to 8 days, preferably about 6 days, is used as the starting material, no cells negative for either BLIMP1 or TFAP2C substantially appear even after long-term passage culture. Therefore, either BLIMP1 or TFAP2C can be used as a selection marker. In the case of hPGCLC that was induced to differentiate from hiMeLC for 4 days or less, BLIMP1-positive/TFAP2C-negative cells may appear during passage, even though the number thereof may be small. Therefore, it is desirable to use TFAP2C positivity or double positivity with BLIMP1 as an index. Alternatively, when hPGC or hPGCLC without a transgenic reporter is used, it is preferable to sort and purify hPGC or hPGCLC expanded by FACS analysis or the like by using one or more types of cell surface antigens specifically expressed in hPGC or hPGCLC. Examples of the cellular surface antigen include at least one marker gene selected from the group consisting of PECAM (CD31), INTEGRIN$\alpha$6 (CD49f), INTEGRIN$\beta$3 (CD61), KIT (CD117), EpCAM, PODOPLANIN and TRA1-81, with preference given to the combination of INTEGRIN$\alpha$6 and EpCAM.

According to the maintenance and expansion culture of the present invention, culture can be performed for at least 30 days even by sorting using a surface antigen marker as an index, and for at least 120 days by sorting using BLIMP1 and TFAP2C as indices, where hPGCLC can be expanded up to about 1 million folds by 120 days of culture. Gell et al. (Stem Cell Reports, 2020, doi:10.1016/j.sterner.2020.01.009) showed that hPGCLC can be cultured in vitro using the maintenance and expansion methodology of mPGCLC developed by the present inventors. The expansion efficiency then was about 2-fold in 10 days. Considering this, the high expansion efficiency by the maintenance and expansion method of the present invention is surprising.

As described below, Wnt signal transduction inhibitors can suppress dedifferentiation of hPGC or hPGCLC. Therefore, in the maintenance and expansion culture of the present invention, the dedifferentiation of hPGC or hPGCLC can be suppressed and the maintenance efficiency of hPGC or hPGCLC can be improved by culturing hPGC or hPGCLC under conditions further including a Wnt signal transduction inhibitor. To be specific, a Wnt signal transduction inhibitor may be added to the above-mentioned medium for the maintenance and expansion culture of the present invention.

The Wnt signal transduction inhibitor is not particularly limited as long as the dedifferentiation of hPGC or hPGCLC can be suppressed, and examples thereof include substances that promote the degradation and/or inhibit nuclear translocation of β-catenin. More specifically, substances that stabilize auxin can be mentioned. More particularly, substances that inhibit tankylase (e.g., IWR1, XAV939, IWR2, JW55, JW74, G007-LK, NVP-TNKS656, WIKI4) can be mentioned. Other examples include substances that inhibit Wnt secretion (e.g., Wnt-C59, IWP2). Preferably, it is a substance that promotes the degradation and/or inhibits nuclear translocation of β-catenin, more preferably a substance that stabilizes axin, further preferably a substance that inhibits tankyrase. Particularly preferably, it may be IWR1 or XAV939. One kind of these Wnt signal transduction inhibitors may be used alone, or two or more kinds thereof may be used in combination.

The concentration of the Wnt signal transduction inhibitor is about 0.1 $\mu$M or more, preferably about 0.5 $\mu$M or more, more preferably about 1 $\mu$M or more. Also, the concentration of the Wnt signal transduction inhibitor is, for example, about 100 $\mu$M or less, preferably about 50 $\mu$M or less, more preferably about 30 $\mu$M or less. In a preferred embodiment, the concentration of the Wnt signal transduction inhibitor can be appropriately selected within the range of about 0.5 to about 50 $\mu$M, preferably about 1 to about 30 $\mu$M. For example, it is about 0.5 $\mu$M, about 1 $\mu$M, about 1.5 $\mu$M, about 2 $\mu$M, or about 2.5 $\mu$M. The Wnt signal transduction inhibitor may include derivatives, salts, or solvates thereof as long as they have activity usable in this step.

The Wnt signal transduction inhibitor may be added throughout the whole period of the maintenance and expansion culture of the present invention, or may be (continuously or intermittently) added for any period during the maintenance culture period. The lower limit of the addition period is not also particularly set, and examples thereof include 1 day or more, 2 days or more, 3 days or more, 4 days or more, 5 days or more, 10 days or more, 15 days or more, 20 days or more, and the like.

The expanded hPGC, or hPGC of hPGCLC population, or hPGCLC obtained by the. maintenance and expansion method of the present invention can be cryopreserved by a method known per se. The cryopreserved hPGC or hPGCLC can be thawed by a method known per se, and then subjected again to culture. The thawed hPGC or hPGCLC may be further cultured by the maintenance and expansion method of the present invention.

Analysis of the gene expression state confirmed that the expanded hPGCLC obtained by the maintenance and expansion method of the present invention proliferated while maintaining the properties of the initial hPGC. Analysis of the genomic DNA methylation state also revealed that hPGCLC generally maintained the DNA methylation state during the culture period. This is in contrast to the mPGCLC maintenance and expansion culture system, in which genome-wide DNA demethylation associated with proliferation was observed,
and suggests that the reorganization of epigenomic information may progress in humans by a mechanism different from that in mice.

To further verify the function of the expanded hPGCLC, xenogenic reconstruction ovary (xrOvaries) culture mimicking the intraovarian environment was performed. As a result, hPGCLC-derived cells expressed genes such as DDX4 (VASA homologue) and DAZL, and differentiated into oogonium-like/gonocyte-like cells with morphological characteristics of oogonium. Therefore, it was confirmed that the properties as germ cells are maintained .in this expansion culture system.

Accordingly, the present invention also provides a method for producing a cell population containing hPGC or hPGCLC, including a step of maintaining and expanding hPGC and/or hPGCLC by the aforementioned method. The cell population obtained by this method may consist of a single cell type or may contain multiple cell types. For example, the cell population contains about 50% or more, about 60% or more, about 70% or more, about 80% or more, about 90% or more, about 95% or more of hPGC or hPGCLC. The expanded hPGC or hPGCLC population can be further purified from this cell population by using a method such as FACS and using the expression of the aforementioned hPGC marker as an index.

[II] Reagent kit for maintenance and expansion of the present invention

As described above, the hPGC or hPGCLC maintenance and expansion culture method of the present invention includes culturing hPGC or hPSC-derived hPGCLC in the presence of

(i) a forskolin or PDE4 inhibitor, and
(ii) one or more cytokines selected from the group consisting of bFGF, LIF, and EGF.

Therefore, the present invention also provides a reagent kit for maintaining and expanding hPGC or hPGCLC, containing, as a constitution, (a) a forskolin or PDE4 inhibitor, and (b) one or more cytokines selected from the group consisting of bFGF, LIF, and EGF. Preferably, the reagent kit of the present invention contains forskolin as the drug of the aforementioned (a), and preferably a combination of LIF and EGF, FGF alone, or a combination of bFGF, LIF, and EGF, as cytokine of the aforementioned (b). The reagent kit of the present invention may further contain a Wnt signal transduction inhibitor as a constitution. As the Wnt signal transduction inhibitor, one or more kinds of those recited above for the maintenance and expansion method of the present invention can be mentioned. These ingredients may be supplied in a state dissolved in water or an appropriate buffer, and may also be supplied as a lyophilized powder which may be used after being freshly dissolved in an appropriate solvent. These ingredients may be supplied as individual reagents in respective kits, and, as far as they do not adversely affect each other, they can be supplied as a single mixed reagent of 2 kinds or more.

The reagent kit of the present invention may further contain, as a constitution, an antibody against a surface antigen marker of hPGC or hPGCLC, preferably an antibody against INTEGRIN$\alpha$6 and/or an antibody against EpCAM, as a reagent for sorting (preferably FACS) hPGC or hPGCLC expanded in the maintenance and expansion method of the present invention.

The reagent kit of the present invention may contain a medium that can be preferably used in the maintenance and expansion method of the present invention. Examples of such preferred medium include Dulbecco's Modified Eagle's Medium (DMEM) with a glucose concentration of 1 g/L.

[III] Expanded hPGCLC of the present invention

hPGCLC expanded by the maintenance and expansion method of the present invention can be maintained and expanded for an extremely long period of time while retaining properties similar to those of early hPGCs, compared with a culture system in which the methodology of the maintenance and expansion culture of mPGCLC is directly applied. The expanded hPGCLC maintained for a long period of time by passage culture show a markedly different transcriptome profile, compared with hPGCLC immediately after differentiation induction from hiMeLC, and shows a survival rate and a proliferation rate superior to those of hPGCLC immediately after differentiation induction (d6), in aggregation culture (xrOvaries) with xenogeneic ovarian somatic cells of mouse and the like. Therefore, it can be said that the expanded hPGCLC obtained by the maintenance and expansion method of the present invention is a heterogeneous cell, as a substance, from the conventionally-known hPGCLC.

Therefore, the present invention also provides expanded hPGCLC obtained by the maintenance and expansion method of the present invention. It is impossible oralmost impractical to directly specify such expanded hPGCLC by the structure or property thereof. In thebelow-mentioned [CLAIMS], therefore, the expanded hPGCLC is specified by the production method thereof. The hPGCLC may be provided in a suspended state in the above-mentioned medium for maintenance and expansion, or in a cryopreserved state by a method known per se in another preferred embodiment.

[IV] Production method of oogonium-/gonocyte-like cell from hPGC or hPGCLC

As described in Science 362: 356-360 (2018) and Examples described later, the expanded hPGC or hPGCLC obtained by the maintenance and expansion method of the present invention can express genes such as DDX4 (VASA homologue) and DAZL in vitro, and can be differentiated into oogonium-like/gonocyte-like cells with morphological characteristics of oogonium, by causing aggregation with somatic cells derived from ovary of mammals, such as non-human mammals (e.g., mouse), to form reconstructed ovary (r ovary), such as xenogenic reconstructed ovary (xrOvaries), and culturing same. The expanded hPGC or hPGCLC used here may be hPGC or hPGCLC purified from a cell population obtained by the aforementioned method for producing a cell population containing hPGC or hPGCLC.

[V] Evaluation method of maintenance and expansion culture system for hPGC or hPGCLC

The present invention also provides an evaluation method of a maintenance and expansion culture system for hPGC or hPGCLC. The method characteristically includes the following steps:

(1) a step.of culturing hPGC or hPGCLC on feeder cells under given conditions,
(2) a step of sorting viable cells from a cell population obtained in (1),
(3) a step of sorting the viable cells obtained in (2) into hPGC marker-positive cells and other cells,
(4) a step of subjecting the cells obtained in (3) other than the hPGC marker-positive cells to flow cytometry, and distinguishing cells dedifferentiated from the hPGC or hPGCLC and the feeder cells by two dimensional plots of FSC/SSC, and
(5) a step of evaluating a maintenance efficiency of the hPGC or hPGCLC under the aforementioned given conditions, based on the number of the hPGC marker-positive cells obtained in (3) and the number of the dedifferentiated cells obtained in (4).

Generally, in a maintenance and expansion culture system using feeder cells, in order to measure the number of human cells dedifferentiated from expanded hPGC, or hPGCLC and hPGC, or hPGCLC, dead cells are first removed from the hPGC or hPGCLC population cultured in the culture system to be evaluated to sort viable cells, and then feeder cells are removed to sort cell populations containing expanded hPGC or hPGCLC and dedifferentiated human cells, and the expanded hPGC or hPGCLC and dedifferentiated human cells are sorted with the presence or absence of hPGC marker as an index. However, by using the evaluation method of the present invention and selecting appropriate feeder cells, a viable cell population can be sorted into hPGC marker-positive cells (expanded hPGC or hPGCLC) and the marker-negative cells (cell population containing dedifferentiated human cells and feeder cells) without sorting a cell population containing expanded hPGCs or hPGCLCs and dedifferentiated human cells, and feeder cells. The hPGC marker-negative cells are analyzed by FACS and subjected to an FSC/SSC two-dimensional plot, whereby the dedifferentiated human cells and the feeder cells can be distinguished from the distribution patterns thereof (both shows mutually exclusive FSC/SSC pattern), and the number of the dedifferentiated human cells can be measured.

The feeder cell used in the evaluation method of the present invention is not particularly limited as to the animal origin or cell type as long as it can support the maintenance and expansion of hPGC or hPGCLC and shows a distribution that can be clearly distinguished from dedifferentiated human cells by FSC/SSC two-dimensional plot when subjected to FACS analysis in the co-presence of human cells dedifferentiated from hPGC or hPGCLC. For example, m220-5 cells can be mentioned as mouse cells.

The "given conditions" in step (1) refers to various conditions used for specifying the culture system to be evaluated by the evaluation method of the present invention, such as combinations of basal medium composition, kind and concentration of medium additive, type of culture vessel, culture temperature, $CO_2$ concentration, culture format, culture period, and the like, and varies depending on the culture system to be evaluate. Specific examples of the "given conditions" include, but are not limited to, culture conditions that can support the maintenance and expansion of existing hPGC or hPGCLC (e.g., any combination of various conditions described in WO 2019/107576), any combination of the above-mentioned various conditions of the maintenance and expansion method of the present invention, and conditions in which one or more conditions are changed from those culture conditions (e.g., addition or deletion of medium additives, replacement with another medium additive, and the like).

Viable cells in step (2) can be sorted by, for example, subjecting the cell population obtained in step (1) to FACS or the like using dead cell markers known per se (e.g., DRAQ-7, DRAQ-5, TO-PRO-3, etc.) and viable cell markers.

In step (3), the viable cell population obtained in step (2) is sorted into marker-positive cells and negative cells by FACS or the like using an hPGC marker, and the former is taken as expanded hPGC or hPGCLC and the number thereof is calculated. The hPGC marker is, for example, BLIMP1 and/or TFAP2C, or at least one marker selected from the group consisting of ECAM (CD31), INTEGRINα6 (CD49f), INTEGRINβ3 (CD61), KIT (CD117), EpCAM, PODOPLANIN, and TRA1-81, preferably the combination of INTEGRINα6 and EpCAM, or the like. More preferably, BLIMP1 and TFAP2C double-positive cells are sorted as hPGC marker-positive cells, i.e., hPGC or hPGCLC.

In step (4), the hPGC marker-negative cells obtained in step (3), that is, cell populations other than hPGC or hPGCLC, are subjected to FACS analysis to create an FSC/SSC two-dimensional plot. Human cells dedifferentiated from hPGC or hPGCLC and feeder cells show mutually exclusive FSC/SSC patterns on the two-dimensional plot. Therefore, they can be easily distinguished and the number of the dedifferentiated human cells can be measured.

In step (5), the maintenance efficiency of hPGC or hPGCLC under the aforementioned given conditions is evaluated by comparing the number of hPGC marker-positive cells (expanded hPGC or hPGCLC) obtained in step (3) with the number of cells dedifferentiated from hPGC or hPGCLC obtained in (4). It can be said that a larger number of expanded hPGC or hPGCLC cells and a smaller number of dedifferentiated cells indicate higher maintenance efficiency of hPGC or hPGCLC.

In preferable one embodiment, the evaluation in step (5) is performed based on the enrichment score represented by the following formula:

$$\text{enrichment score} = \log_2 (\text{number of hPGC marker-positive}$$
$$\text{cells/number of dedifferentiated cells}).$$

When the enrichment score is positive, it indicates that the number of expanded hPGC or hPGCLC cells is dominant.

A dedifferentiation inhibitor of hPGC or hPGCLC can be screened for by using the evaluation method of the present invention. Therefore, the present invention also provides a method for screening for an hPGC or hPGCLC dedifferentiation inhibitor.

In this method, hPGC or hPGCLC is cultured on feeder cells under given conditions in the presence or absence of a candidate dedifferentiation inhibitor, similar to the above-mentioned step (1) of the evaluation method of the present invention. As used herein, the "given conditions" are as defined above. Furthermore, a hPGC or hPGCLC population cultured in the presence or absence of a candidate substance is subjected to steps similar to steps (2) to (5) of the above-mentioned evaluation method of the present invention, and the maintenance efficiency of each of hPGC and hPGCLC is calculated. When the maintenance efficiency in the presence of a candidate substance is high as compared with the maintenance efficiency in the absence of the candidate substance, the candidate substance can be selected as a dedifferentiation inhibitor of hPGC or hPGCLC.

As shown in the below-mentioned Examples, the present inventors have found Wnt signal transduction inhibitors as the dedifferentiation inhibitors of hPGC or hPGCLC by using this screening method, and demonstrated the effectiveness of the screening system.

[VI] Method for maintaining and expanding PGC, PGCLC, etc. using Wnt signal transduction inhibitors

As described above, Wnt signal transduction inhibitors can suppress dedifferentiation of PGC or PGCLC. Therefore, the present invention also provides a method for maintaining and expanding PGC or PGCLC, comprising culturing PGC or PGCLC in the presence of a Wnt signal transduction inhibitor.

The method can be used for PGCs or PGCLCs derived not only from humans but also from other mammals (e.g., mouse etc.).

In the method for maintaining and expanding PGC or PGCLC by using a Wnt signal transduction inhibitor, culture is performed, for example, under conditions that can support the maintenance and expansion of PGC or PGCLC known per se. For example, WO 2019/107576 describes that PGCs or PGCLCs can be maintained and expanded by culturing PGCs or PGCLCs in the presence of forskolin and/or PDE4 inhibitors, using mice as an example. In the case of hPGCs or hPGCLCs, forskolin and a PDE4 inhibitor may be used in combination, as shown in the below-mentioned Examples, in which use of either one alone shows better maintenance and expansion efficiency. Therefore, even in the maintenance and expansion of PGCs or PGCLCs using Wnt signal transduction inhibitors, the culture is preferably performed under conditions further containing forskolin and/or a PDE4 inhibitor.

When PGC or PGCLC is a human cell, the culture is more preferably performed under the conditions

(i) further containing forskolin or a PDE4 inhibitor, and/or

(ii) further containing one or more cytokines selected from the group consisting of bFGF, LIF, and EGF, in addition to Wnt signal transduction inhibitors, and a combined use of (i) and (ii) is further preferred. In this case, the concentration of various additives, the composition of the basal medium, the culture temperature, the culture period, and the like can be appropriately selected by referring to the above-mentioned maintenance and expansion method of the present invention.

On the other hand, when PGC or PGCLC is a cell of a non-human animal such as mouse and the like, the culture is more preferably performed under the conditions

(i) further containing forskolin and a PDE4 inhibitor, and/or

(ii) further containing cyclosporin A (CsA), in addition to Wnt signal transduction inhibitors, and a combined use of (i) and (ii) is further preferred. In this case, the concentration of various additives, the composition of the basal medium, the culture temperature, the culture period, and the like can be appropriately selected by referring to, for example, WO 2019/107576.

The Wnt signal transduction inhibitor is not particularly limited as long as the dedifferentiation of hPGC or hPGCLC can be suppressed, and examples thereof include substances that promote the degradation and/or inhibit nuclear translocation of $\beta$-catenin (e.g., IWR1, XAV939, IWR2, JW55, JW74, G007-LK, NVP-TNKS656, WIKI4), substances that inhibit Wnt secretion (e.g., Wnt-C59, IWP2), and the like. Preferably, it is a substance that promotes the degradation and/or inhibits nuclear translocation of $\beta$-catenin, more preferably IWR1 or XAV939. One kind of these Wnt signal transduction inhibitors may be used alone, or two or more kinds thereof may be used in combination.

The concentration of the Wnt signal transduction inhibitor is about 0.1 $\mu$M or more, preferably about 0.5 $\mu$M or more, more preferably about 1 $\mu$M or more. Also, the concentration of the Wnt signal transduction inhibitor is, for example, about 100 $\mu$M or less, preferably about 50 $\mu$M or less, more preferably about 30 $\mu$M or less. In a preferred embodiment, the concentration of the Wnt signal transduction inhibitor can be appropriately selected within the range of about 0.5 to about 50 $\mu$M, preferably about 1 to about 30 $\mu$M. For example, it is about 0.5 $\mu$M, about 1 $\mu$M, about 1.5 $\mu$M, about 2 $\mu$M, or about 2.5 $\mu$M.

The Wnt signal transduction inhibitor may be added throughout the whole period of the maintenance and expansion culture, or may be (continuously or intermittently) added for any period during the maintenance culture period.

The lower limit of the addition period is not also particularly set, and examples thereof include 1 day or more, 2 days or more, 3 days or more, 4 days or more, 5 days or more, 10 days or more, 15 days or more, 20 days or more, and the like.

The above-mentioned method for maintaining and expanding PGC or PGCLC is intended to improve the maintenance efficiency of PGC or PGCLC by suppressing the dedifferentiation of PGC or PGCLC in the maintenance and expansion culture by the action of Wnt signal transduction inhibitors. Therefore, in another aspect, the present invention provides a method for suppressing dedifferentiation of PGC or PGCLC, comprising, in maintenance and expansion culture of PGC or PGCLC, culturing the cells in the presence of a Wnt signal transduction inhibitor.

The present invention also provides a PGC or PGCLC dedifferentiation inhibitor containing a Wnt signal transduction inhibitor. As the Wnt signal transduction inhibitors, those similar to the above-mentioned inhibitors can be used. Wnt signal transduction inhibitors may be provided in the form of being dissolved in water or a suitable buffer, provided as a freeze-dry powder and can also be used upon dissolution in a suitable solvent when in use.

In the process of inducing differentiation of pluripotent stem cells (PSCs) into PGCLCs, Wnt signal transduction inhibitors can also suppress the appearance of cells that do not differentiate into PGCLCs. Therefore, the present invention also provides a method for producing PGCLCs, including inducing differentiation of PSCs into PGCLCs in the presence of a Wnt signal transduction inhibitor.

As a method for inducing differentiation of PSCs into PGCLCs, for example, the above-mentioned direct differentiation induction method from PSCs into PGCLCs, or a differentiation induction method via early mesoderm-like cells (iMeLC) can be used. The Wnt signal transduction inhibitor may be added throughout the whole period of the differentiation induction culture, or may be (continuously or intermittently) added for any period during the maintenance culture period.

When used in the present specification, unless otherwise indicated, the term "about" referring to a numerical value or range allows for some variation in that value or range. For example, it can mean up to ±10% or ±5% of the stated value or stated range limits. The present invention is explained in more specifically in the following by referring to Examples. It is needless to say that the present invention is not limited thereto.

[Example]

Example 1

<Materials>

Information on reagents and other resources used in. Example 1 is summarized in Tables 1-1 to 1-4.

[Table 1-1]

| Reagent/Resource | Reference or Source | Identifier or Catalog Number |
|---|---|---|
| **Experimental Models** | | |
| 585B1-BTAG | Sasaki et al., 2015. | BTAG 585B1-868 |
| 1390G3-AGVT | Yamashiro et al., 2018. | 1390G3 AGVT_5-2-6 |
| 1383D6 | Yokobayashi et al., 2017. | 1383D6 |
| m220 | Ohta et al., 2017. | N/A |
| ICR | Shimizu Laboratory Suppliers | Slc:ICR |
| **Antibodies** | | |
| mouse anti-BLIMP1, monoclonal | R&D Systems | Cat # MAB36081 |
| goat anti-SOX17, polyclonal | R&D Systems | Cat # AF1924 |
| mouse anti-AP2γ/TFAP2C, monoclonal | Santa Cruz | Cat # sc-12762 |
| mouse anti-OCT4/POU5F1, monoclonal | Santa Cruz | Cat # sc-5279 |
| goat anti-NANOG, polyclonal | R&D Systems | Cat # AF1997 |
| goat anti-SOX2, polyclonal | Santa Cruz | Cat # sc-17320 |
| goat anti-FOXL2, polyclonal | Novus Biologicals | Cat # NB100-1277 |
| mouse anti-DAZL, monoclonal | Santa Cruz | Cat # sc-390929 |
| goat anti-DDX4, polyclonal | R&D Systems | Cat # AF2030 |
| mouse anti-human | Millipore | Cat # MAB1273 |

(continued)

| Antibodies | | |
|---|---|---|
| Mitochondria, monoclonal | | |
| rat anti-GFP, monoclonal | Nacalai Tesque | Cat # 04404-84 |
| mouse anti-UHRF1, monoclonal | Millipore | Cat # MABE308 |
| rabbit anti-DNMT1, polyclonal | abcam | Cat # ab19905 |
| rat anti-CD49f/ITGA6 conjugated with Brilliant Violet 421, monoclonal | BioLegend | Cat # 313624 |
| mouse anti-EpCAM conjugated with APC, monoclonal | BioLegend | Cat # 324208 |
| mouse anti-TRA-1-85 conjugated with Brilliant Violet 421, monoclonal | BD Horizon | Cat # 563302 |
| anti-SSEA-1 antibody, monoclonal | Miltenyi Biotec | Cat # 130-094-530 |
| anti-CD31 antibody, | Miltenyi Biotec | Cat # 130-097-418 |

[Table 1-2]

| monoclonal | | |
|---|---|---|
| donkey anti-rat IgG conjugated with Alexa Fluor 488 | Invitrogen | Cat # A21208 |
| donkey anti-mouse IgG conjugated with Alexa Fluor 568 | Invitrogen | Cat # A10037 |
| donkey anti-mouse IgG conjugated with Alexa Fluor 647 | Invitrogen | Cat# A31571 |
| donkey anti-goat IgG conjugated with Alexa Fluor 647 | Invitrogen | Cat# A21447 |
| donkey anti-rabbit IgG conjugated with Alexa Fluor 647 | Invitrogen | Cat # A31573 |
| **Oligonucleotides and sequence-based reagents** | | |
| qPCR primers | present invention | Table 3 |
| **Chemicals, enzymes and other reagents** | | |
| Stem Fit AK03/AK03N | Ajinomoto | N/A |
| iMATRIX | Nippi | 892014 |
| TrypLE select | Gibco | 12563-011 |
| EDTA | Nacalai-Tesque | 06894-14 |
| trypsin-EDTA | Gibco | 15400-054 |
| Dnase I | Sigma | DN25 |
| knockout serum replacement | Gibco | 10828-128 |
| NEAA | Gibco | 11140-050 |
| penicillin & streptomycin | Gibco | 15140-122 |
| L-glutamine | Gibco | 25030-081 |
| sodium pyruvate | Gibco | 11360-070 |
| 2-mercaptoethanol | Gibco | 21985-023 |
| FBS | Gibco / Sigma | 10437-028 /F7524 |
| BSA for FACS buffer | Gibco | 15260-037 |
| BSA for immunofluorescence | Sigma | A3059 |
| normal donkey serum | Jackson ImmunoResearch | 017-000-121 |
| Triton-X 100 | Nacalai-Tesque | 35501-02 |
| ibidi mounting media | ibidi | 50001 |
| mitomycin C | Sigma / Kyowa Kirin | M0503 / MITOMYCIN Injection |

[Table 1-3]

| fibronectin | Merck Millipore | FC010 |
|---|---|---|

(continued)

| | | |
|---|---|---|
| ascorbic acid | Sigma | A4403 |
| Dnase-fice water | Gibco | 15230-160 |
| unmethylated λ phage DNA | Promega | D1521 |
| Phusion Hot Start II DNA polymerase | Thermo Fisher Scientific | F-549S |
| Axy-preg MAG PCR Clean-Up Kit | Coming | MAG-PCR-CL-250 |
| activin A | Peprotech | AF-120-14 |
| BMP4 | R&D Systems | 314-BP |
| SCF | R&D Systems | 255-SC |
| LIF | Merck Millipore | LIF1010 |
| EGF | R&D Systems / Peprotech | 236-EG / AF-100-15 |
| bFGF | Wako | 064-04541 |
| forskolin | Sigma | F3917 |
| rolipram | abcam | ab120029 |
| cyclosporin A | Sigma | 30024 |
| CHIR99021 | Tocris | 4423 |
| Y27632 | Wako / Tocris | 253-00513 / 1254 |
| DMEM(4.5 g/L glucose) | Gibco | 10313-021 |
| αMEM | Gibco | 32571-036 |
| other media used in the culture condition considerations | N/A | Table EV1 |
| **Software** | | |
| FV10-ASW | Olympus | N/A |
| XnConvert | https://www.xnview.com/ | N/A |
| R version 3.6.1 | R Core Team. 2019. | N/A |
| DAVID 6.8 | Huang et al., 2009. | N/A |
| Feature Extraction 12.1.0.3 | Agilent Technologies | N/A |
| Cytogenomics 5.0.2.5 | Agilent Technologies | N/A |
| cutadapt v 1.9.1 | Martin, 2011. | N/A |
| Tophat v2.1.0 | Kim, Pertea et al., 2013. | N/A |
| Bowtie2 v2.2.7 | Ben and Salzberg, 2012. | N/A |
| HTSeq v0.9.1 | Anders. Pyl et al., 2015. | N/A |
| Trim_galore program | http://www.bioinformatics.babra ham.ac.uk/projects/trim_galore | N/A |
| Bismark v0.17.0 | Krueger and Andrews, 2011. | N/A |
| Samtools v1.3 | Li, Handsaker et al., 2009. | *N/A* |
| IGVTools v2.3.52 | Robinson, Thorvaldsdottir et al., 2011 | N/A |

[Table 1-4]

| | | |
|---|---|---|
| Methpipe v3.4.3 | Song et al., 2013. | N/A |
| bedtools v2.29.2 | Quinlan et al., 2010. | N/A |
| Homer v4.9.1 | http://homer.ucsd.edu/homer | N/A |
| **Other** | | |
| Rneasy Micro Kit | QIAGEN | 74004 |
| Nucleospin RNA XS | Macherey-Nagel | U0902A |
| Nucleospin Tissue | Macherey-Nagel | U0952Q |
| EZ DNA Methylation-Gold Kit | ZymoResearch | D5005 |

(continued)

| Other | | |
|---|---|---|
| Qubit RNA-HS Assay Kit | In vitro gen | Q32855 |
| Power SYBR Green PCR Master Mix | Applied Biosystems | 4367659 |
| SureTag Complete DNA Labeling Kit | Agilent Technologies | 5190-4240 |
| SurePrint G3 Human CGH Microarray 8 × 60K | Agilent Technologies | G4405A |
| v-bottom 96-well plate | NOF / Greiner | 51011612 651970 |
| u-bottom 96-well plate | Thermo Fisher Nunc | 3496 |
| FALCON cell strainer | Coming | 352235 |
| film-bottom dish | Matsunami Glass | FD10300 |
| CKX41 inverted microscope | Olympus | N/A |
| DS-Fi2 | Nikon | N/A |
| M205C microscope | Leica | N/A |
| DP72 | Olympus | N/A |
| FV1000-IX81 confocal microscope system | Olympus | N/A |
| CFX384 Touch Real-Time PCR detection system | Bio-Rad Laboratories | N/A |
| Surescan Microarray scanner G2600D | Agilent Technologies | N/A |
| NextSeq500/550 | Illumina | N/A |
| Hiseq2500 | Illumina | N/A |

In addition, the basal medium used in Example 1 and the composition of the medium examined are summarized in Table 2.

[Table 2]

· Basal media

| Name in the formulation | company | cotalog# | glucose concentration | description |
|---|---|---|---|---|
| DMEM | GIBCO | 11054020 | 1.0 g/L | DMEM, low glucose, pyruvate, no glutamine, no phenol red |
| DMEM, no glucose | GIBCO | A1443001 | 0 g/L | DMEM, no glucose, no glutamine, no phenol red 500mL |
| GMEM | GIBCO | 11710035 | 4.5 g/L | Glasgow's MEM (GMEM) |
| DMEM/F12 | GIBCO | 10565018 | 3.151 g/L | DMEM/F-12. GlutaMAX™ |
| F12 | GIBCO | 31765035 | 1.802 g/L | Ham's F-12 Nutrient Mix, GlutaMAX™ |
| aMEM | GIBCO | 32571036 | 1.0 g/L | MEM α, Nucleosides, GlutaMAX™ |
| Temin's MEM | GIBCO | 11935046 | 9.0 g/L | MEM (Temin's modification) (2X), no phenol red |
| IMDM | GIBCO | 31980030 | 4.5 g/L | IMDM, GlutaMAX™ Supplement |
| RPMI | GIBCO | 61870036 | 2.0 g/L | RPMI 1640 Medium, GlutaMAX™ |
| CMRL | GIBCO | 11530037 | 1.0 g/L | CMRL Medium, no glutamine |

· Formulation of culture media examined

| Name in the text | basal medium | KSR | NEAA | Penicillin & Streptomycin | Sodium Pyruvate | Glutamax or L-Glutamine | 2-Mercaptoethanol | note |
|---|---|---|---|---|---|---|---|---|
| GMEM | GMEM | 15% | 1% | 1% | 2 mM | L-Glutamine, 2 mM | 1 mM | |
| DMEM(4.5g/L Glucose) | DMEM, no glucose | 15% | 1% | 1% | 2 mM | Glutamax, 2 mM | 1 mM | D-Glucose(Nacalaitesque, #16806-25) was supplemented to be 4.5g/L |
| DMEM(1.0g/L Glucose) | DMEM, no glucose | 15% | 1% | 1% | 2 mM | Glutamax, 2 mM | 1 mM | D-Glucose(Nacalaitesque, #16806-25) was supplemented to be 1.0g/L |
| DMEM(0.22g/L Glucose) | DMEM, no glucose | 15% | 1% | 1% | 2 mM | Glutamax, 2 mM | 1 mM | D-Glucose(Nacalaitesque, #16806-25) was supplemented to be 0.22g/L |
| DMEM/F12 | DMEM/F12 | 15% | 1% | 1% | 2 mM | Glutamax, 2 mM | 1 mM | Glutamax was included in the basal medium |
| F12 | F12 | 15% | 1% | 1% | 2 mM | Glutamax, 2 mM | 1 mM | Glutamax was included in the basal medium |
| aMEM | aMEM | 15% | 1% | 1% | 2 mM | Glutamax, 2 mM | 1 mM | Glutamax was included in the basal medium |
| Temin's MEM | Temin's MEM | 15% | 1% | 1% | 2 mM | Glutamax, 2 mM | 1 mM | Glutamax was included in the basal medium |
| IMDM | IMDM | 15% | 1% | 1% | 2 mM | Glutamax, 2 mM | 1 mM | Glutamax was included in the basal medium |
| RPMI | RPMI | 15% | 1% | 1% | 2 mM | Glutamax, 2 mM | 1 mM | Glutamax was included in the basal medium |
| CMRL | CMRL | 15% | 1% | 1% | 2 mM | Glutamax, 2 mM | 1 mM | |

<Experiment method>

Animals and hiPSC

All animal experiments were performed under the ethical guidelines of Kyoto University (Approval no.

MedKyo19001). All experiments using hiPSC were performed with the approval of the Institutional Board of Kyoto University and according to the guidelines of The Ministry of Education, Culture, Sports, Science and Technology.

Culture of hiPSC

The cells used in this Example were maintained in a humidified 5% $CO_2$ incubator at 37°C. hiPSC line 585B1 BTAG(46XY) (Sasaki et al., Cell Stem Cell. 2015 Aug 6; 17(2):178-94) and 1383D6 (46XY) (Yokobayashi et al., Biol Reprod. 2017 Jun 1; 96(6):1154-1166) were cultured in StemFit AK03 or AK03N (Ajinomoto) on a plate coated with laminin-511 E8 fragment (iMATRIX-511; Nippi, Inc., 892014). For passaging, the cells were dissociated into single cells by treating with a 1:1 mixture of TrypLE select (GIBCO, 12563-011), and PBS(-) containing 0.5 mM EDTA (Nacalai Tesque, 06894-14). At the time of plating, 10 μM ROCK inhibitor (Y-27632; WAKO, 253-00513 or TOCRIS1254) was added, and the medium was exchanged the next day with a fresh medium free of T27632. The image of hiPSC colony was taken with a CKX41 inverted microscope (Olympus) equipped with a DS-i2 camera (Nikon).

Induction of hPGCLC

As reported previously, hPGCLC was induced from hiPSC via iMeLcC (Sasaki et al, 2015 (aforementioned), Yokobayashi et al., 2017 (aforementioned)). In brief, MeLcC was induced from hiPSC (1.0 to $2.0{\times}10^5$ cells/well) in a GK medium [GMEM (GIBCO, 11710-035) containing 15% knockout serum replacement (KSR) (GIBCO, 10828-028), 1% MEM non-essential amino acid solution (NEAA) (GIBCO, 11140-050), 1% penicillin·streptomycin, 2 mM L-glutamine, 2 mM sodium pyruvate (GIBCO, 11360-070), and 0.1 mM 2-mercaptoethanol] supplemented with 3 μM CHIR99021 (TOCRIS, 4423), 50 ng/mL activin A (ProproTech, AF-120-14), and 10 μM Y-27632 on the wells of a 12-well plate coated with fibronectin (Millipore, FC010) (induction time was 44 to 48 hr for 585B1 BTAG hiPSC, and 60 hr for 1683D6 hiPSC). hPGCLC was induced from iMeLC in a GK15 medium supplemented with 200 ng/mL BMP4 (R&D SYSTEMS, 314-BP), 100 ng/mL SCF (R&D SYSTEMS, 255-SC), 10 ng/mL LIF(Merck Millipore, LIF1010), 50 ng/mL EGF(R&D SYSTEMS, 236-EG or ProproTech AF-100-15), and 10 μM Y-27632 on a V-bottom 96-well plate (NOF, 51011612 or Greiner, 651970). After induction of hPGCLC for 4 to 6 days, hPGCLC was isolated by FACS sorting[see the section of Fluorescence activated cell sorting]. The images of iPSC and iMeLC were taken with a CKX41 inverted microscope (Olympus) equipped with a DS-Fi2 camera (Nikon). The image of iMeLC aggregate under the hPGCLC induction conditions was taken with a M205C microscope equipped with a DP72 camera (Olympus).

xrOvary culture

The xrOvary was prepared and cultured as previously reported (Yamashiro et al., Science. 2018 Oct 19; 362(6412) :356-360). In brief, $5.0{\times}10^3$ d6 hPGCLC or d6c30 hPGCLC were aggregated at E12.5 with $7.5{\times}10^4$ ICR strain mouse embryonic ovarian somatic cells (Shimadzu Laboratory Supply). After a floating culture in GK cells containing 10 μM Y-27632 for 2 days in U-bottom 96-well plate (Thermo Fisher Nunc, 174925) to form aggregates, the aggregates were transferred and cultured in a xrOvary culture medium [αMEM containing 10% FBS, 1% penicillin·streptomycin, 150 μM L-ascorbic acid (Sigma, A4403), and 55 μM 2-mercaptoethanol] on Transwell-COL membrane insert (Corning, 3496) under liquid-gas interface conditions. The xrOvary culture medium was exchanged with a fresh medium every 3 days. Isolation of mouse embryonic ovarian somatic cells was performed as previously described (Hayashi & Saitou, Nat Protoc. 2013 Aug; 8(8):1513-24, Yamashiro et al., 2018 (aforementioned)). For magnetic-activated sorting, dissociated somatic cells were incubated with anti-SSEA-1 antibody (Miltenyi Biotec, 130-094-530) and anti-CD31 antibody (Miltenyi Biotec, 130-097-418) for 20 to 40 min on ice. For statistical analysis of the number of $BT^+AG^+$ cells in Xr ovary, Welch's t-test was performed with the t-test function using the "var.equal=F" option in R software version 3.6.1.

Fluorescence activated cell sorting (FACS)

As previously reported (Sasaki et al, 2015 (aforementioned), Yokobayashi et al., 2017 (aforementioned)), hPGCLC was isolated from iMeLS cell aggregates. Typically, aggregates were washed once with PBS(-) and incubated with a 1:1 mixture of 0.5% trypsin-EDTA (GIBCO, 15400-054) and PBS(-) for 15 min. The aggregates were dispersed by pipetting and trypsin treatment was neutralized with STOP medium [DMEM (GIBCO, 10313-021) containing 10% FBS, 1% penicillin·streptomycin, 2 mM L-glutamine (Sigma, A4403), 10 μM Y-27632, and 0.1 mg/mL DNase I]. After pelleting, the cells were resuspended in FACS buffer and filtered through a FALCON cell strainer (Corning, 352235).

To isolate hPGCLCs with cell markers (Sasaki et al., 2015 (aforementioned), Yokobayashi et al., 2017 (aforementioned)), dissociated iMeLC aggregates were incubated with BV421-binding anti-CD49f (INTEGRINα6) antibody (Biolegend, 313624) and APC-binding CD326 (EpCAM) antibody (Biolegend, 324208) in FACS buffer in the dark for 15 min on ice and washed once with PBS(-). After pelleting, the cells were resuspended in FACS buffer and filtered through a FALCON cell strainer.

BT$^+$AG$^+$ or INTEGRINα6$^{high or low}$/EpCAM$^{high}$ cells were sorted by FACSAriaIII (BD Bioscience). For expansion culture, cells were sorted in FACS buffer. In order to collect cells for the following analyses (RT-qPCR, RNA-seq, and whole-genome bisulfite sequencing (WGBS)), cells were sorted in cellotion (ZENOAQ, CB051). For xrOvary culture, BT$^+$AG$^+$ cells were sorted in GK15 medium supplemented with 10 μM Y-27632.

For passage of expanded hPGCLC, cells were washed once with PBS(-) and treated in a 1:4 mixture of 0.5% trypsin-EDTA and PBS(-) at 37°C for 5 min. After vigorous pipetting, trypsin treatment was neutralized with STOP medium or PBS(-) containing 10% FBS, 10 μM Y-27632, and 0.1 mg/mL DNase. The cells were then pelleted and resuspended in FACS buffer. Cells were subjected to FACS sorting after removing intercellular clamp provided with FALCON cell strainer. In order to passage expanded hPGCLCs based on CD49f and CD326 expression, dissociated cells were immunolabeled as previously described. The BT$^+$AG$^+$ or INTEGRINα6$^{hign or low}$/EpCAM$^{high}$ cells were isolated with FACSAriaIII. For passage, the T$^+$AG$^+$ or INTEGRINα6$^{high or low}$/EpCAM$^{high}$ cells were sorted in FACS buffer.

To analyze TRA-1-85 expression in hPGCLC-derived cells in maintenance and expansion culture, cells suspended in FACS buffer were incubated with anti-TRA-1-85 antibody (BD Horizon, 563302) on ice and in the dark for 30 min and washed once with PBS (-). The cells were pelleted, resuspended in FACS buffer, and passed through a FALCON cell strainer. To the cell suspension was added 3 mM DRAQ7 (abcam, ab109202), and the cells were incubated at room temperature for 10 min and subjected to FACS analysis.

hPGCLC expansion culture

The m220-5 cell line (Dolci et al., Nature. 1991 Aug 29; 352(6338) :809-11; Majumdar et al., J Biol Chem. 1994 Jan 14; 269(2) :1237-42; Ohta et al., EMBO J. 2017 Jul 3; 36(13) :1888-1907; Miyauchi et al., Methods Cell Biol. 2018; 144:409-429) was maintained in DMEM (GIBCO, 10310-021) containing 10% FBS (GIBCO 10437-028 or SIGMA F7524), 1% penicillin·streptomycin (GIBCO, 15140-122), and 2 mM L-glutamine (GIBCO, 25030-081). For hPGCLC expansion culture, mitomycin-C (MMC, SIGMA M0503 or KYOWA KIRIN MITOMYCIN for injection: 2 or 4 ug/ml, 2 hr)-treated m220-5 cells were used as feeder cells. Prior to MMC treatment, m220-5 cells were passaged with 10 μM forskolin (Sigma, F3917) and 10 μM rolipram (abcam, ab120029) for expansion and adaptation to the added chemical substances.

In order to examine the influence of cytokine and/or chemical substances on hPGCLC proliferation, hPGCLC was treated with MMC in GK15 containing 2.5% FBS and 100 ng/mL SCF, and supplemented with cytokine and/or chemical substances [10 ng/mL LIF; 50 ng/mL EGF; 20 ng/mL bFGF; 10 μM forskolin; 10 μM rolipram; 5 μM cyclosporin A (SIGMA, 30024)], and the cells were passaged every 10 days [see the section of Fluorescence activated cell sorting]. On the day of passage, about $5.0 \times 10^3$ cells were plated on 24-well plates (0.5 mL of medium containing 10 μM Y27632), and 0.5 mL of medium free of Y27632 was 0.5 mL added the next day. On day 3, the whole medium was exchanged (1 mL/well). A half of the medium was exchanged with a fresh medium on days 5, 7, and 9 after passage. In order to evaluate the effect of basal medium and glucose concentration, hPGCLCs were cultured on MMC-treated m220-5 cells in various .media supplemented with 2.5% FBS, 10 μM forskolin, 100 ng/ml SCF, and 20 ng/mL bFGF, and passaged every 10 days [see the section of Fluorescence activated cell sorting]. The media used are as shown in Table 2. On the day of passage, 4.5 to $5.0 \times 10^3$ cells were plated on 24-well plates (0.5 mL of medium containing 10 μM Y27632) and medium free of Y27632 was added the next day. The medium exchange was performed as described above.

The defined hPGCLC expansion culture medium included the following : DMEM containing 15% KSR, 2.5% FBS, 1% NEAA, 2 mM GlutaMAX (GIBCO, 35050-061), 1% penicillin·streptomycin, and 0.1 mM 2-mercaptoethanol (GIBCO, 21985-023), and supplemented with 10 μM forskolin, 100 ng/mL SCF, and 20 mg/mL bFGF. Passage and medium exchange operations are as described above.

For a detailed step-by-step protocol of hiPSC culture, hPGCLC induction, xrOvary culture, and accompanying FACS analysis, see Yamashiro et al., Nat Protoc. 2020 Apr; 15(4) :1560-1583.

The step-by-step protocol for maintenance and expansion culture of hPGCLC is as follows.

1. Flat bottom cell culture plate (FALCON, 353043) is coated with 0.1% gelatin solution for 1 hr at room temperature.

2. MMC-treated (4 μg/mL, 2 hr) m220-5 feeders are seeded on gelatin-coated cell culture plates. Generally, $5.0 \times 10^5$ cells are seeded per well of a 12-well plate (containing 1 ml of DMEM containing 10% FBS, 1% penicillin·streptomycin, and 2 mM L-glutamine).

3. d6 hPGCLCs or expanded hPGCLCs (BT$^+$AG$^+$ cells or INTEGRINα6$^{high}$/EpCAM$^{high}$ cells) are collected by FACS sorting (see section above) in FACS buffer and centrifuged at 200 g for 7 min to form pellet sediment.

4. The supernatant is removed, and the cells are suspended in hPGCLC maintenance and expansion culture medium containing 10 μM Y27632 to a cell concentration of $1.0 \times 10^4$ cells/mL.

5. d6 hPGCLC or expanded hPGCLC is seeded on MMC-treated m220-5 feeders. Generally, 1 mL of cell suspension corresponding to $1.0 \times 10^4$ cells is seeded per well of a 12-well plate.

6. The next day, hPGCLC maintenance and expansion culture medium is added. Generally, 1 mL of medium is added per well of 12-well plate.

7. All media are exchanged with fresh media (2 mL) on day 3.

8. On days 5, 7 and 9, half of the medium is exchanged with fresh medium (1 mL).

9. On day 10, expanded hPGCLCs are passaged to a new culture plate.

Immunofluorescence (IF) analysis

Expanded PGCLCs were cultured in a film bottom dish (Matsunami Glass, FD10300) for several days and washed once with PBS(-). The cells were fixed with 4% paraformaldehyde for 15 min at room temperature and washed three times with PBS(-). For blocking and permeation treatment, fixed cells were incubated in a blocking buffer [10% normal donkey serum (Jackson ImmunoResearch, 017-000-121), 3% BSA (SIGMA, A3059), and 0.1% Triron-X 100 (Nacalai Tesque, 35501-02)]. The cells were then incubated in a $0.5 \times$ blocking buffer containing a primary antibody [1:1 mixture of blocking buffer and PBS(-)]. The conditions used for incubation with the primary antibody were as follows: As for mouse anti-BLIMP1, goat anti-SOX17, mouse anti-AP2y/TFAP2C, mouse anti-OCT4/POU5F1, goat anti-SOX2, and mouse anti-human mitochondria antibodies, the cells were incubated overnight at 4°C. As for mouse anti-UHRF1 and rabbit anti-DNMT1 antibodies, the cells were incubated for 30 min at room temperature. As for goat anti-NANOG antibody, the cells were incubated overnight at 4°C or 30 min at room temperature. After the primary antibody reaction, the cells were washed three times with PBS(-), and incubated for 1 hr at room temperature in $0.5 \times$ blocking buffer containing 1 pg/mL DAPI and secondary antibody. The cells were washed three times with PBS(-), and mounted in ibidi mounting agent (Mounting Media) (ibidi, 50001).

IF analysis of xrOvary was performed as previously reported (Yamashiro et al., 2018 (aforementioned)). To mount the stained sections, ibidi mounting agent without DAPI was used instead of VECTASHIELD mounting agent (Vector Laboratories, H-1000).

. The following antibodies were used in the indicated dilution solutions: mouse anti-BLIMPI (1/200; R&D SYSTEMS MAB36081); goat anti-SOX17 (1/100; R&D SYSTEMS MAB36081); mouse anti-AP2y/TFAP2C (1/100; Santa Cruz sc-12762); mouse anti-OCT4/POU5F1 (1/100; Santa Cruz sc-5279); goat anti-NANOG (1/100; R&D SYSTEMS AF1997); goat anti-SOX2 (1/100; SantaCruz sc-17320); mouse anti-UHRF1 (1:200; Milipore MABE308); rabbit anti-DNMT1 (1:200; abcam ab19905); mouse anti-human mitochondria (1:500; Millipore MAB1273); goat anti-FOXL2 (1/500; Novus Biological NB100-1277); mouse anti-DAZL (1/100; Santa Cruz sc-390929); goat anti-DDX4 (1/400; R&D SYSTEMS AF2030); rat anti-GFP (1/250; Nacalai Tesque 04404-84).

The following secondary antibodies from Invitrogen were used at 1/800 dilution: Alexa Flour488 donkey anti-rat IgG (A21208); AlexaFluor568 donkey anti-mouse IgG (A10037); Alexa Flour647 donkey anti-mouse IgG (A41571); Alexa Flour647 donkey anti-goat IgG (A21447); Alexa Flour647 donkey anti-rabbit IgG (A31573).

Images were acquired using the FV1000-IX81 confocal microscope system (Olympus) and processed with FV10-AV software. The processed images were trimmed using XnConvert software (https://xnview.com/en/xconvert/).

RNA extraction, reverse transcription, and cDNA amplification

For RNA extraction, the cells collected in Celltion were pelleted, lysed, and stored at -80°C until use. According to the manufacturer's instructions, RNA extraction was performed using the RNeasy Micro Kit (QIAGEN, 74004) or NucleoSpin RNA XS (MACHEREY-NAGEL, U0902A). Additional centrifugation was performed immediately before the elution stopped when using the NucleoSpin RNA XS. The concentration of RNA was measured using Qubit RNA HS assay kit (Invitrogen, Q32855). Using 1 ng of total RNA for reverse transcription from each sample, cDNA amplification was performed as previously reported (Nakamura et al., Nucleic Acids Res. 2015 May 19; 43(9):e60). When the RNA concentration was too low to measure (RNA extraction was performed with 1606 to 10968 cells in this study), the RNA solution was centrifuged to reduce its volume and the total concentrated RNA solution was used for reverse transcription and cDNA amplification.

PCR (qPCR) and RNA sequence (RNA-seq) analysis

The amplified cDNA described above was used for qPCR analysis. qPCR was performed with Power SYBR Green PCR Master Mix (Applied Biosystems, 4367659) on the CFX384 Touch Real-Time PCR Detection System (BIO-Rad Laboratories).

The primer sequences used are shown in Table 3.

[Table 3]

| target | forward primer sequence | reverse primer sequence |
|--------|------------------------|-------------------------|
| ERCC1806 | GATCCCGGAAGATACGCTCTAAG (1) | CGCAGGTTGATGCTTCCAATAAA (2) |
| ERCC451 | CAGGCAAGAGTTCAATCGCTTAG (3) | TAGCCTTCAGTGACTGTGAGATG (4) |
| ERCC56.4 | CCAACCCCACATTGTAACTTCG (5) | GTCTTTACTTACGCGCTCCTCT (6) |
| RPLP0 | GAAACTCTGCATTCTCGCTTCC (7) | ACTCGTTTGTACCCGTTGATGA (8) |
| PPIA | TTGATCATTTGGTGTGTTGGGC (9) | AAGACTGAGATGCACAAGTGGT (10) |
| POU5F1 | CTGTCTCCGTCACCACTCTG (11) | AAACCCTGGCACAAACTCCA (12) |
| NANOG | AGAGGTCTCGTATTTGCTGCAT (13) | AAACACTCGGTGAAATCAGGGT (14) |
| SOX2 | TGAATCAGTCTGCCGAGAATCC (15) | TCTCAAACTGTGCATAATGGAGT (16) |
| PRDM1 | AAACCAAAGCATCACGTTGACA (17) | GGATGGATGGTGAGAGAAGCAA (18) |
| TFAP2C | ATTAAGAGGATGCTGGGCTCTG (19) | CACTGTACTGCACACTCACCTT (20) |
| SOX17 | ACACGTCAGGATAGTTGCAGT (21) | TTCGTGTGCAAGCCTGAGAT (22) |
| DPPA3 | AAGCCCAAAGTCAGTGAGATGA (23) | GCTATAGCCCAACTACCTAATGC (24) |
| DAZL | TTTTTGTCTTTGTTGGAGTGAAGCA (25) | ACAGTATCAGCAATAGGCAGAAGCA (26) |
| DDX4 | ACTGATACAAATGGTGTTAACTGGGA (27) | AAACATGTCTAAGCCCCCTAAAGAA (28) |
| FRAME | CAAATGTTCAGTGTGAGTGAGGAAA (29) | TCTCCCCAAAGATCACATTAACTCC (30) |
| PIWIL2 | GTTTCATTTGGAGTGTGGGACATTA (31) | AACGCTTCCAAGGAAAATCAGAAAA (32) |
| (In the Table, numbers in parentheses indicate SEQ ID NO) | | |

For RNA sequence analysis, a previously reported method (Nakamura et al., 2015 (aforementioned); Ishikura et al., Cell Rep. 2016 Dec 6; 17(10):2789-2804) was modified slightly, and a sequence library was constructed using the amplified cDNA. AXyprep PCR amplification MAG PCR Clean-Up kit (Axygen, Mag-PCR-CL-250) was used instead of AMpure XP (Beckman Coulter, A63881) when purifying the cDNA library. Sequencing was performed on the ILLUMINA NextSeq 500/550 platform (ILLUMINA) with NextSeq 500/550 High output Kit v2.5 (75 cycles).

Whole-genome bisulfite sequencing (WGBS)

A WGBS library was prepared by the post-bisulfite adaptor tagging (PBAT) method (http://www.crestihec.jp/english/epigenome/index.html) (Miura et al., Nucleic Acids Res. 2012 Sep 1; 40(17):e136, Shirane et al., Dev Cell. 2016 Oct 10; 39(1):87-103). 10000-15000 cells were lysed in lysis buffer containing unmethylated λ phage DNA (Promega, D1521) [0.1% SDS and 1 pg/L proteinase K in water free of DNase.(GIBCO, 15230-160)] at 37°C for 60 min and incubated at 98°C for 15 min to inactivate Proteinase K. Assuming that a single cell has 6 pg of genomic DNA, unmethylated λ phage DNA corresponding to 0.5% of the added DNA was added. For bisulfite treatment, cell lysates were treated with EZ DNA Methylation-Gold kit (Zymogen D5005) according to the manufacturer's instructions. To construct a sequence library, the bisulfite-treated DNA was subjected to the synthesis of complementary DNA fragments tagged with a series of single-end adapters (http://www.crestihec.jp/english/epigenome/index.html). Phusion Hot Srart II DNA polymerase (Thermo Fisher Scientific, F549S) and AxyPrep MAG PCR Clean-UP Kit (Corning, Mag-PCR-CL-250) were respectively used instead of Phusion Hot Srart High-Fidelity DNA Polymerase (Thermo Fisher Scientific, F540S) and Agencourt AMPure XP. Sequencing was performed on the Illumina Hiseq 2500 plat form (Illumina) with TruSeq SR Cluster kit v3-cBot-HS and TruSeq SBS kit v3-HS.

Array comparative genomic hybridization (aCGH)

To extract genomic DNA, cells were collected in Cellotion and pelleted, and genomic DNA was extracted using NucleoSpin Tissue (MACHEREY-NAGEL, U0952Q) according to the manufacturer's instructions. Microarray CGH experiments and image processing were performed by Takara Bio using the Complete NA Labeling kit (Agilent Technologies, 5190-4240). Human male reference DNA (Agilent Technologies) was used for gender-matched reference DNA. Briefly, 200 ng of genomic DNA was digested with Alu1 and Rsa1 and then labeled with Cyanine 3 (reference genomic DNA) or Cyanine 5. Labeled genomic DNA was hybridized on a SurePrint G3 Human CGH Microarray 8×60K (Agilent Technologies, G4450A) at 200 rpm, 60°C for 24 hr. The obtained images were processed with Feature Extraction 12.1.0.3 (Agilent Technologies) (see CGH data analysis).

RNA-seq data processing and analysis

The human genome sequence and transcription product annotation GFE3 file of annotation release 107 (GRCh38.p2) were obtained from the NCBI ftp site (ftp://ftp.ncbi.nlm.nih.gov/). The reference gene annotation at the 3'-terminal extended to 10 kb, sufficiently covering the transcription termination site (TSS).

Processing of reads to expression levels was performed by a modified method of a previously described method (Nakamura et al, 2015 (aforementioned)). Briefly, all reads were processed with cutadapt v1.9.1 (Martin, EMBnetjournal. 2011 17:10), and low quality (quality score <30) sequence or adapter sequence was removed. Reads (>30mer) were mapped on the human genome GRCh38.3P2 by using Tophat (v2.1.0)/Bowtie (v2.2.7) with the "-no-coverage-search" option (Langmead & Salzberg, Nat Methods. 2012 Mar 4; 9(4):357-9; Kim et al, Genome Biol. 2013 Apr 25; 14(4):R36), and the forward reads per gene were evaluated by HISeq (v0.9.1; Anders et al, Bioinformatics. 2015 Jan 15; 31(2):166-9). The sum of all forward reads for all genes was used to convert read counts to loads per million mapped reads (RPM). The sum of all forward reads for all genes was used to convert read counts to loads per million mapped reads (RPM). Genes with $\log_2$ (RPM + 1)> ≥4 in at least one sample were used for unsupervised hierarchical clustering (UHC) and principal component analysis (PCA). UHC and PC were performed using R software version 3.6.1 (R CoreTeam, 2019). PCA was performed by the prcomp function. UHC was performed by the Dist function with method="euclidean" setting and the hclust function with method="ward.D2" setting. Gene ontology (GO) analysis was performed using DAVID6.8 (Huang et al., Nat Protoc. 2009; 4 (1) : 44-57).

WGBS data processing and analysis

The genome release GRCh38.p2 was used as the human genome and transcription product reference. The promoter region was defined as a region between 900 bp upstream and 400 bp downstream of the transcription start site (TSS). Strong, moderate, and weak CpG promoters (HCP, ICP, and LCP, respectively) were calculated as described in Borgel et al., Nat Genet. 2010 Dec; 42(12):1093-100. A list of CpG island (CpG) was obtained from Illingworth et al., PLoS Genet. 2010 Sep 23; 6(9):e1001134. CGI in the hg19 genome was converted to GTCh38 using the LiftOver program (https://genome.ucsc.edu/cgi-bin/hgLiftOver). Imprinted differentially methylated region (DMR) was obtained from Court et al et al., Genome Res. 2014 Apr; 24(4):554-69. Regions showing differential methylation in spermatozoon and oocytes (≥50% in spermatozoon or ≥50% in oocytes, plus 50% or more difference in spermatozoon and oocyte numbers are shown in drawings. RepeatMasker data for human GRCh38 used for genomic repeat element information was obtained from the UCSC table browser (https://genome.ucsc.edu/cgi-bin/hgTables).

For intergenic regions, 2 kb bins between genes excluding repeat elements were randomly selected. 200 Randomly selected regions are shown in the heat map. Processing of reads, mapping, and estimation of methylated C levels were performed as previously described (Shirane et al., 2016 (aforementioned)). Briefly, all reads were processed by the Trim_galore program (http://www.bioinformatics.babraham.ac.uk/projects/trim_galore/ ), and 4 bases from the 5'-terminal, 1 base from the 3'-terminal, an adapter sequence, and low quality reads (quality score<20) were removed. The processed reads were mapped on the human genome (GRCh38.p2) by the bismark program derived from Bismark (v0.17.0) with the "--pbat" option (Krueger & Andrews 2011). The mapped data (BAM format) were converted to methylation levels using the bismark_methylation_extractor program derived from Bismark. Using Samtool (v1.3) (Li et al., Bioinformatics. 2009 Aug 15; 25(16):2078-9) and IGV Tool (v2.3.52) (Robinson et al., Nat Biotechnol. 2011 Jan; 29(1):24-6), BAM files were manipulated and wig files were created.

Only CpGs with read depths between 4 and 200 were used in the calculation of methylated C (mC) %. Only regions with a CpG read (read depth ≥4) count of 4 or more within the genome-wide 2 kb bins and promoters, exons, introns, intergenic regions or imprinted DMRs were used for calculating averages or other analyses. For analyses relating to CpA, CpC, and CpT methylation, % methylation was calculated as mC % per kb as follows: the number of methylation calls over the genome-wide 1 kb bin was totaled and divided by the total number of unmethylated calls in the same region. Methylation levels per kb for CpA, CpC, and CpT were calculated separately across the whole genome.

Escapes from DNA demethylation ("escapees") in c30ag77 cells (the present invention), ag77_2 and ag120 AG⁺VT⁺ cells (Yamashiro et al., 2018 (aforementioned)), and pooled 7-9 week human germ cells (Tang et al., Cell. 2015 Jun 4; 161(6) :1453-67) were clarified using the hypermr program in the MethPipe v3.4.3 package (Song, et al., PLoS One. 2013 Dec 6; 8(12) :e81148) with default settings. Escapes common to these cells or specific to some cell types were calculated using the merge and intersect commands of bedtools v2.29.2 (Quinlan & Hall, Bioinformatics. 2010 Mar 15; 26(6) :841-2) . Annotations of escapes were performed using the annotate Peaks program in Homer v4.9.1. Data were visualized with R software using the 'upset' function in the 'UpSetR' library. Enrichment scores were calculated from the number of overlapping escapes at the indicated genomic element which exceeds the number of overlapping shuffled bins in the same category (average of 5 trials).

Data processing for hPGCLC-derived cells derived from xrOvaries (Yanmashiro et al., 2018 (aforementioned)), spermatozoon and oocyte (Okae et al., PLoS Genet. 2014 Dec 11; 10(12):e1004868) was performed as described above. The data for mPGCLC (Shirane et al., 2016 (aforementioned)) were mapped on the mouse genome mm10/GRCm38 and processed in the same manner as described above.

aCGH data analysis

Raw aCGH data were analyzed using CytoGenomics5.0.2.5 (Agilent Technologies) with the analysis method of "Default Analysis method - CGH v2" that detects abnormal intervals by algorithm ADM2. In addition, a "Fuzzy Zero" calibration was applied to reduce abnormally spaced false calls. A list of copy number variations in the human Aligent male reference DNA for hg19 is available from CytoGenomics 5.0.2.5.

Statistical analysis

All statistical tests were performed using R software version 3.6.1 (R CoreTeam, 2019). Welch's t-test was performed with the t-test function and using the "var. equal=F" option, and Wilcoxon signed-rank test was performed using the wilcox. exact function.

Data availability

Accession number of the data generated in the present invention:

RNA-Seq data: GSE147498 (GEO database);
WGBS data: GSE147499 (GEO database);
aCGH data: GSE148415 (GEO database)

Accession number of RNA-seq data:

hiPSCs/iMeLCs: GSE99350 (GEO database);
d6 hPGCLCs and ag7/21/35/49/63/77/120 cells: GSE117101;
WGBS data:

hiPSCs/iMeLCs/d6 hPGCLCs and ag35/77 cells: DRA006618 (DDBJ database);
ag120 cells: DRA007077 (DDBJ database)

Accession number of WGBS data of d4c3/d4c7 mPGCLCs:

DRA005166;
mESCs/EpiLCs/d2/d4/d6 mPGCLCs: DRA003471;
E10.5/E13.5/E16.5 germ cells: DRA000607;

<Experiment results>

Exploration of conditions for in vitro expansion of hPGCLC

To establish conditions for in vitro expansion of hPGCLC, it was investigated whether hPGCLC could proliferate under conditions similar to those for in vitro expansion of mPGCLC (Ohta et al., 2017 (aforementioned)). For this purpose, hiPSC having BLIMP1-tdTomato (BT) and TFAP2C-EGFP (AG) allele [585B1 BTAG hiPSC(XY)] (Sasaki et al., 2015 (aforementioned), Yokobayashi et al., 2017 (aforementioned)) were induced into iMeLCs by activin A (ActA) and a WNT signal transduction activator (CHIR99021), and thereafter into BTAG-positive (BT$^+$AG$^+$) hPGCLC by bone morphogenic protein 4 (BMP4), leukemia inhibitory factor (LIF), stem cell factor (SCF), and epidermal growth factor (EGF) (Fig. 1A). BT$^+$AG$^+$ hPGCLCs (d6 hPGCLCs) induced for 6 days were isolated by fluorescence-activated cell sorting (FACS), and $5.0 \times 10^3$ cells thereof were seeded on feeders of m220 subline in GMEM containing 15% knockout serum replacement (KSR), 2.5% bovine fetal serum (FBS), 100 ng/ml SCF, and various combinations of chemical substances [forskolin (10 $\mu$M), rolipram (10 $\mu$M), and cyclosporin A (5 uM)] and cytokines [LIF (10 ng/ml), EGF (50 ng/ml), and basic fibroblast growth factor (bFGF: 20 ng/ml)] known to have positive effects on mPGC (LC) expansion (32 combinations in total) (De Felici et al., Dev Biol. 1993 May; 157(1):277-80, Dolci et al., 1991 (aforementioned), Matsui et al., Nature. 1991 Oct 24; 353(6346):750-2, Ohta et al., 2017 (aforementioned)) (Fig. 1A, 1B).

After culturing for 10 days while exchanging the medium every 2 days, FACS analysis was performed to examine whether the number of d6 hPGCLCs was expanded under each condition. As for chemical substances, it was found that the addition of forskolin alone contributes to an expansion of the number of $BT^+AG^+$ cells more than the addition of either forskolin + rolipram or forskolin + cyclosporin A (cyclosporin A had a negative effect on $BT^+AG^+$ cell expansion) (Fig. 1B). As for cytokines, addition of LIF and EGF, FGF alone, or all three cytokines had a positive effect on the expansion of $BT^+AG^+$ cells (Fig. 1B). Therefore, addition of forskolin/LIF/EGF or forskolin/bFGF resulted in more than.4-fold expansion of $BT^+AG^+$ cells after 10 days of culture (Fig. 1B). It was decided to use forskolin/bFGF conditions in the following experiments because the strategy is more convenient.

Then, it was tested whether the basal composition affects the expansion of hPGCLC. First, the effect of four different basal media [GMEM and DMEMs with three different glucose concentrations (4.5 g/L, 1.0 g/L, 0.22 g/L)] was tested. $5.0 \times 10^3$ $BT^+AG^+$ d6 hPGGLC cells were seeded on m220 feeders in four media respectively supplemented with 15% KSR, 2.5% FBS, 100 ng/ml SCF, 10 μM forskolin, and 20 ng/ml bFGF. After culturing for 10 days, the number of $BT^+AG^+$ cells was evaluated by FACS, $5.0 \times 10^3$ $BT^+AG^+$ cells were passaged, and the analysis was repeated up to 30 culture days (c30) under each condition (Fig. 1C). The results of two independent experiments are shown in Fig. 1D. While the expansion rate varied slightly between experiments under each condition, DMEM containing 1 g/L glucose was most effective in expanding $BT^+AG^+$ cells during 30 days of culture (80-fold expansion at maximum). Thereafter, the effects of DMEM (1 g/L glucose), GMEM, and 7 different basal media (DMEM/F12, F12, Temin's MEM, αMEM, IMDM, RPMI and CMRL; Table 2) were similarly compared to find that DMEM (1 g/L glucose) had the most remarkable effect on the expansion of $BT^+AG^+$ cells, whereas basal media such as F12 and DMEM/F12 had a poor effect (Fig. 1E). The superior effect of DMEM (1 g/L glucose) on the expansion of $BT^+AG^+$ was also verified by other experiments comparing the effects of DMEM (1 g/L glucose), GMEM, RPMI, and CMRL (Fig. 1E).

Fig. 1F shows a representative culture of $BT^+AG^+$ cells (c21 to c30) on m220 feeders in DMEM (1 g/L glucose) containing selected supplements (15% KSR, 2.5% FBS, 100 ng/ml SCF, 10 μM forskolin, and 20 ng/ml bFGF). During culture, $BT^+$ cells characterized by oil droplet-like vesicles around the nuclei (AG fluorescence becomes relatively weak under an inverted fluorescence microscope) showed a slow but progressive expansion and formed colonies with distinct morphology after 10 days of culture (Fig. 1F). These findings indicate that 6d hPGCLC can proliferate in culture as $BT^+AG^+$ cells, and the mode of proliferation thereof as well as the requirements for efficient proliferation thereof appear to be somewhat different from those of mPGCLC (Ohta et al., 2017 (aforementioned)).

Sustained expansion of hPGCLC under defined conditions

The extent to which hPGCLC can be expanded as $BT^+AG^+$ cells under selected conditions [on m220 feeders in DMEM (1 g/L glucose) containing 15% KSR, 2.5% FBS, 100 ng/ml SCF, 10 μM forskolin, and 20 ng/ml bFGF] was examined. As starting cell populations, d4 and d6 hPGCLCs were used and cultured with passage of $BT^+AG^+$ cells every 10 days. As shown in Fig. 2A and Fig. 3A, both d4 and d6 hPGCLC cultures were successful and passaged to at least c120 as $BT^+AG^+$ cells. When d4 hPGCLC was used as the starting cell population, the percentage of $BT^+AG^+$ cells at passage (measured 10 days after each passage) was around ~20% at initial passage (~c20), -10% at later passages (-c30 to c120), and the percentage of non-$BT^+AG^+$ cells including $BT^+AG^-$ cells progressively increased during culture (~95% at maximum) (Fig. 3A, Fig. 3B). On the other hand, when d6 hPGCLC was used as the starting cell population, the percentage of $BT^+AG^+$ cells after passage was higher and around ~40% at early passage (~c20) and -20% at later passages (~c30 to c120) (Fig. 2A, Fig. 2B). Therefore, the percentage of non-$BT^+AG^+$ cells was lower (~80%) and substantially free of $BT^+AG^-$ or $BT^-AG^+$ cells during culture (Fig. 2A, Fig. 2B).

The composition of the cell population after passage of d6 hPGCLC expansion culture was examined more closely. In one experiment, $BT^+AG^+$ cells constituted about 46% of all cells at c20, whereas TRA-1-85$^+$ [human-specific antigen (Draper et al., J Anat. 2002 Mar; 200(Pt 3):249-58)J, non-$BT^+AG^+$ cells constituted about 13%, and TRA-1-85$^-$ cells (m220 feeders) constituted about 36% (dead cells: ~5%) (Fig. 2C). In c60, $BT^+AG^+$ cells accounted for about 37% of the total cell population, TRA-1-85$^+$, non-$BT^+AG^+$ cells constituted about 38%, and TRA-1-85$^-$ cells (m220 feeders) constituted about 20% (Fig. 2C). TRA-1-85$^-$ m220 feeders were plotted diagonally below $BT^+AG^+$ cells when sorted by BTAG fluorescence and showed weak autologous fluorescence (Fig. 3C). Combined with the analysis of changes in cell populations relating to BTAG expression during the entire culture period (Fig. 2A, Fig. 2B, Fig. 3A, Fig. 3B), these findings indicate that, under selected conditions, d6 hPGCLC can proliferate as $BT^+AG^+$ cells in a more stable manner than d4 hPGCLC, a certain fraction of $BT^+AG^+$ cells differentiate into non-$BT^+AG^+$ cells, particularly at later passages (see below), whereas the majority of d6 hPGCLC cultures remain as $BT^+AG^+$ cells, and had the ability to form characteristic colonies even at c120 (Fig. 2D). Taken together, these results indicate that $BT^+AG^+$ cells derived from d6 hPGCLC proliferate nearly ~$1.0 \times 10^6$-fold by c120 (Fig. 2E) in two repeat experiments, and the karyotype of the $BT^+AG^+$ cells during expansion was demonstrated to be substantially normal by karyotype analysis based on comparative genomic hybridization (CGH) at c70 (Fig. 3D).

In good agreement with positive BTAG expression, immunofluorescence (IF) analysis revealed that both colonies at c28 and c66 derived from d6 hPGCLC uniformly expressed BLIMP1 and TFAP2C (AP2γ), indicating that SOX17, POU5F1 (OCT4), and NANOG, which are key markers of hPGC(LC)s, were also clarified to be positive (Fig. 2F). The present inventors found that most AG⁻ cells in culture expressed SOX2 and that these cells generated in the vicinity of AG⁺/SOX2⁻ cells (Fig. 3E, see below). Total RNA of BT⁺AG⁺ cells derived from c10 to c120 (c10, c30, c50, c70, c90, and c120) was isolated and analyzed for the expression of key genes by quantitative PCR (qPCR). As a result, BT⁺AG⁺ cells continuously expressed genes such as BLIMP1 (PRDM1), TFAP2C, SOX17, POU5F1, and NANOG at similar levels at least up to c120, while SOX2 expression was stably suppressed (Fig. 2G). These findings indicate that hPGCLC, particularly d6 hPGCLC, can be proliferated $\sim 1.0 \times 10^6$-fold or more under the defined conditions while maintaining the properties of hPGC.

Expansion of hPGCLC using surface marker selection

It was tested whether hPGCLC can be proliferated with a passage operation that can be easily applied to other hPSC lines. Based on the observation that no BT⁻AG⁺ cells appeared under the present inventors' culture conditions when d6 hPGCLC was used as the starting cell population (Fig. 2A, Fig. 2B), it was first tested whether hPGCLC could be proliferated by passaging using an AG reporter alone. It was found that 585B1 BTAG hiPSC-derived d6 hPGCLC can be proliferated as AG⁺ cells by passage up to c60 or higher (Fig. 4A, Fig. 4B), and the cell population resulting in c60 was essentially the same as that passaged using BTAG positivity (Fig. 4C).

Then, it was tested whether hPGCLC can be proliferated with passage using a cell surface marker. An independent hiPSC line 1383D6 (XY) (Yokobayashi et al., 2017), which does not have a fluorescent reporter, was induced into hPGCLC, and d6 hPGCLC expressing INTEGRINα6 and EpCAM was isolated (Sasaki et al., 2015 (aforementioned), Yokobayashi et al., 2017 (aforementioned)) (Fig. 4D), and cultured under the conditions selected by the present inventors. At c10, formation of colonies with a characteristic morphology was observed (Fig. 4D, Fig. 4E), and FACS analysis revealed that a clear population of cells expressing high levels of INTEGRINα6 and EpCAM was enriched in culture, and INTEGRINα6^low/EpCAM^high cell population (see below for property analysis thereof) was simultaneously developed (Fig. 4D). BT⁺AG⁺ cells at c40 derived from 585B1 BTAG hiPSC showed INTEGRINα6 and EpCAM expression profiles similar to those of INTEGRINα6^high/EpCAM^high cell population derived from 1383D6 hiPSC, whereas INTEGRINα6^low/EpCAM^high cell population corresponding to BT⁺AG⁺ cells and INTEGRINα6⁻/EpCAM⁻ cell population were mainly composed of m220 feeders (Fig. 4F, Fig. 3C). Therefore, when INTEGRINα6^high/EpCAM^high cell population was passaged, such cells were successfully proliferated and passaged to at least c30 and expanded to ~50-fold at maximum (Fig. 4D, Fig. 4E, Fig. 4G). Furthermore, qPCR analysis showed that INTEGRINα6^high/EpCAM^high cells expressed key hPGC genes such as BLIMP1 (PRDM1), TFAP2C, SOX17, POU5F1, and NANOG, and suppressed SOX2 in expansion culture in a manner similar to BT⁺AG⁺ cells (Fig. 4H). The somewhat lower proliferation efficiency (~50-fold vs ~100- to 200-fold at c30) of INTEGRINα6^high/EpCAM^high cells compared with the proliferation efficiency of BT⁺AG⁺ cells derived from 1383D6 line derived from 585B1 BTAG line may be related to damage to the starting cell population resulting from a relatively complicated sorting procedure for surface markers, or may be due to difference in the clone of the original hiPSC line. It was concluded that hPGCLC can be successfully proliferated by passaging using a cell surface marker.

Expanded hPGCLC retains early hPGC(LC) transcriptome

To further investigate the properties of hPGCLC-derived cells in expansion culture, transcriptome of relevant cell type was measured by an RNA sequencing method (Nakamura et al., 2015 (aforementioned)), and the properties thereof were analyzed by comparison with the transcriptome of hPGCLC-derived cells in xrOvaries (Yamashiro et al., 2018 (aforementioned)). The cell types analyzed were as follows: hiPSC and iMeLC (585B1 BTAG, 1383D6), BT⁺AG⁺ cells (585B1 BTAG) at c10, c30, c50, c70, c90, and c120, INTEGRINα6^high/EpCAM^high cells at c10 and c30, INTEGRINα6^low/EpCAM^high cells (1383D6) at c10, BT⁺AG⁺ cells (585B1 BTAG) at aggregation days (ag) 7, 21, 35, 49, 63, and 77 (Yamashiro et al., 2018 (aforementioned)), and AG⁺VT⁻/AG⁺/⁻VT⁺/AG⁺VT⁺/AG⁻VT⁺ cells [1390G3 AGVT (DDX4 (also known as human Vasa homolog)-tdTomato)] at ag120 (Fig. 5A).

Unsupervised hierarchical clustering clarified that the analyzed cells were mainly divided into two large clusters; one consisting of hiPSC/iMeLC and the other consisting of d6 hPGCLC-derived cells (Fig. 7 A). In large clusters of d6 hPGCLC-derived cells, BT⁺AG⁺ cells and INTEGRIN6^high/EpCAM^high cells in expansion culture were mixed with those with different culture periods to form robust clusters, and all clusters of such cells showed similarity not only to clusters of hPGCLC-derived cells (BT⁺AG⁺ cells at ag7) in xrOvaries early in the culture period (Yamashiro et al., 2018 (aforementioned)) but also to clusters of d6 hPGCLCs (Fig. 7A).

On the other hand, the clusters of BT⁺AG⁺/INTEGRINα6^high/EpCAM^high cells in expansion culture showed relatively distant relationship with the clusters of hPGCLC-derived cells [including oogonia/protogerm-like cells (cells at ag77 and ag120)] in later culture periods (BT⁺AG⁺ cells at ag21, ag35, ag49, ag63, and ag77, and AG⁺VT⁻/AG⁺/⁻VT⁺/AG⁺VT⁺/AG⁻VT⁺ cells at ag120) in xrOvaries (Fig. 7 A) (Yamashiro et al., 2018 (aforementioned)). Interestingly, INTEGRIN6^low/EpCAM^high cells at c10 were found to cluster with hiPSC/iMeLC and express pluripotency-related genes and other key genes in a manner similar to hiPSC/iMeLC (Fig. 7 A, Fig. 5 B), suggesting that they represent a dedifferentiated cell population. In good agreement with these results, principal component analysis (PCA) showed that BT⁺AG⁺/INTEGRINα6^high/EpCAM^high cells in expansion culture are plotted close to the position of BT⁺AG⁺ cells at ag7, which is in between the positions of d6 hPGCLC and BT⁺AG⁺ cells at ag21, regardless of their culture periods (Fig. 7B).

Next, the expression of a set of genes that characterize the developmental progression from hPGC(LC) to oogonium/gonocyte was examined (already defined in BT⁺AG⁺/INTEGRINα6^high/EpCAM^high cells during expansion culture by the present inventors (Yamashiro et al., 2018 (aforementioned))). As already reported (Yamashiro et al., 2018 (aforementioned)) and also shown in Fig. 7C, the genes of clusters 1 and 2 are upregulated by hPGCLC fate determination, cluster 1 genes (e.g., SOX17, PRDM1, NANOS3, KLF4, TCLIA) are continuously expressed thereafter, cluster 2 genes (e.g., HLA-DQB1, HLA-DPA1, MT2A, TRPC5, TRPC6) are gradually suppressed after about ag35, cluster 3 genes are specifically upregulated after about ag35, which characterizes the development of oogonium/gonocyte (e.g., DAZL, DDX4, MAEL, TDRD9, PIWIL1), the genes of clusters 4 and 5 are downregulated after hPGCLC fate determination, cluster 4 genes (e.g., SOX2, TDGF1, GJA1, OTX2, CDH1) are suppressed relatively quickly, and cluster 5 genes (e.g., IFITM2, IFITM3, SLC2A3, SLC2A1) are gradually suppressed. As is clear from Fig. 7C and Fig. 7D, BT⁺AG⁺/INTEGRINα6^high/EpCAM^high cells in expansion culture continue to express the genes of clusters 1, 2, and 5, suppress cluster 4 gene like d6 hPGCLC/BT⁺AG⁺ cells at ag7, do not upregulate cluster 3 gene except genes such as BRDT, IRX4, and IL12B, and show very similar properties to those of BT⁺AG⁺ cells at ag7. In agreement therewith, Spearman's rank correlation analysis using this gene set showed that the transcriptomes of BT⁺AG⁺/INTEGRINα6^high/Ep-CAM^high cells in expansion cultures at various culture periods are highly correlated, and showed the closest correlation with the transcriptome of BT⁺AG⁺ cells at ag7 and then a correlation with the transcriptome at ag21 (Fig. 7E).

Genes expressed differentially [$\log_2$ (RPM+1)$\geq$4, $\log_2$ (fold change)$\geq$1] among BT⁺AG⁺ cells were measured at various culture periods during expansion culture. The number of differentially expressed genes was highest between d6 hPGCLC and c10 BT⁺AG⁺ cells (377 and 548 genes were up/down-regulated in c10 BT⁺AG⁺ cells, respectively), the number substantially decreased between c10 and c30 BT⁺AG⁺ cells (72 and 56 genes were up/down-regulated in c30 BT⁺AG⁺ cells, respectively), BT⁺AG⁺ cells showed almost identical transcriptome after c30, and a few exceptions include TCL1A which is progressively downregulated during expansion culture (see Fig. 8A, Fig. 5B, Fig. 6, and below). Since TCL1A is known to act as a co-activator of AKT and promote glycolysis in PSC (Laine et al., Mol Cell. 2000 Aug; 6(2):395-407, Nishimura et al., Stem Cell Reports. 2017 Mar 14; 8(3):787-801), it is suggested that AKT and the glycolytic pathway are progressively downregulated during amplification culture. It is known that genes upregulated in c10 BT⁺AG⁺ cells as compared with d6 hPGCLC are enriched in Gene Ontology (GO) functional terms such as "negative regulation of growth" and "mineral uptake", contain metallothionein (MT) 1E, 1F, 1G, 1H, 1X, and 2A, as well as superoxide dismutases (SOD) 1 and 2, which function for homeostasis of essential heavy metals such as zinc, or for removal of reactive oxygen species (ROS) (Fig. 8B) (Haq et al., Mutat Res. 2003 Dec 10; 533 (1-2) :211-26). The MT gene is also upregulated in BT⁺AG⁺ cells at ag7 in xrOvaries and subsequently downregulated (Fig. 5B). In contrast, c10 BT⁺AG⁺ cells are enriched in GO terms such as "cardiogenic" (e.g., GATA2, GATA3, HANDI1, ID1, ID3, SALL1), "nephrogenic" (e.g. NPHP3, LZTS2, LHX1, ARID5B, OVOL1), and "extracellular matrix organization" (e.g., COL2A1, COL3A1, COL4A5, LAMA4, LAMB2) (Fig. 8B), and it is suggested that genes associated with somatic programs and simultaneously activated by hPGCLC fate determination are suppressed during hPGCLC expansion culture.

To gain insight into the potential mechanism that distinguishes the developmental pathway of BT⁺AG⁺ cells in expansion culture from that in xrOvaries, genes differentially expressed at [$\log_2$(RPM+1)$\geq$4, fold change $\geq$3] were searched between c30 BT⁺AG⁺ cells and ag7/ag21 BT⁺AG⁺ cells (Fig. 8C). The genes upregulated in c30 BT⁺AG⁺ cells (221 genes) as compared with ag7 BT⁺AG⁺ cells include CENPN/C/K, CETN3, CDC7/26, CCNA2, and E2F3, and GO terms such as "CENP-A-containing nucleosome assembly" and the GO terms such as "mitotic cell division" were enriched (Fig. 8C), and this is consistent with the idea that mitosis is more active in BT⁺AG⁺ cells during expansion culture as compared with BT⁺AG⁺ cells in xrOvaries. The genes (162 genes) upregulated in c30 BT⁺AG⁺ cells as compared with ag21 BT⁺AG⁺ cells further include members of the MT gene family, and reflect downregulation thereof in ag21 BT⁺AG⁺ cells (Fig. 8C). In contrast, compared with c30 BT⁺AG⁺ cells, genes upregulated in ag7/21 BT⁺AG⁺ cells (101 and 70 genes, respectively) included FOS, FOSB, EGR1, EGR2, and EGR3 (Fig. 8C). These are immediate early genes that respond to various cellular stimuli, and suggest that the xrOvary environment triggers separate signal transduction pathway in BT⁺AG⁺ cells. Taken together, these findings indicate that, in d6 hPGCLC-derived BT⁺AG⁺/INTEGRINα6^high/EpCAM^high cells, upregulation of some genes during culture may indicate adaptability to culture, but in expansion culture, they take a developmental pathway toward oogonium-/gonocyte-like cells, discontinue the developmental progress thereof in early stages and faithfully maintain the properties of early hPGCLC.

Expanded hPGCLC retain early hPGC(LC) DNA methylome

Next, the genome-wide DNA methylation profile of BT⁺AG⁺ cells at c10, c70, and c120 in expansion culture was measured by whole-genome bisulfite sequence (WGBS) analysis (Miura et al., 2012 (aforementioned), Shirane et al., 2016 (aforementioned)), and the properties thereof were tested by comparison with the properties of other related cell types including hiPSC, iMeLC, d6 hPGCLC, and hPGCLC-derived cells in xrOvaries (BT⁺AG⁺ cells at ag35 and ag77, and AG⁺VT⁻/AG⁺VT⁺/AG⁻ VT⁺ cells at ag120) (Yamashiro et al., 2018 (aforementioned)) (Fig. 10A). hiPSC and iMeLC displayed highly similar genome-wide 5-methylcytosine (5mC) profiles, with an average 5mC of -80%, and d6 hPGCLC showed a slight but significant decrease in overall 5mC level (-75% on average) (Yamashiro et al., 2018 (aforementioned)) (Fig. 9A, Fig. 9B). In expansion culture, BT⁺AG⁺ cells showed a further decrease of 5mC from d6 hPGCLC and acquired an-average 5mC level of ~65% at c10 (Fig. 9A, Fig. 9B, Fig. 10A). However, subsequently at c70 and c120, their genome-wide 5mC levels and profiles were substantially maintained, with a slight increase (~68%) at c120 (Fig. 9A, 9B, Fig. 10A). Therefore, it was clarified by the test of 5mC levels in representative elements across the genome, including promoters, exons, introns, intergenic regions, representative repetitive elements, non-promoter CpG islands (CGIs), and imprinted control regions (ICRs) that all of these elements essentially retained the 5mC levels thereof during the expansion culture (Figs. 9C to 9E). These observations are in good agreement with the finding that BT⁺AG⁺ cells substantially retain the transcriptome of early hPGC during expansion culture thereof (Fig. 7), whereas d6 hPGCLC differentiated into oogonium/gonocyte and underwent genome-wide DNA demethylation in xrOvaries (Yamashiro et al., 2018 (aforementioned)) (Fig. 9A), which is in sharp contrast to the finding that mPGCLC undergoes genome-wide DNA demethylation during expansion culture thereof (Ohta et al., 2017 (aforementioned), Shirane et al., 2016 (aforementioned)). Interestingly, the locus of the MT1 gene cluster that showed characteristic upregulation in c10 BT⁺AG⁺ cells as compared with d6 hPGCLC (Figs. 8A, 8B, 5B) exhibited unique low methylation state over the process of hPGCLC induction/differentiation/expansion (Fig. 10B). Therefore, the upregulation of MT gene during maintenance and expansion culture was not the result of demethylation of the promoters thereof. In contrast, among the few genes that showed progressive suppression during expansion culture of BT⁺AG⁺ cells, the promoter of TCL1A restored methylation during culture (Fig. 8A, Fig. 8B, Fig. 10C). Moreover, the ICRs of IG-DMR, H19, and IGF1R were slightly demethylated during maintenance and expansion culture of BT⁺AG⁺ cells (Fig. 9E), which did not appear to affect the expression of the imprinted genes under the control thereof, except for the H19 gene (Fig. 10D).

To investigate the mechanism of regulation of the whole DNA methylation profile in BT⁺AG⁺ cells during expansion culture, the expression dynamics of genes associated with DNA methylation/demethylation in those cells was first examined. Among *de novo* DNA methyltransferases (DNMTs), DNMT3A showed low expression levels [$\log_2(RPM+1) \cong 5$, i.e., ~20 copies/cell (Nakamura et al., 2015)] not only in BT⁺AG⁺ cells during expansion culture, but also in hiPSC, iMeLC, and BT⁺AG⁺ cells in xrOvaries, whereas DNMT3B showed rapid decrease upon hPGCLC fate determination, indicating lower expression level [$\log_2(RPM+1) \cong 5$] in BT⁺AG⁺ cells during expansion culture and in xrOvaries (Fig. 5B). DMNT3L did not show remarkable expression in any of the cells tested (Fig. 5B). Among the genes for maintaining DNA methylation, DNMT1 was expressed at remarkable levels [$\log_2(RPM+1) \cong 8$] in all analyzed cell types, whereas UHRF1 which encodes a key protein for recruiting DNMT1 to replication foci (Bostick et al., Science. 2007 Sep 21; 317(5845):1760-4., Sharif et al., Nature. 2007 Dec 6; 450(7171):908-12) was progressively downregulated in BT⁺AG⁺ cells in xrOvaries (Fig. 5B) (Yamashiro et al., 2018 (aforementioned)), but continued to be expressed in BT⁺AG⁺ cells at the level of d6 hPGCLC/BT⁺AG⁺ cells at ag7 [$\log_2(RPM+1) \cong 5$] over the period of expansion culture (Fig. 5B). Among the genes for DNA demethylation, TET1 was expressed at a substantial level, whereas TET2 and TET3 showed only a very low level of expression in all analyzed cell types (Fig. 5B).. These findings suggest that the maintenance of UHRF1 expression level is a key property that distinguishes BT⁺AG⁺ cells in expansion culture from BT⁺AG⁺ cells in xrOvaries, and may be the basis for the maintenance of the whole 5mC level in the BT⁺AG⁺ cells in expansion culture. To further investigate this point, the expression of DNMT1 and UHRF1 proteins in BT⁺AG⁺ cells in maintenance and expansion culture was analyzed by IF analysis. As shown in Fig. 9F, hiPSC strongly and relatively uniformly expressed both DNMT1 and UHRF1 in the nucleus. In some nuclei, DNMT1 shows clear punctate localizations, corresponding to replication foci (Fig. 9F) (Leonhardt., J Cell Biol. 2000 Apr 17; 149(2):271-80., O'Keefe., J Cell Biol. 1992 Mar; 116(5) :1095-110). On the other hand, BT⁺AG⁺ cells in c66 strongly expressed DNMT1, but its punctate nuclear localizations were not detected. This is probably due to the slower proliferation dynamics of BT⁺AG⁺ cells as compared with hiPSC. BT⁺AG⁺ cells expressed UHRF1 somewhat weakly and non-uniformly. That is, about half of them expressed UHRF1 in the nucleus, but others expressed UHRF1 only a little (Fig. 9F). UHRF1 was not detected in the cytoplasm either in hiPSC or in BT⁺AG⁺ cells at c66 (Fig. 9F). From these findings, it was concluded that BT⁺AG⁺ cells in maintenance and expansion culture expressed UHRF1, even though at low levels and somewhat non-uniformly.

Next, the dynamics of methylation levels of non-CpG sites (CpH: CpA, CpT, and CpC) not only during BT⁺AG⁺ cell expansion culture, but also in other relevant environments, including mPGCLC induction and expansion were analyzed. This is because such methylation levels serve as an appropriate index for *de novo* DNMT activity in a given cell (Ramsahoye et al., Proc Natl Acad Sci U S A. 2000 May 9; 97(10):5237-42; Liao et al., Nat Genet. 2015 May;

47(5) :469-78.). Consistent with previous findings (Seisenberger et al., Mol Cell. 2012 Dec 28; 48(6):849-62; Kobayashi et al., Genome Res. 2013 Apr; 23(4):616-27.; Kubo et al., BMC Genomics. 2015 Aug 20; 16(1):624.), mouse germ cells at embryonic days (E)10.5 and E13.5 during the deletion of genome-wide CpG methylation show low CpH methylation levels (<1% on average), after which non-female, male germ cells acquire high CpH methylation level particularly for methyl CpA (mCpA) at E16.5 (>2.5% on average) (accompanied by the acquisition of genome-wide androgen CpG methylation profile (Kobayashi et al., 2013 (aforementioned), Kubo et al., 2015 (aforementioned), Seisenberger et al., 2012 (aforementioned))), as clarified by reanalysis (Fig. 9G). In the mPGCLC induction/expansion system, mESC is cultured under the "ground state" conditions in which $de\ novo$ DNMT activity is known to be suppressed (Habibi et al., Cellstem Cell. 2013 Sep 5; 13(3):360-9., Yamaji et al., Cellstem Cell. 2013 Mar 7; 12(3):368-82, Shirane et al., 2016 (aforementioned)), shows low CpG methylation level (<1% on average), whereas EpiLC that upregulates de $novo$ DNMT activity (Shirane et al., 2016 (aforementioned), Yamaji, et al., 2013 (aforementioned)) substantially increased mCpA (~2% on average) (Fig. 9H). The mCpA level in mPGCLC, in which $de\ novo$ DNMT expression is strongly suppressed (Hayashi et al., 2011 (aforementioned), Ohta et al., 2017 (aforementioned), Shirane et al., 2016 (aforementioned))) decreased rapidly to a basal level (<1% on average) (Fig. 9H). These findings demonstrate that mCpH is suitable as an index for $de\ novo$ DNMT activity. hiPSC, which is known to have gene expression property homologous to EpiLC (Nakamura et al., 2016 (aforementioned)), exhibited high mCpA levels (~2% on average), slightly decreased in iMeLC (-1.5% on average), and further decreased to a basal level (<1% on average) in d6 hPGCLC, BT⁺AG⁺ cells in expansion culture, and BT⁺AG⁺ cells in xrOvaries (Fig. 9I). Taken together, these findings support the theory that BT⁺AG⁺ cells in expansion culture show a decrease in $de\ novo$ DNMT activity but retain maintenance DNMT activity, which in turn leads to the preservation of genome-wide 5mC profile of early hPGC(LC) of BT⁺AG⁺ cells in expansion culture.

Expanded hPGCLC undergo epigenetic reprogramming to differentiate into oogonium/gonocyte in xrOvaries

Next, it was evaluated whether the BT⁺AG⁺ cells in expansion culture have a differentiation capacity in the manner of human germ cell. To this end, 585B1 BTAG hiPSCs were induced into hPGCLCs, d6 hPGCLCs were cultured for 30 days, and xrOvary was produced using c30 BT⁺AG⁺ cells (5,000 cells) and E12.5 mouse embryonic ovarian somatic cells (75,000 cells) (Yamashiro et al., 2018 (aforementioned)) (Fig. 11A). xrOvary with C30 BT⁺AG⁺ cells developed in a manner similar to xrOvaries with d6 hPGCLC (Fig. 11B). FACS analysis clarified that the number of surviving BT⁺AG⁺ cells varied among xr ovaries [per xrOvary: ag35: ~149 to 984; c30ag35: ~189 to 2267; ag77: ~0 to 4942; c30ag77: ~15 to 7322], but C30 BT⁺AG⁺ cells showed a better survival/proliferation tendency as BT⁺AG⁺ cells than d6 hPGCLCs at both ag35 and ag77 in xrOvaries (Fig. 11C). As a result, IF analysis at ag77 showed that AG⁺ cells derived from d6 hPGCLC and AG⁺ cells derived from c30 BT⁺AG⁺ cells were characterized by only faint DAPI staining, were positive for SOX17, TFAP2C, and human mitochondria antigen, and delineated by FOXL2⁺ mouse granulosa cell (Fig. 11D). Importantly, similar to AG⁺ cells derived from d6 hPGCLC, many of the AG⁺ cells derived from such c30 BT⁺AG⁺ cells were found to be positive for DAZL and DDX4, which are key markers of oogonium/gonocyte (Fig. 11D) (Gkountela et al., Cell. 2015 Jun 4; 161(6):1425-36.; Guo et al., Cell. 2015 Jun 4; 161(6):1437-52; Li et al., Cellstem Cell. 2017 Jun 1; 20(6):858-873.e4; Tang et al., 2015 (aforementioned); Yamashiro et al., 2018 (aforementioned)).

Gene expression in BT⁺AG⁺ cells derived from c30 BT⁺AG⁺ cells was measured at ag7, ag35, and ag77 (c30ag7, c30ag35 and c30ag77 cells) by qPCR (c30ag7/c30ag35/c30ag77) or RNA-seq analysis (c30ag35/c30ag77). As shown in Fig. 11E, such cells continued to express markers of hPGC, including PRDM1, TFAP2C, SOX17, POU5F1, and NANOG, where, like 6hPGCLC-derived cells, DNMT1 was maintained while UHRF1 was progressively suppressed. Interestingly, however, compared with d6hPGCLC-derived cells, they upregulated earlier the genes relating to oogonium/gonocyte, including DPPA3, PRAME, PIWIL2, DAZL, and DDX4. They also upregulated these genes at ag7, and d6 hPGCLC-derived cells caught up with c30 BT⁺AG⁺ cell-derived cells by ag77 as regards the expression levels of these genes (Fig. 11E). What is to be noted is that, as compared with d6hPGCLC, BT⁺AG⁺ cells in proliferation culture reduced 5mC levels of promoters for DPPA3, PIWIL2, and PRAME to a substantial extent (DPPA3; ~52%, PRAME; ~19%, PIWIL2; ~16%), and reduced by genome-wide averages (~10%) for DAZL and DDX4 (Fig. 11F). In fact, promoters of cluster 3 genes (they were methylated the high levels in d6hPGCLC) showed substantial demethylation during the first 10 days of expansion culture (Fig. 11G, Fig. 12A).

Unsupervised hierarchical clustering of transcriptome of related cell type was different from that of BT⁺AG⁺ cells in expansion culture, and both c30ag35 and c30ag77 cells were classified in clusters of oogonium-/gonocyte-like cells, c30ag35 and c30ag77 cells were respectively classified as early and late oogonium-/gonocyte-like cells (Fig. 12B), and the PCA and expression profile of a set of genes that characterize the developmental progression from hPGC(LC) to oogonium/gonocyte provided consistent results (Fig. 11H). Consistent with qPCR analysis (Fig. 11E), compared with ag35 and ag77 cells derived from d6 hPGCLC (ag35 and ag77 cells), c30ag35 and c30ag77 cells showed more progressed properties; for example, they downregulated genes in cluster 2 and upregulated genes in cluster 3 in more, remarkable manners (Fig. 11I, Fig. 11J). These findings are consistent with the view that, as compared with d6hPGCLC, BT⁺AG⁺ cells in expansion culture have developmentally more advanced properties that they more rapidly differentiate into oogonium-/gonocyte-like cells in response to the signal/environment provided by the xrOvaries.

Whole-genome bisulfite sequence analysis of c30ag77 cells revealed that they undergo substantial genome-wide DNA methylation and reach a genome-wide 5mC level of -15% (Fig. 11K), which is a value equivalent to that of human oogonium/gonocyte (Guo et al., 2015 (aforementioned), Tang et al., 2015 (aforementioned)) and ag120 cells (Yamashiro et al., 2018 (aforementioned)) at 7 to 10 weeks of development. Thus, c30ag77 cells deleted 5mC from the whole genome, including promoters, exons, introns, intergenic regions, representative repetitive elements, non-promoter CGI and ICR (Fig. 11L, Fig. 11M, Fig. 12C) and acquired an epigenetic "naive" state of human germline. We analyzed the "escape" that avoids DNA demethylation (5mC> 20%) in ag77 cells, c30ag77 cells, and ag120AG+VT+ cells as compared with that in human germ cells in vivo (Tang et al., 2015 (aforementioned)), and found that the majority of the escapes overlapped among these cells, and the numbers thereof correlated with residual 5mC levels in these cell types (Fig. 12D). This indicates that the cells derived from d6hPGCLC/c30BT+AG+ cells of xrOvaries eliminate DNA methylation of the whole genome in a manner similar to that in human germ cells in vivo. Therefore, escape was thus concentrated around repetitive elements such as SVA, ERVK, and ERV1, but depleted around other major genomic elements (Fig. 12D). Taken together, these findings clarify that BT+AG+ cells in expansion culture have sufficient capacity to differentiate as human germ cells, and thus the culture system developed in the present invention can be used as *bona fide* hPGC to proliferate hPGCLC in vitro.

Example 2 Establishment of culture system evaluation method

Further detailed analysis of the FACS analysis results of 585B1-868BTAG (BLIMP1-tdTomato and AP2y-EGFP) iPS cell-derived proliferated human PGCLC was performed. The results are shown in Fig. 13. In the FACS analysis, the expression of the human cell-specific cell surface antigen TRA-1-85 in the population excluding dead cells by DRAQ7 staining was analyzed. The TRA-1-85-positive cell population was defined as a BTAG double-positive population and other population (hereinafter referred to as TRA-1-85-positive and BTAG-negative cell population) from the BTAG expression patterns. Then, it was clarified that the TRA-1-85-negative cell population and the TRA-1-85-positive BTAG-negative cell population showed mutually exclusive side scatter (SSC) and forward scatter (FSC) patterns. Regardless of TRA-1-85 expression, when all BTAG-negative cell populations were plotted for SSC and FSC, those with relatively small SSC values and broad FSC values correspond to TRA-1-85 positive cells. From this, BTAG-negative cells with relatively small SSC value and wide FSC value were considered to be dedifferentiated cells derived from human PGCLC. The logarithmically transformed value of the ratio of the number of proliferated PGCLC cells to the number of dedifferentiated cells was defined as the enrichment score, and used as an index for evaluating the state of the culture system (formula 1). If the enrichment score takes a positive value, it indicates that the cell number of proliferated PGCLC is dominant.

$$\text{Enrichment score} = \log_2 \text{(hPGC marker-positive cell number/dedifferentiated cell number)} \quad \text{(formula 1)}$$

wherein expanded PGCLCs are PGCLC marker-positive cells (e.g. BT(+)AG(+) cells) in the proliferation culture system, and dedifferentiated cells are PGCLC marker-negative cells with relatively small SSC values and a wide range of FSC values.

Example 3 Suppression of dedifferentiation of PGCLC by inhibition of WNT signal transduction pathway

Proliferation culture was performed using 585B1-868BTAG iPSC-derived PGCLCs on day 6 of differentiation induction. At that time, culture was performed for 30 days under the conditions including addition of each concentration of IWR1, 2.5 $\mu$M of IWR1, XAV939, Wnt-C59, and IWP2, or the solvent DMSO, in addition to the conditions adopted in Example 1. Cells were passaged and counted by FACS every 10 days to draw a proliferation curve. In addition, an enrichment score was calculated at respective culture day numbers. The results are shown in Fig. 14A to Fig. 14C. IWR1 had no effect on hPGCLC proliferation (Fig. 14A) and significantly suppressed the appearance of dedifferentiated cells (Fig. 14B). XAV939 also showed a similar tendency as IWR1 (Fig. 14C).

While the present invention has been described with emphasis on preferred embodiments, it is obvious to those skilled in the art that the preferred embodiments can be modified.

The contents disclosed in any publication including specifications of patents and patent applications are hereby incorporated in their entireties by reference, to the extent that they have been disclosed herein.

[Industrial Applicability]

According to the method of the present invention, it is possible to culture hPGCLC for 120 days or longer and expand the hPGC one million times or more. Therefore, various experiments that were difficult to apply due to the problem of cell number become possible, and it is possible to verify humoral factors/genes such as cytokines that are important for further differentiation of the human germ line and functions thereof.

**Claims**

1. A method for maintaining and expanding a human primordial germ cell (hPGC) or a human primordial germ cell-like cell (hPGCLC) derived from a human pluripotent stem cell, comprising culturing hPGC or hPGCLC in the presence of (i) a forskolin or phosphodiesterase 4 (PDE4) inhibitor, and (ii) one or more cytokines selected from the group consisting of a basic fibroblast growth factor (bFGF), a leukemia inhibitory factor (LIF), and an epidermal growth factor (EGF).

2. The method according to claim 1, wherein the inhibitor of (i) is forskolin.

3. The method according to claim 1 or 2, wherein the cytokine is a combination of LIF and EGF, bFGF alone, or a combination of LIF, EGF, and bFGF.

4. The method according to any one of claims 1 to 3, wherein the hPGCLC is induced via a human initial mesoderm-like cell (hiMeLC).

5. The method according to claim 4, wherein the hPGCLC is induced by culturing hiMeLC for 5 to 8 days in the presence of bone morphogenic protein 4 (BMP4), and optionally one or more cytokines selected from the group consisting of a stem cell factor (SCF), LIF, and EGF.

6. The method according to any one of claims 1 to 5, wherein the culture comprises sorting hPGC or hPGCLC with hPGC marker positivity as an index and passaging the hPGC or hPGCLC.

7. The method according to claim 6, wherein the hPGC marker is BLIMP1 and/or TFAP2C, or INTEGRINα6 and EpCAM.

8. The method according to claim 6 or 7, wherein the hPGC or hPGCLC is cultured for at least 30 days.

9. The method according to any one of claims 1 to 8, wherein, in the culture, the hPGC or hPGCLC is cultured under the conditions further including (iii) a Wnt signal transduction inhibitor.

10. The method according to claim 9, wherein the Wnt signal transduction inhibitor is a substance that promotes degradation and/or inhibits nuclear translocation of β-catenin.

11. A method for producing a cell population comprising hPGC or hPGCLC, comprising a step of maintaining and expanding the hPGC and/or hPGCLC by the method according to any one of claims 1 to 10.

12. An expanded hPGC population or hPGCLC population obtained by the method according to any one of claims 1 to 10.

13. A reagent kit for maintaining and expanding hPGC or hPGCLC, comprising (a) a forskolin or PDE4 inhibitor, and (b) one or more cytokines selected from the group consisting of bFGF, LIF, and EGF.

14. The reagent kit according to claim 13, wherein the inhibitor of the (a) is forskolin, and the cytokine of the (b) is a combination of LIF and EGF, bFGF alone, or a combination of LIF, EGF, and bFGF.

15. The reagent kit according to claim 13 or 14, further comprising a Dulbecco's modified Eagle medium (DMEM) with a glucose concentration of 1 g/L.

16. The reagent kit according to any one of claims 13 to 15, further comprising a Wnt signal transduction inhibitor.

17. The reagent kit according to any one of claims 13 to 16, further comprising an antibody against INTEGRINα6 and/or an antibody against EpCAM.

18. A method for producing a human oogonia-/gonocyte-like cell comprising culturing in aggregates the hPGC or hPGCLC according to claim 12 with ovarian somatic cells.

19. A method for evaluating maintenance culture of hPGC or hPGCLC, comprising

(1) a step of culturing hPGC or hPGCLC on feeder cells under given conditions,
(2) a step of sorting viable cells from a cell population obtained in (1),
(3) a step of sorting the viable cells obtained in (2) into hPGC marker-positive cells and other cells,
(4) a step of subjecting the cells obtained in (3) other than the hPGC marker-positive cells to flow cytometry, and distinguishing cells dedifferentiated from the hPGC or hPGCLC and the feeder cells by two dimensional plots of FSC/SSC, and
(5) a step of evaluating a maintenance efficiency of the hPGC or hPGCLC under the given conditions, based on the number of the hPGC marker-positive cells obtained in (3) and the number of the dedifferentiated cells obtained in (4).

20. The method according to claim 19, wherein the hPGC marker-positive cell is a BLIMP1 and TFAP2C double-positive cell.

[Fig. 1A]

[Fig. 1B]

[Fig. 1C]

[Fig. 1D]

[Fig. 1E]

**E**

[Fig. 1F]

[Fig. 2A]

[Fig. 2B]

[Fig. 2C]

[Fig. 2D]

[Fig. 2E]

[Fig. 2F]

EP 4 202 041 A1

[Fig. 2G]

EP 4 202 041 A1

[Fig. 3A]

EP 4 202 041 A1

[Fig. 3B]

d4 hPGCLC

[Fig. 3C]

EP 4 202 041 A1

[Fig. 3D]

**D**  iPSC, 585B1 BTAG

c70 expnaded hPGCLC

EP 4 202 041 A1

[Fig. 3E]

EP 4 202 041 A1

**E**

c29

[Fig. 4A]

EP 4 202 041 A1

[Fig. 4B]

[Fig. 4C]

[Fig. 4D]

[Fig. 4E]

# E

c10

c30

relief contrast

EP 4 202 041 A1

[Fig. 4F]

[Fig. 4G]

[Fig. 4H]

[Fig. 5A]

[Fig. 5B]

[Fig. 6]

[Fig. 7A]

[Fig. 7B]

[Fig. 7C]

[Fig. 7D]

[Fig. 7E]

[Fig. 8B]

**B**

d6 hPGCLC → c10 tranition

upregulated 377 genes

GO:0045926~negative regulation of growth
GO:0071276~cellular response to cadmium ion
GO:0071294~cellular response to zinc ion
hsa04978:Mineral absorption
GO:0048678~response to axon injury
GO:0002040~sprouting angiogenesis
GO:0043647~inositol phosphate metabolic process
GO:0042493~response to drug
GO:0007155~cell adhesion
GO:2000772~regulation of cellular senescence

%
4
3
2
1

gene count
• 4
• 8
• 12
● 16

$10^{-1}\ 10^{-2}\ 10^{-3}\ 10^{-4}\ 10^{-5}\ 10^{-6}$

downregulated 548 genes

GO:0007507~heart development
GO:0008285~negative regulation of cell proliferation
hsa04510:Focal adhesion
GO:0030198~extracellular matrix organization
GO:0001822~kidney development
GO:0045669~positive regulation of osteoblast differentiation
GO:0009887~organ morphogenesis
GO:0071230~cellular response to amino acid stimulus
GO:0007417~central nervous system development
GO:0000122~negative regulation of transcription from RNA polymerase II promoter

%
6
5
4
3
2

gene count
• 10
• 20
● 30

$10^{-1}\ 10^{-2}\ 10^{-3}\ 10^{-4}\ 10^{-5}$
P-value

·**Negative regulation of growth**
...*PNPT1, MT2A, MT1E, MT1H, MT1X, MT1G, MT1F*
·**Celluler responce to cadmium ion**
...*MT1E, SOD1, MT1H, MT1X, MT1G, MT1F*
·**Celluler responce to zinc ion**
...*MT2A, MT1E, MT1H, MT1X, MT1G, MT1F*
·**Mineral absorption**
...*SLC30A1, MT2A, MT1E, MT1H, MT1X, MT1G, MT1F*
·**Response to axon injury**
...*NTRK3, GIP, LGALS1, SOD1, SOD2*

·**Heart development**
... *OXTR, APLNR, GATA2, HAND1, ADM, ID1, SALL1, GATA3, HEG1 ID3, HDAC9, ACVR1, etc.*
·**Negative regulation of cell proliferation**
...*LZTS2, MSX2, GATA3,LYN, ITGA1, DLL1, NOTCH2, BTG2, SFRP2, ROR2, KLF4, etc*
·**Foacl adhesion**
...*EGFR, COL3A1, ITGA1, IGF1, COL2A1, COL4A5, LAMA4, LAMB2, PIK3CA, FN1, etc.*
·**Extracellular matrix organization**
...*COL18A1, LUM, COL3A1, POSTN, COL2A1, COL4A5, NPHP3, COL9A2, LAMA4, LAMB2, etc.*
·**Kidney development**
...*NPHP3, LZTS2, SULF2, ASS1, LHX1, SALL1, GATA3, ARID5B, OVOL1, TIPARP, ADAMTS1*
·**Positive regulation of osteoblast differentiation**
...*MSX2, SFRP2, ATP6AP1, FAM20C, IGF1, ID4, GNAS, FBN2, ACVR1*
·**Organ morphogenesis**
...*COL18A1, NOTCH2, GATA2, LHX1, GATA3, EFNB2, FGF16, TLE1, PDGFC, PALB2, SLIT3*

[Fig. 8C]

[Fig. 9A]

[Fig. 9B]

[Fig. 9C]

[Fig. 9D]

[Fig. 9E]

EP 4 202 041 A1

[Fig. 9F]

79

[Fig. 9G]

[Fig. 9H]

[Fig. 9I]

[Fig. 10A]

[Fig. 10C]

[Fig. 10D]

EP 4 202 041 A1

[Fig. 11A]

EP 4 202 041 A1

[Fig. 11B]

EP 4 202 041 A1

[Fig. 11C]

[Fig. 11D]

EP 4 202 041 A1

[Fig. 11E]

EP 4 202 041 A1

[Fig. 11F]

[Fig. 11G]

EP 4 202 041 A1

[Fig. 11H]

[Fig. 11I]

[Fig. 11J]

[Fig. 11K]

[Fig. 11L]

[Fig. 11M]

[Fig. 12A]

EP 4 202 041 A1

[Fig. 12B]

[Fig. 12C]

[Fig. 12D]

EP 4 202 041 A1

[Fig. 12E]

EP 4 202 041 A1

[Fig. 13]

[Fig. 14A]

[Fig. 14B]

B

EP 4 202 041 A1

[Fig. 14C]

## INTERNATIONAL SEARCH REPORT

| International application No. |
| --- |
| **PCT/JP2021/030701** |

**A.    CLASSIFICATION OF SUBJECT MATTER**

*C12N 5/075*(2010.01)i; *C12N 5/0735*(2010.01)i
FI:   C12N5/075; C12N5/0735

According to International Patent Classification (IPC) or to both national classification and IPC

**B.    FIELDS SEARCHED**

Minimum documentation searched (classification system followed by classification symbols)

C12N5/075; C12N5/0735

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
| --- | --- | --- |
| X | SHAMBLOTT, Michael J. et al. Derivation of pluripotent stem cells from cultured human primordial germ cells, Proc. Natl. Acad. Sci. USA, 1998, vol. 95, pp. 13726-13731        abstract, materials and methods | 1-3, 11-15, 18 |
| Y | | 1-8, 11-15,17 |
| A | | 9-10, 16, 19-20 |
| X | JP 2001-521380 A (THE JOHNS HOPKINS UNIVERSITY SCHOOL OF MEDICINE) 06 November 2001 (2001-11-06)        example 1 | 1-3, 11-15, 18 |
| Y | | 1-8, 11-15, 17 |
| A | | 9-10, 16, 19-20 |
| X | WO 2019/107576 A1 (KYOTO UNIVERSITY) 06 June 2019 (2019-06-06)        claims | 12 |
| Y | | 1-8, 11-15, 17 |

☑ Further documents are listed in the continuation of Box C.          ☑ See patent family annex.

| | |
| --- | --- |
| *     Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | "&"   document member of the same patent family |
| "P"   document published prior to the international filing date but later than the priority date claimed | |

| Date of the actual completion of the international search | Date of mailing of the international search report |
| --- | --- |
| **27 October 2021** | **09 November 2021** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2021/030701**

| C. | DOCUMENTS CONSIDERED TO BE RELEVANT | |
|---|---|---|
| Category* | Citation of document, with indication, where appropriate, of the relevant passages | Relevant to claim No. |
| Y | GELL, Joanna J. et al. An Extended Culture System that Supports Human Primordial Germ Cell-like Cell Survival and Initiation of DNA Methylation Erasure, Stem Cell Reports, 10 March 2020, vol. 14, pp. 433-446, doi:10.1016/j.stemcr.2020.01.009<br>p. 434, hPGCLCs Maintain hPGC Identity when Cultured on STO Cells, p. 436, table 1 | 4-8 |
| Y | YOKOBAYASHI Shihori et al. Clonal variation of human induced pluripotent stem cells for induction into the germ cell fate, Biology of Reproduction, 2017, vol. 96, no. 6, pp. 1154-1166, doi:10.1093/biolre/iox038<br>p. 1155, Induction of incipient mesoderm-like cells and primordial germ cell-like cells, Fluorescence-activated cell sorting analysis | 4-8, 17 |
| Y | WO 2017/002888 A1 (KYOTO UNIVERSITY) 05 January 2017 (2017-01-05)<br>claims, paragraph [0118], example 5 | 4-8, 17 |
| A | JP 2011-182720 A (KYOTO UNIVERSITY) 22 September 2011 (2011-09-22)<br>whole document | 19-20 |

Form PCT/ISA/210 (second sheet) (January 2015)

**INTERNATIONAL SEARCH REPORT**

International application No.

**PCT/JP2021/030701**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☐ forming part of the international application as filed:

      ☐ in the form of an Annex C/ST.25 text file.

      ☐ on paper or in the form of an image file.

   b. ☑ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☑ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2021/030701**

| Patent document cited in search report | | | Publication date (day/month/year) | Patent family member(s) | | | Publication date (day/month/year) |
|---|---|---|---|---|---|---|---|
| JP | 2001-521380 | A | 06 November 2001 | US example 1 | 6090622 | A | |
| | | | | US | 6331406 | B1 | |
| | | | | US | 6245566 | B1 | |
| | | | | US | 6562619 | B1 | |
| | | | | US | 2003/0148514 | A1 | |
| | | | | WO | 1998/043679 | A1 | |
| | | | | EP | 1023085 | A1 | |
| | | | | AU | 6947798 | A | |
| | | | | CA | 2285274 | A | |
| WO | 2019/107576 | A1 | 06 June 2019 | US claims | 2020/0362303 | A1 | |
| WO | 2017/002888 | A1 | 05 January 2017 | US claims, paragraph [0141], example 5 | 2018/0187147 | A1 | |
| | | | | US | 2020/0181568 | A1 | |
| JP | 2011-182720 | A | 22 September 2011 | WO whole document | 2011/111588 | A1 | |

Form PCT/ISA/210 (patent family annex) (January 2015)

## REFERENCES CITED IN THE DESCRIPTION

**Patent documents cited in the description**

- US 63066914 B
- US 63067776 B
- WO 2019107576 A
- WO 2007069666 A
- WO 2008118820 A
- WO 2009007852 A
- WO 2009032194 A
- WO 2009058413 A
- WO 2009057831 A
- WO 2009075119 A
- WO 2009079007 A
- WO 2009091659 A
- WO 2009101084 A
- WO 2009101407 A
- WO 2009102983 A
- WO 2009114949 A
- WO 2009117439 A
- WO 2009126250 A
- WO 2009126251 A
- WO 2009126655 A
- WO 2009157593 A
- WO 2010009015 A
- WO 2010033906 A
- WO 2010033920 A
- WO 2010042800 A
- WO 2010050626 A
- WO 2010056831 A
- WO 2010068955 A
- WO 2010098419 A
- WO 2010102267 A
- WO 2010111409 A
- WO 2010111422 A
- WO 2010115050 A
- WO 2010124290 A
- WO 2010147395 A
- WO 2010147612 A
- WO 2010008054 A
- JP 5963309 B
- WO 2010137746 A
- WO 2009123349 A
- WO 9830679 A
- WO 2017002888 A

**Non-patent literature cited in the description**

- **OHTA et al.** *The EMBO Journal,* 2017, vol. 36, 1888-1907
- **GELL et al.** *Stem Cell Reports,* 2020, vol. 14, 433-446
- **THOMSON JA et al.** *Science,* 1998, vol. 282, 1145-1147
- **TAKAHASHI, K. ; S. YAMANAKA.** *Cell,* 2006, vol. 126, 663-676
- **TAKAHASHI, K. et al.** *Cell,* 2007, vol. 131, 861-872
- **YU, J. et al.** *Science,* 2007, vol. 318, 1917-1920
- **NAKAGAWA, M. et al.** *Nat. Biotechnol.,* 2008, vol. 26, 101-106
- **HUANGFU, D. et al.** *Nat. Biotechnol.,* 2008, vol. 26, 795-797
- **SHI, Y. et al.** *Cell Stem cell,* 2008, vol. 2, 525-528
- **EMINLI, S. et al.** *Stem Cells,* 2008, vol. 26, 2467-2474
- **HUANGFU, D. et al.** *Nat. Biotechnol.,* 2008, vol. 26, 1269-1275
- **SHI, Y. et al.** *Cell Stem Cell,* 2008, vol. 3, 568-574
- **ZHAO, Y. et al.** *Cellstem Cell,* 2008, vol. 3, 475-479
- **MARSON, A.** *Cell Stem Cell,* 2008, vol. 3, 132-135
- **FENG, B. et al.** *Nat. Cell Biol.,* 2009, vol. 11, 197-203
- **JUDSON, R.L. et al.** *Nat. Biotechnol.,* 2009, vol. 27, 459-461
- **LYSSIOTIS, C.A. et al.** *Proc. Natl. Acad. Sci. USA,* 2009, vol. 106, 8912-8917
- **KIM, J.B. et al.** *Nature,* 2009, vol. 461, 649-643
- **ICHIDA, J.K. et al.** *Cell Stem Cell,* 2009, vol. 5, 491-503
- **HENG, J.C. et al.** *Cell Stem Cell,* 2010, vol. 6, 167-74
- **HAN, J. et al.** *Nature,* 2010, vol. 463, 1096-100
- **MALI, P. et al.** *Stem Cells,* 2010, vol. 28, 713-720
- **MAEKAWA, M. et al.** *Nature,* 2011, vol. 474, 225-9
- *Cell Stem Cell,* 08 May 2009, vol. 4 (5), 381-4
- *Cell,* 2006, vol. 126, 663-676

- *Cell,* 2007, vol. 131, 861-872
- *Science,* 2007, vol. 318, 1917-1920
- *Science,* 2008, vol. 322, 945-949
- *Science,* 2008, vol. 322, 949-953
- **WARREN, L.** *Cell Stem Cell,* 2010, vol. 7, 618-630
- *Cell Stem Cell.,* 05 November 2010, vol. 7 (5), 618-30
- *Cell Stem Cell.,* 03 June 2011, vol. 8 (6), 633-8
- *Science,* 09 August 2013, vol. 341 (6146), 651-4
- **TAKAHASHI K et al.** *PLoS One,* 2009, vol. 4, e8067
- **TAKAHASHI et al.** *Cell,* 2007, vol. 131, 861-872
- *Cell,* 01 May 2009, vol. 137 (3), 571-84
- *Cell Stem Cells,* 04 June 2010, vol. 6 (6), 535-46
- *Proc. Natl. Acad. Sci. USA,* 18 May 2010, vol. 107 (20), 9222-7
- **KLIMANSKAYA I et al.** *Lancet,* 2005, vol. 365, 1636-1641
- **HAYASHI K et al.** *Cell,* 19 August 2011, vol. 146 (4), 519-32
- *CHEMICAL ABSTRACTS,* 252917-06-9
- **GELL et al.** *Stem Cell Reports,* 2020
- *Science,* 2018, vol. 362, 356-360
- **SASAKI et al.** *Cell Stem Cell,* 06 August 2015, vol. 17 (2), 178-94
- **YOKOBAYASHI et al.** *Biol Reprod.,* 01 June 2017, vol. 96 (6), 1154-1166
- **YAMASHIRO et al.** *Science,* 19 October 2018, vol. 362 (6412), 356-360
- **HAYASHI ; SAITOU.** *Nat Protoc.,* August 2013, vol. 8 (8), 1513-24
- **DOLCI et al.** *Nature,* 29 August 1991, vol. 352 (6338), 809-11
- **MAJUMDAR et al.** *J Biol Chem.,* 14 January 1994, vol. 269 (2), 1237-42
- **OHTA et al.** *EMBO J.,* 03 July 2017, vol. 36 (13), 1888-1907
- **MIYAUCHI et al.** *Methods Cell Biol.,* 2018, vol. 144, 409-429
- **YAMASHIRO et al.** *Nat Protoc.,* April 2020, vol. 15 (4), 1560-1583
- **NAKAMURA et al.** *Nucleic Acids Res.,* 19 May 2015, vol. 43 (9), e60
- **ISHIKURA et al.** *Cell Rep.,* 06 December 2016, vol. 17 (10), 2789-2804
- **MIURA et al.** *Nucleic Acids Res.,* 01 September 2012, vol. 40 (17), e136
- **SHIRANE et al.** *Dev Cell.,* 10 October 2016, vol. 39 (1), 87-103
- **LANGMEAD ; SALZBERG.** *Nat Methods.,* 04 March 2012, vol. 9 (4), 357-9
- **KIM et al.** *Genome Biol.,* 25 April 2013, vol. 14 (4), R36
- **ANDERS et al.** *Bioinformatics,* 15 January 2015, vol. 31 (2), 166-9
- **HUANG et al.** *Nat Protoc.,* 2009, vol. 4 (1), 44-57
- **BORGEL et al.** *Nat Genet.,* December 2010, vol. 42 (12), 1093-100
- **ILLINGWORTH et al.** *PLoS Genet,* 23 September 2010, vol. 6 (9), e1001134
- **COURT et al.** *Genome Res.,* April 2014, vol. 24 (4), 554-69
- **LI et al.** *Bioinformatics,* 15 August 2009, vol. 25 (16), 2078-9
- **ROBINSON et al.** *Nat Biotechnol.,* January 2011, vol. 29 (1), 24-6
- **TANG et al.** *Cell,* 04 June 2015, vol. 161 (6), 1453-67
- **SONG et al.** *PLoS One,* 06 December 2013, vol. 8 (12), e81148
- **QUINLAN ; HALL.** *Bioinformatics,* 15 March 2010, vol. 26 (6), 841-2
- **OKAE et al.** *PLoS Genet,* 11 December 2014, vol. 10 (12), e1004868
- **DE FELICI et al.** *Dev Biol.,* May 1993, vol. 157 (1), 277-80
- **MATSUI et al.** *Nature,* 24 October 1991, vol. 353 (6346), 750-2
- **DRAPER et al.** *J Anat.,* March 2002, vol. 200, 249-58
- **LAINE et al.** *Mol Cell,* August 2000, vol. 6 (2), 395-407
- **NISHIMURA et al.** *Stem Cell Reports.,* 14 March 2017, vol. 8 (3), 787-801
- **HAQ et al.** *Mutat Res.,* 10 December 2003, vol. 533 (1-2), 211-26
- **BOSTICK et al.** *Science,* 21 September 2007, vol. 317 (5845), 1760-4
- **SHARIF et al.** *Nature,* 06 December 2007, vol. 450 (7171), 908-12
- **LEONHARDT.** *J Cell Biol.,* 17 April 2000, vol. 149 (2), 271-80
- **O'KEEFE.** *J Cell Biol.,* March 1992, vol. 116 (5), 1095-110
- **RAMSAHOYE et al.** *Proc Natl Acad Sci U S A.,* 09 May 2000, vol. 97 (10), 5237-42
- **LIAO et al.** *Nat Genet.,* May 2015, vol. 47 (5), 469-78
- **SEISENBERGER et al.** *Mol Cell.,* 28 December 2012, vol. 48 (6), 849-62
- **KOBAYASHI et al.** *Genome Res.,* April 2013, vol. 23 (4), 616-27
- **KUBO et al.** *BMC Genomics,* 20 August 2015, vol. 16 (1), 624
- **HABIBI et al.** *Cellstem Cell.,* 05 September 2013, vol. 13 (3), 360-9
- **YAMAJI et al.** *Cellstem Cell.,* 07 March 2013, vol. 12 (3), 368-82
- **GKOUNTELA et al.** *Cell,* 04 June 2015, vol. 161 (6), 1425-36

- **GUO et al.** *Cell,* 04 June 2015, vol. 161 (6), 1437-52

- **LI et al.** *Cellstem Cell,* 01 June 2017, vol. 20 (6), 858-873